# EUROPÄISCHE PATENTSCHRIFT

(11) **EP 2 906 572 B1**
(45) Veröffentlichungstag und Bekanntmachung des Hinweises auf die Patenterteilung: **23.11.2016**
(21) Anmeldenummer: 13773207.9
(22) Anmeldetag: 27.09.2013
(51) Int. Cl.: C07F 9/6574, B01J 31/02, B01J 31/18, C07C 45/50

(54) **GEMISCHE KONSTITUTIONSISOMERER BISPHOSPHITE**
MIXTURES OF CONSTITUTIONAL ISOMERS OF BIPHOSPHITES
MÉLANGES D'ISOMÈRES DE CONSTITUTION DE BIPHOSPHITES

(30) Priorität: 12.10.2012 DE 102012218627; 12.10.2012 DE 102012218625; 12.10.2012 DE 102012218629; 12.10.2012 DE 102012218630
(43) Veröffentlichungstag der Anmeldung: 19.08.2015
(73) Patentinhaber: Evonik Degussa GmbH, 45128 Essen (DE)
(72) Erfinder: CHRISTIANSEN, Andrea, 18119 Rostock (DE); FRANKE, Robert, 45772 Marl (DE); FRIDAG, Dirk, 45721 Haltern am See (DE); HESS, Dieter, 45770 Marl (DE); DYBALLA, Katrin Marie, 45657 Recklinghausen (DE); HANNEBAUER, Bernd, 63165 Mühlheim (DE)
(86) Internationale Anmeldenummer: PCT/EP2013/070224
(87) Internationale Veröffentlichungsnummer: WO 2014/056735

(56) Entgegenhaltungen:
- EP-B1- 1 294 731
- DE-A1-102008 002 187
- US-A- 4 769 498

## Beschreibung

Die Erfindung betrifft Gemische von konstitutionsisomeren Bisphosphiten, ein Verfahren zu deren Herstellung, sowie deren Umsetzung mit Metallen zu Gemischen enthaltend Komplexverbindungen aus den konstitutionsisomeren Bisphosphiten und dem Metall sowie deren Verwendung als katalytisch aktive Zusammensetzung in Hydroformylierungsreaktionen, wobei die hydroformylierungsaktive Zusammensetzung neben den Komplexverbindungen aus Metall und den konstitutionsisomeren Bisphosphiten, ungebundene Bisphosphite der konstitutionsisomeren Bisphosphite und zumindest eine weitere Komponente umfasst.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. Als Katalysatoren in diesen Reaktionen werden häufig Verbindungen der Übergangsmetalle der VIII. Gruppe des Periodensystems der Elemente verwendet. Bekannte Liganden sind beispielsweise Verbindungen aus den Klassen der Phosphine, Phosphite und Phosphonite mit jeweils dreiwertigem Phosphor P^{III}. Eine gute Übersicht über den Stand der Hydroformylierung von Olefinen findet sich in B. CORNILS, W. A. HERRMANN, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI:10.1021/cr3001803.

Jede katalytisch aktive Zusammensetzung hat ihre spezifischen Vorzüge. Je nach Einsatzstoff und Zielprodukt kommen daher unterschiedliche katalytisch aktive Zusammensetzungen zum Einsatz.

Die Patente US 4 694 109 und US 4 879 416 beschreiben Bisphosphinliganden und ihren Einsatz in der Hydroformylierung von Olefinen bei niedrigen Synthesegasdrücken. Besonders bei der Hydroformylierung von Propen werden mit Liganden dieses Typs hohe Aktivitäten und hohe n/i-Selektivitäten (n/i = das Verhältnis von linearem Aldehyd (=n) zu verzweigtem (=iso) Aldehyd) erreicht. In WO 95/30680 werden zweizähnige Phosphinliganden und ihr Einsatz in der Katalyse, unter anderem auch in Hydroformylierungsreaktionen, offen gelegt. Ferrocenverbrückte Bisphosphine werden beispielsweise in den Patentschriften US 4 169 861, US 4 201 714 und US 4 193 943 als Liganden für Hydroformylierungen beschrieben.

Der Nachteil von zwei- und mehrzähnigen Phosphinliganden ist ein relativ hoher Aufwand, der zu ihrer Darstellung notwendig ist. Daher ist es oftmals nicht rentabel, solche Systeme in technischen Prozessen einzusetzen. Hinzu kommt eine vergleichsweise geringe Aktivität, die durch hohe Verweilzeiten reaktionstechnisch kompensiert werden muss. Dies wiederum führt zu unerwünschten Nebenreaktionen der Produkte.

Rhodium-Monophosphit-Komplexe in katalytisch aktiven Zusammensetzungen sind geeignet für die Hydroformylierung von verzweigten Olefinen mit innenständigen Doppelbindungen, jedoch ist die Selektivität für endständig oxierte Verbindungen gering. Aus EP 0 155 508 ist die Verwendung von bisarylensubstituierten Monophosphiten bei der rhodiumkatalysierten Hydroformylierung von sterisch gehinderten Olefinen, z. B. Isobuten, bekannt.

Katalytisch aktive Zusammensetzungen auf Basis von Rhodium-Bisphosphit-Komplexen sind geeignet für die Hydroformylierung von linearen Olefinen mit end- und innenständigen Doppelbindungen, wobei überwiegend endständig hydroformylierte Produkte entstehen. Dagegen werden verzweigte Olefine mit innenständigen Doppelbindungen nur im geringen Maße umgesetzt. Diese Phosphite ergeben bei ihrer Koordination an ein Übergangsmetallzentrum Katalysatoren von gesteigerter Aktivität, doch ist das Standzeitverhalten dieser katalytisch aktiven Zusammensetzungen, unter anderem wegen der Hydrolyseempfindlichkeit der Phosphitliganden, unbefriedigend. Durch den Einsatz von substituierten Bisaryldiolen als Bausteine für die Phosphitliganden, wie in EP 0 214 622 oder EP 0 472 071 beschrieben, konnten erhebliche Verbesserungen erreicht werden.

Der Literatur zufolge sind die katalytisch aktiven Zusammensetzungen dieser Liganden auf Basis von Rhodium äußerst aktiv in der Hydroformylierung von α-Olefinen. In den Patenten US 4 668 651, US 4 748 261 und US 4 885 401 werden Polyphosphitliganden beschrieben, mit denen α-Olefine, aber auch 2-Buten mit hoher n/i-Selektivität zu den terminal oxierten Produkten umgesetzt werden können. Zweizähnige Liganden dieses Typs wurden auch zur Hydroformylierung von Butadien eingesetzt (US 5 312 996).

Die in EP 1 294 731 offenbarten Bisphosphite weisen bei der Hydroformylierung von n-Octengemischen Olefinumsätze bis zu 98 % auf. Jedoch ist die ebenfalls gewünschte n-Selektivität zum Nonanal mit 36,8 % bis maximal 57,6 % verbesserungswürdig. Dies gilt umso mehr, als dass die Verwendung der katalytisch aktiven Zusammensetzung in technischen Prozessen eine Standzeit verlangt, welche sich in Tagen anstelle von Stunden bemisst.
Literaturbekannt ist die Synthese symmetrisch aufgebauter Bisphosphite, wie sie seit US 4769498 offenbart wurden und deren Verwendung in katalytisch aktiven, übergangsmetallhaltigen Zusammensetzungen zur Hydroformylierung ungesättigter Verbindungen.

In US 4769498, wie auch in US 5723641 werden bevorzugt symmetrisch aufgebaute Bisphosphite hergestellt und als Liganden zur Hydroformylierung verwendet. Die in der Hydroformylierung verwendeten symmetrisch aufgebauten Bisphosphitliganden werden bei tiefen Temperaturen hergestellt. Die Einhaltung dieser tiefen Temperaturen ist zwingend erforderlich, da höhere Temperaturen gemäß dieser US-Schriften zu Umlagerungen und letztlich zu unsymmetrisch aufgebauten Bisphosphiten führen würde, was aber hier nicht erwünscht ist.
Diese unsymmetrisch aufgebauten Bisphosphite weisen bei der Verwendung als Ligand in der übergangsmetallkatalysierten Hydroformylierung deutlich geringere Reaktivitäten und geringere n-Regioselektivität auf; siehe in Rhodium-catalyzed Hydroformylation, ed. by P.W.N.M. van Leeuwen et C. Claver, Kluwer Academic Publishers 2006, AA Dordrecht, NL, Seite 45-46.
Wie von van Leeuwen ausgeführt, weisen die symmetrischen Bisphosphite neben höheren Selektivitäten auch eine größere Reaktivität auf. Neben dem Bestreben einer hohen Reaktivität und n-Selektivität in Bezug auf die zu carbonylierenden, ungesättigten Verbindungen ist die Stabilität - konkret die Standzeit - der katalytisch aktiven Zusammensetzung aus jeweils verwendetem Metall, Liganden sowie weiteren Komponenten mit aktivierender Wirkung mit Blick auf die als Liganden eingesetzten Bisphophite eine ständige Aufgabe der Forschung. Dies gilt insbesondere hinsichtlich olefinhaltiger Gemische, speziell in der Hydroformylierung von Gemischen linearer Olefine.

In US 5364950, wie auch in US 5763677 und in "Catalyst Separation, Recovery and Recycling", herausgegeben v. D.J. Cole-Hamilton, R.P. Tooze, 2006, NL, Seiten 25-26, wird die Bildung von sogenannten "*Poisoning Phosphites"* als Neben- bzw. Ligandenabbaureaktion beschrieben. Diese "*Poisoning Phosphites"* bilden sich bei der Verwendung von arylphosphit-modifizierten Rhodium-Komplexen während der Hydroformylierungsreaktion. Hierbei kommt es im Zuge des Ligandenabbaus zu einem Austausch einer Arylgruppe durch eine Alkylgruppe des Hydroformylierungsproduktes.
Neben der Bildung der unerwünschten "*Poisoning Phosphites"* kann der Phosphitligand auch im Zuge einer Hydrolysereaktion durch die bei der Aldehydkondensation gebildeten Wasserspuren abgebaut werden.

Eine Konsequenz aus diesen Abbaureaktionen der Liganden ist, dass die Konzentration an hydroformylierungs-aktiven Rhodiumkomplexspezies im Laufe der Zeit abnimmt und mit einem Verlust an Reaktivität einher geht.

Es ist allgemein bekannt, dass bei einer kontinuierlichen Fahrweise der Hydroformylierung Ligand/en und - gegebenenfalls weitere Komponenten - während des Reaktionsverlaufs nachdosiert, d. h. nach Reaktionsbeginn zusätzlich zugegeben werden müssen (siehe DE 10 2008 002 187 A1).

Die technische Aufgabe der vorliegenden Erfindung ist die Bereitstellung neuer Liganden, welche in der Hydroformylierung von ungesättigten Verbindungen nicht die aus dem Stand der Technik zuvor aufgezeigten Nachteile, sondern die folgenden Eigenschaften aufweisen:
1.) eine hohe Aktivität;
2.) eine hohe n-Regioseliktivität in Bezug auf die Hydroformylierung und
3.) eine hohe Standzeit.

Eine hohe Standzeit bedeutet, dass die hydroformylierungsaktive Zusammensetzung, welche die Liganden neben weiteren Komponenten umfasst, eine geringe Tendenz zum Abbau dieser Liganden und/oder den Zerfall dieser Liganden in hydroformylierungsinhibierende Komponenten, wie z.B. die sogenannten "*Poisoning Phosphites"* aufweist.

Die Aufgabe wird gelöst durch ein Gemisch der Verbindungen der Formeln (1a) und (2a):

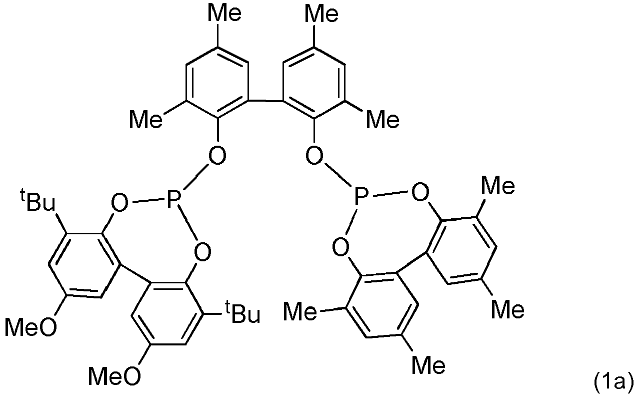

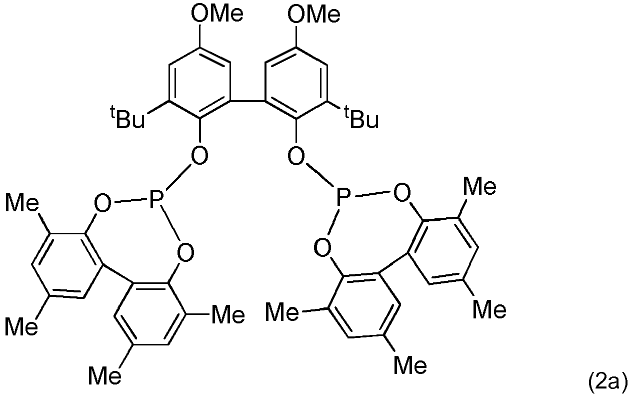

Die Erfindung umfasst folgende Gegenstände:
a) Gemische konstitutionsisomerer Bisphosphite der Formeln (1a) und (2a);
b) Verfahren zu deren Herstellung;
c) Metall-Komplexgemische der Formeln (1 b) und (2b), wobei M ein Metall der 4. bis 10. Gruppe des Periodensystems der Elemente (Fe, Ru, Os, Co, Rh, Ir, Ni, Pd, Pt) darstellt und zusätzliche Bindungen eingehen kann und die Konstitutionsisomeren der Formeln (1a) und (2a) vorliegen, die nicht an das Metall M gebunden sind; 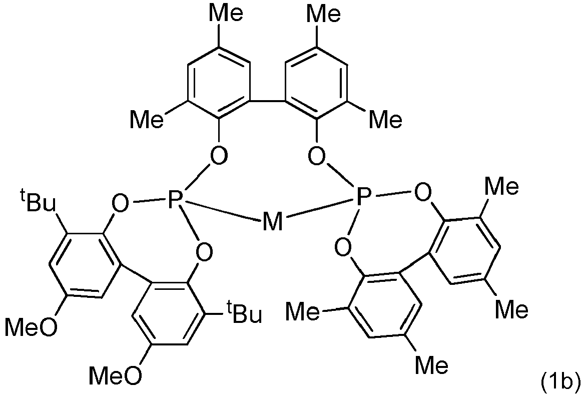 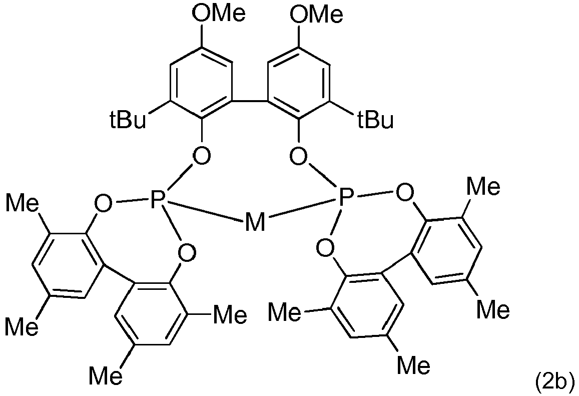
d) Zusammensetzungen, enthaltend die unter a) genannten Konstitutionsisomeren, Metalle der 4. bis 10. Gruppe des Periodensystems der Elemente (Fe, Ru, Os, Co, Rh, Ir, Ni, Pd, Pt) sowie freie, d.h. ungebundene konstitutionsisomere Bisphosphite der Formeln (1a) und (2a) und zumindest eine weitere Komponente, ausgewählt aus der Gruppe, welche Basen, organische Amine, Epoxide, Ionenaustauscher, Puffersysteme umfasst;
e) Verfahren zur Hydroformylierung ungesättigter Verbindungen und deren Gemischen unter Verwendung von Zusammensetzungen nach d), eines Gasgemisches bestehend aus Kohlenmonoxid und Wasserstoff, ungesättigter Verbindungen und deren Gemischen unter den für eine Hydroformylierung erforderlichen Reaktionsbedingungen. In einer Ausführungsform liegt der Gehalt an Isomerem der Formel (1a) in einem Bereich von 74 bis 99 Massen-%, der Gehalt an Isomerem der Formel (2a) in einem Bereich von 1 bis 26 Massen-%.

Die Gehalte an Konstitutionsisomeren der Formel (1a) und der Formel (2a) addieren sich zu 100 Massen-%. Diese definierten Gemische der konstitutionsisomeren Bisphosphite können beispielsweise direkt zu Beginn einer Hydroformylierungsreaktion vorgelegt werden. Diese Vorgehensweise unterscheidet sich somit von dem allgemeinen Vorgehen bei einer Stabilitätsuntersuchung, bei der ein definiertes Isomer vorgelegt wird und die weiteren Isomeren sich erst im Reaktionsverlauf bilden.

Normalerweise werden im Stand der Technik möglichst reine Liganden in der Hydroformylierungsreaktion eingesetzt, da das jeweils andere Isomer starke negative Einflüsse auf die Gesamtperformance des Systems ausübt. In der Regel würde das unsymmetrische Isomer als Nebenkomponente vorliegen, da ausschließlich symmetrische Liganden in der Hydroformylierung eingesetzt werden,

In Rhodium-catalyzed Hydroformylation, ed. by P.W.N.M. van Leeuwen et C. Claver, Kluwer Academic Publishers 2006, AA Dordrecht, NL, Seite 45-46, Tabelle 2 sind die Hydroformylierungsergebnisse des symmetrischen Biphephosliganden und seines unsymmetrischen Isomers beschrieben. Dabei geht deutlich hervor, dass sich der symmetrische Biphephosligand (in der Literaturstelle Ligand 5a) durch eine deutlich höhere n/i-Selektivität und eine höhere Aktivität auszeichnet als sein unsymmetrisches Isomer (in der Literaturstelle Ligand 7). In der Hydroformylierungsreaktion von Propen weist der symmetrische Ligand eine n/i-Selektivität von 53 und eine Reaktionsrate von 402 auf, wohingegen der unsymmetrische Ligand lediglich eine n/i-Selektivität von 1.2 und eine Reaktionsrate von 280 auf. Würde man nun ein Gemisch beider Liganden einsetzen, so würde dies zu deutlich schlechteren Ausbeuten und n/i-Selektivitäten führen. Eine deutlich schlechtere Gesamtperformance konnte auch mit der Isomeren-Mischung aus den Liganden (7) und (8) verzeichnet werden.
Verwendet man nun die erfindungsgemäße Isomerenmischung in der Hydroformylierung, so ist dies nicht der Fall, und das andere Isomer kann als Nebenkomponente im Isomerengemisch vorliegen ohne die Gesamtperformance des Systems negativ zu beeinflussen.

Dies ist besonders vorteilhaft, da somit während der Ligandenherstellung keine weiteren Aufreinigungsschritte notwendig sind, um ein Isomer mit einer 100%igen Reinheit zu erhalten. Dies ist besonders günstig, da jeder weitere Aufreinigungsschritt in der Ligandenherstellung zu einer Verteuerung desselbigen führt. In der Regel werden für diese Aufreinigungen verschiedenen Lösungsmittel verwendet und es sind unter Umständen verschiedenen Aufreinigugen notwendig, wie beispielsweise Umkristallisationen, die zwangsläufig zu Produktverlusten führen. Dies führt wiederum dazu, dass der Ligand in seiner Herstellung deutlich teurer wird und das wiederum wirkt sich negativ auf die Gesamtwirtschaftlichkeit eines großtechnischen Prozesses aus. Somit ist es besonders vorteilhaft, wenn es möglich ist, auf teure Aufreinigungsschritte zu verzichten und entsprechende Isomerenmischungen in einem großtechnischen Hydroformylierungsprozess einzusetzen.

Das erfindungsgemäße Verfahren zur Herstellung des Gemisches der konstitutionsisomeren Verbindungen der Formeln (1a) und (2a) umfasst die Schritte:
i) oxidative Kopplung von 2,4-Dimethylphenol zu 3, 3', 5, 5'-Tetramethyl-2,2'-dihydroxybiphenyl;
ii) oxidative Kopplung von 3-tert.-Butyl-4-Hydroxyanisol zu 5, 5'-Dimethoxy-3, 3'-di-tert.-Butyl-2, 2'-dihydroxybiphenyl;
iii) Umsetzung von 3, 3', 5, 5'-Tetramethyl-2,2'-dihydroxybiphenyl aus i) mit PCl₃ zu einem Phosphorochloriditderivat unter Inertgasatmosphäre;
iv) Umsetzung von zumindest 2 Äquivalenten des Phophorochloriditderivats aus iii) mit 1 Äquivalent des 5, 5'-Dimethoxy-3, 3'-di-tert.-Butyl-2, 2'-dihydroxybiphenyl aus ii) unter Inertgasatmosphäre.

In einer Variante des Verfahrens wird Verfahrensschritt iv) ein Lösungsmittelgemisch verwendet.

In einer Variante ist das Gemisch der konstitutionsisomeren Bisphosphite (1a) und (2a) erhältlich, wobei das Lösungsmittelgemisch, welches im Verfahrensschritt iv) verwendet wird ausgewählt aus: organischen Stickstoffverbindungen, organischen Estern, Aromaten. In einer besonderen Ausführungsform sind die organischen Stickstoffverbindungen ausgewählt aus: Nitrilen, Aminen, Amiden.

In einer bevorzugten Ausführungsform ist die organischen Stickstoffverbindungen ausgewählt aus: Pyridin, Triethylamin, Dimethylaminobutan, Tributylamin, Tripentylamin, Trihexylamin.

In einer Ausführungsform wird im Verfahrensschritt iv) ein Lösungsmittelgemisch verwendet, welches zwei organischen Stickstoffverbindungen aufweist. Vorzugsweise sind die zwei organischen Stickstoffverbindungen ausgewählt aus: Pyridin, Triethylamin, Dimethylaminobutan, Tributylamin, Tripentylamin, Trihexylamin.

In einer bevorzugten Ausführungsform werden Acetonitril, Triethylamin, Dimethylaminobutan, Di-i-propylethylamin, N-Methylpyrolidon, Pyridin, Ethylacetat, Toluol verwendet.

In besonders bevorzugten Varianten des Verfahrens erfolgt der Verfahrensschritt iv) in einem aprotisch-polaren Lösungsmittel, oder einem Gemisch, welches mindestens ein aprotisch-polares Lösungsmittel umfasst.

Unter dem Begriff aprotisches Lösungsmittel werden im Rahmen dieser Anmeldung nichtwäßrige Lösemittel, die kein ionisierbares Proton im Molekül enthalten, verstanden, welche weiter in aprotisch-unpolare und aprotisch-polare Lösungsmittel unterteilt sind (siehe Thieme Römpp online).

Unter der Bezeichnung aprotisch-unpolare oder apolar aprotische Lösungsmittel werden aliphatische und aromatische sowie halogenierte Kohlenwasserstoffe (Alkane, Alkene, Alkine, Benzol, Aromaten mit aliphatischen oder aromatischen Seitenketten), perhalogenierte Kohlenwasserstoffe wie Tetrachlorkohlenstoff und Hexafluorbenzol, Tetramethylsilan und Schwefelkohlenstoff zusammengefasst.

Aprotisch-unpolare Lösemittel sind durch niedrige relative Permittivitäten (εr <15), niedrige Dipolmomente (µ <2,5 D) und niedrige ETN-Werte (0,0-0,3; ETN = normalisierte Werte der empirischen Parameter der Lösemittelpolarität) charakterisiert. Aprotisch-unpolare Lösemittel sind lipophil und hydrophob. Zwischen ihren Molekülen herrschen Van-der-Waals-Wechselwirkungen.

Die unter dem Begriff aprotisch-polare oder dipolar aprotische Lösungsmittel zusammengefassten Lösungsmittel besitzen stark polarisierende funktionelle Gruppen und zeigen daher ein gewisses permanentes Dipolmoment, das zu den nun untergeordneten Van-der-Waals-Wechselwirkungen hinzukommt. Ihr Lösevermögen für polare Stoffe ist somit in der Regel besser als das der aprotisch-unpolaren Lösungsmittel. Beispiele aprotischpolarer Lösungsmittel sind Ketone wie Aceton, Ether, Ester, *N*,*N*-disubstituierte Amide wie Dimethylformamid, tertiäre Amine, Pyridin, Furan, Thiophen, 1,1,1-Trichlorethan, Nitroalkane wie Nitromethan, Nitrile wie Acetonitril, Sulfoxide wie Dimethylsulfoxid, Sulfone, Hexamethylphosphorsäuretriamid, flüssiges Schwefeldioxid, Selenoxychlorid. Diese besitzen große Permittivitäten (εr >15), Dipolmomente (µ >2,5 D) und ETN-Werte im Bereich von 0,3-0,5.

Das aprotische Lösungsmittelgemisch, welches zu der erfindungsgemäßen Herstellung der konstitutionsisomeren Bisphosphite (1a) und (2a) verwendet wird, ist ausgewählt aus organischen Stickstoffverbindungen, organischen Estern sowie Aromaten.

Neben dem Gemisch wird auch ein Komplexgemisch beansprucht.

Komplexgemisch aus Verbindungen der Formeln (1 b) und (2b): 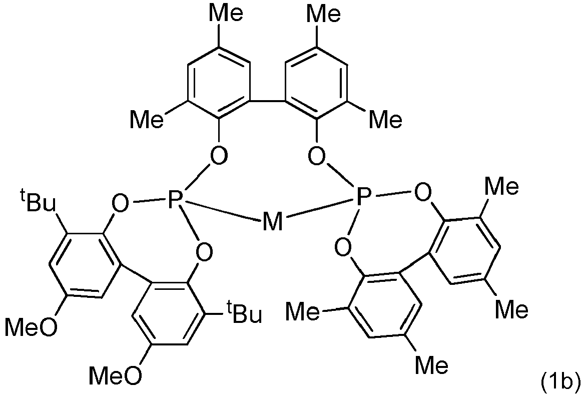 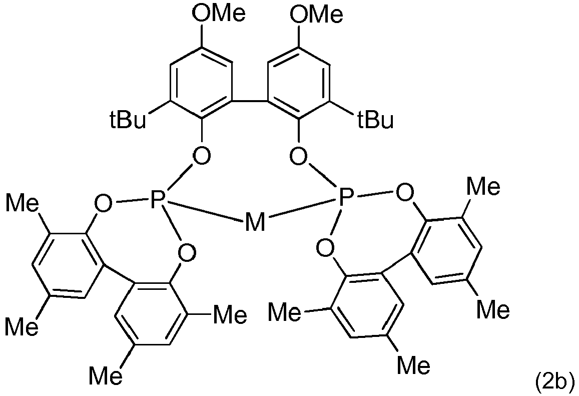 wobei M jeweils für ein Element steht ausgewählt aus: Fe, Ru, Os, Co, Rh, Ir, Ni, Pd, Pt und M zusätzliche Bindungen eingehen kann.

Hierbei sind Co, Rh, Ru, Ir, Fe bevorzugt; und Rh besonders bevorzugt.

In einer Ausführungsform umfasst das Komplexgemisch zusätzlich mindestens eine der Verbindungen (1a) oder (2a), die nicht an das Metall M gebunden sind.

Die erfindungsgemäßen Komplexverbindungen der Formeln (1b) und (2b) werden in situ während der Hydroformylierungsreaktion gebildet.

In einer besonderen Ausführungsform der Erfindung liegen die Komplexverbindungen (1c) und (2c) als Gemisch neben den konstitutionsisomeren Bisphosphiten der Formeln (1a) und (2a) vor, 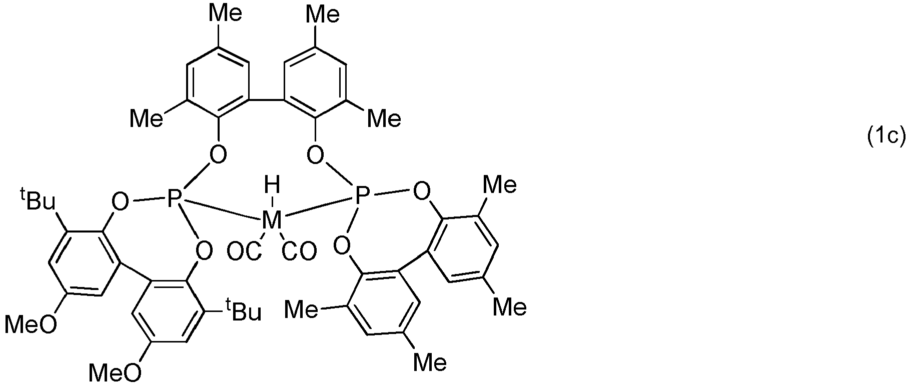 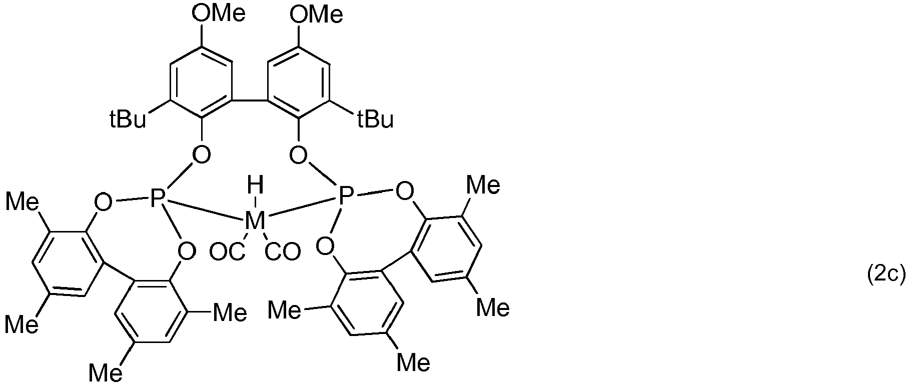 wobei M jeweils für ein Element steht ausgewählt aus: Fe, Ru, Os, Co, Rh, Ir, Ni, Pd, Pt.

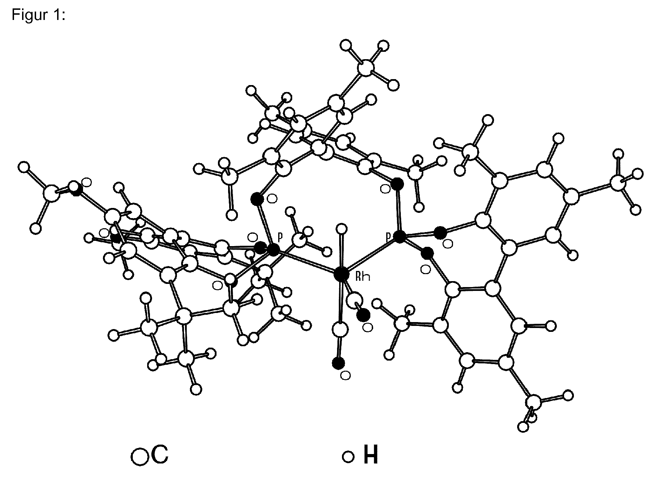

Hierbei sind Co, Rh, Ru, Ir, Fe bevorzugt; und Rh besonders bevorzugt und ist als Verbindung (1ca) offenbart in Figur 1.

Neben dem Komplexgemisch wird auch eine Zusammensetzung beansprucht, welche dieses umfasst.

Zusammensetzung umfassend:
- ein zuvor beschriebenes Komplexgemisch,
- eine weitere Komponente ausgewählt aus: Basen, organische Amine, Epoxide, Pufferlösungen, Ionenaustauscher.
In US 4567306, US 5364950, US 5741942 und US 5763677 werden Beispiele für diese weiteren Komponenten offenbart.

In einer bevorzugten Ausführungsform werden als weitere Komponenten sterisch gehinderte sekundäre Amine Verbindungen mit der allgemeinen Formel (I) eingesetzt, 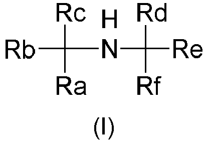 wobei Ra, Rb, Rc, Rd, Re und Rf gleiche oder unterschiedliche Kohlenwasserstoffreste, die auch untereinander verbunden sein können, sind.

In einer bevorzugten Ausführungsform weist das organische Amin eine Struktur gemäß Formel (Ia) auf: 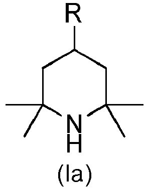 mit R gleich H, wie das 2,2,6,6-Tetramethylpiperidin selbst, einem organischen Rest R, einer Hydroxylgruppe oder einem Halogen.

Der organische Rest R kann auch ein über ein Heteroatom, beispielsweise ein Sauerstoffatom, an die 2,2,6,6-Tetramethylpiperidin-Struktureinheit gebundener, organischer Rest sein. Insbesondere kann der organische Rest polymere Strukturen aufweisen oder ein 1 bis 50 Kohlenstoffatome und gegebenenfalls Heteroatome aufweisender organischer Rest sein. Besonders bevorzugt weist der organische Rest Carbonylgruppen, wie Keto-, Ester- oder Säureamid-Gruppen auf. Der organische, gegebenenfalls Heteroatome aufweisende Rest kann insbesondere ein substituierter oder unsubstituierter, aliphatischer, alicyclischer, aliphatisch-alicyclischer, heterocyclischer, aliphatischheterocyclischer, aromatischer, aromatisch-aromatischer oder aliphatisch-aromatischer Kohlenwasserstoffrest mit 1 bis 50 Kohlenstoffatomen sein, wobei die substituierten Kohlenwasserstoffreste Substituenten, ausgewählt aus primären, sekundären oder tertiären Alkylgruppen, alicyclischen Gruppen, aromatischen Gruppen, -N(R¹)₂, -NHR¹,-NH₂, Fluor, Chlor, Brom, Jod, -CN, -C(O)-R¹, -C(O)H oder -C(O)O-R¹, -CF₃, -O-R¹,-C(O)N-R¹, -OC(O)-R¹ und/oder -Si(R¹)₃, mit R¹ gleich einem monovalenten, bevorzugt 1 bis 20 Kohlenstoffatome aufweisenden Kohlenwasserstoffrest, aufweisen können. Sind mehrere Kohlenwasserstoffreste R¹ vorhanden, so können diese gleich oder unterschiedlich sein. Die Substituenten sind vorzugsweise beschränkt auf solche, die keinen Einfluss auf die Reaktion selbst haben. Besonders bevorzugte Substituenten können ausgewählt sein aus den Halogenen, wie z. B. Chlor, Brom oder Jod, den Alkylresten, wie z. B. Methyl, Ethyl, Propyl, iso-Propyl, Butyl, sec-Butyl, t-Butyl, neo-Pentyl, sec-Amyl, t-Amyl, iso-Octyl, t-Octyl, 2-Ethylhexyl, iso-Nonyl, iso-Decyl oder Octadecyl, den Arylresten, wie z. B. Phenyl, Naphthyl oder Anthracyl, den Alkylarylresten, wie z. B. Tolyl, Xylyl, Dimethylphenyl, Diethylphenyl, Trimethylphenyl, Triethylphenyl oder p-Alkylphenyl, den Aralkylresten, wie z. B. Benzyl oder Phenylethyl, den alicyclischen Resten, wie z. B. Cyclopentyl, Cyclohexyl, Cyclooctyl, Cyclohexylethyl oder 1-Methylcyclohexyl, den Alkoxyresten, wie z. B. Methoxy, Ethoxy, Propoxy, Butoxy oder Pentoxy, den Aryloxyresten, wie z. B. Phenoxy oder Naphthoxy, -OC(O)R¹ oder -C(O)R¹, wie z. B. Acetyl, Propionyl, Trimethylacetoxy, Triethylacetoxy oder Triphenylacetoxy, und den drei Kohlenwasserstoffreste aufweisenden Silylresten -Si(R¹)₃, wie z. B. Trimethylsilyl, Triethylsilyl oder Triphenylsilyl. Besonders bevorzugt sind Verbindungen der Formel IIa, die als Reste R solche aufweisen, die einen 2,2,6,6,-Tetramethylpiperidin-Rest und gegebenenfalls eine weitere -N(R¹)₂, -NHR¹ und/oder-NH₂ Gruppe enthalten.

Als sekundäre Amine, die eine Struktureinheit gemäß Formel (I) aufweisen, können ganz besonders bevorzugt die nachfolgend aufgeführten Verbindungen mit den Strukturformeln (Ib) bis (Ig) oder deren Derivate eingesetzt werden. 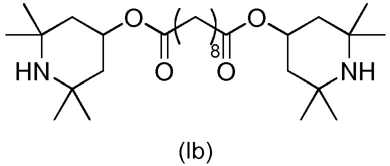 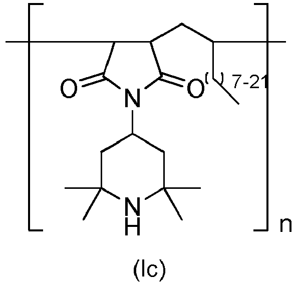 mit n = 1 bis 20, vorzugsweise 1 bis 10 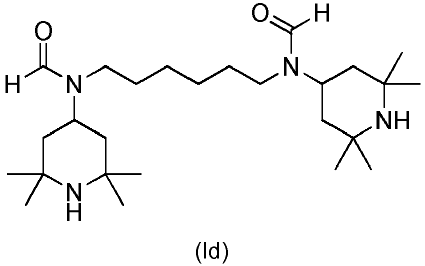 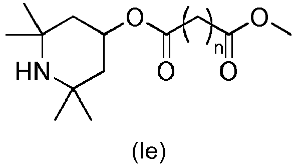 mit n = 1 bis 12, vorzugsweise 8 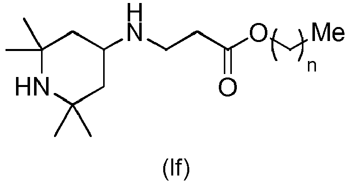 mit n = 1 bis 17, vorzugsweise 13

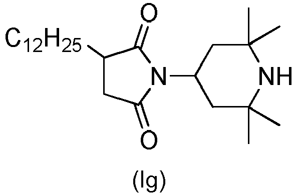

Es können auch Gemische, enthaltend zwei oder mehrere sterisch gehinderte Amine, eingesetzt werden.

Die Zusammensetzung umfasst ein zuvor beschriebenes Gemisch, welche zusätzlich zu dem Gemisch zumindest ein Amin mit einer 2,2,6,6-Tetramethylpiperidineinheit aufweist. Insbesondere wird im erfindungsgemäßen Verfahren das Amin mit der Formel (Ib), Sebacinsäuredi-4-(2,2,6,6-tetramethylpiperidinyl)ester, bevorzugt eingesetzt.
Ein besonders bevorzugtes Metall in der erfindungsgemäßen Zusammensetzung ist Rhodium.

Des Weiteren wird ein Verfahren zur Hydroformylierung von ungesättigten Verbindungen und deren Gemischen beansprucht.

Ein besonderer Vorteil der vorliegenden Erfindung liegt in der Verwendung der zuvor beschriebenen Komplexgemische (1b) und (2b), insbesondere (1c) und (2c), in der Hydroformylierung darin begründet, dass die erfindungsgemäße Verwendung der konstitutionsisomeren Bisphophite anstelle eines reinen Isomeren eine aufwendige und kostspielige Auftrennung der konstitutionsisomeren Bisphosphite überflüssig macht.
Aus dem Stand der Technik erwartete man eine herabgesetzte Reaktivität sowie geringere n/i-Selektivität aufgrund der Anwesenheit des unsymmetrischen konstitutionsisomeren Bisphosphit (1a), insbesondere dessen Derivat (1ca). Wie in den nachfolgenden Hydroformylierungsexperimenten offenbart wird, weisen die konstitutionsisomeren Bisphosphite (1a) und (2a) überraschenderweise neben hohen Reaktivitäten und n/i-Selektivitäten eine deutlich erhöhte Standzeit gegenüber den aus dem Stand der Technik bekannten Bisphosphiten auf.

Das erfindungsgemäße Verfahren zur Hydroformylierung einer ungesättigten Verbindung oder eines Gemisches ungesättigter Verbindungen umfasst die Schritte:
a) Vorlegen einer Verbindung nach den Formeln (1a) und (2a), (1b) und (2b) und/oder (1 c) und (2c) oder einer Zusammensetzung enthaltend die Verbindungen der Formeln (1 a) und (2a), (1 b) und (2b) und (1 c) und (2c) zusammen mit einer weiteren Komponente ausgewählt aus Basen, organischen Aminen, Epoxiden, Pufferlösungen, Ionenaustauschern;
b) Einleiten eines Gasgemisches umfassend Kohlenmonoxid und Wasserstoff;
c) Zugabe mindestens einer ungesättigten Verbindung oder eines Gemisches von ungesättigten Verbindungen.

Die ungesättigten Verbindungen, welche in dem erfindungsgemäßen Verfahren hydroformyliert werden, umfassen Kohlenwasserstoffgemische, die in petrochemischen Verarbeitungsanlagen anfallen. Hierzu gehören beispielsweise sogenannte C₄-Schnittte. Typische Zusammensetzungen von C₄-Schnitten, aus denen der größte Teil der mehrfach ungesättigten Kohlenwasserstoffe entfernt worden ist und die im erfindungsgemäßen Verfahren eingesetzt werden können, sind in der folgenden Tabelle 1 aufgelistet (siehe DE 10 2008 002188).

**Tabelle 1:**

| | Dampfspaltanlage | | Dampfspaltanlage | | Katalytische Spaltanlage | |
|---|---|---|---|---|---|---|
| Komponente | HCC₄ | HCC₄ / SHP | Raff. I | Raff. I / SHP | CC₄ | CC₄ / SHP |
| Isobutan [Massen-%] | 1 - 4.5 | 1 - 4.5 | 1.5 - 8 | 1.5 - 8 | 37 | 37 |
| n-Butan [Massen-%] | 5 - 8 | 5 - 8 | 6 - 15 | 6 - 15 | 13 | 13 |
| E-2-Buten [Massen-%] | 18 - 21 | 18 - 21 | 7 - 10 | 7 - 10 | 12 | 12 |
| 1-Buten [Massen-%] | 35 - 45 | 35 - 45 | 15 - 35 | 15 - 35 | 12 | 12 |
| Isobuten [Massen-%] | 22 - 28 | 22 - 28 | 33 - 50 | 33 - 50 | 15 | 15 |
| Z-2-Buten [Massen-%] | 5 - 9 | 5 - 9 | 4 - 8 | 4 - 8 | 11 | 11 |
| 1,3-Butadien [Massen-ppm] | 500 - 8000 | 0 - 50 | 50 - 8000 | 0 - 50 | < 10000 | 0 - 50 |

Erläuterung:
- HCC₄: typisch für eine C₄ Mischung, die aus dem C₄-Schnitt einer Dampfspaltanlage (High Severity) nach der Hydrierung des 1,3-Butadiens ohne zusätzliche Moderation des Katalysators erhalten wird.
- HCC₄ / SHP: Zusammensetzung HCC₄, bei dem Reste an 1,3-Butadien in einem Selektivhydrierungsprozess/SHP weiter reduziert wurden.
- Raff. I (Raffinat I): typisch für eine C₄ Mischung, die aus dem C₄-Schnitt einer Dampfspaltanlage (High Severity) nach der Abtrennung des 1,3-Butadiens, beispielsweise durch eine NMP-Extraktivrektifikation, erhalten wird.
- Raff. I / SHP: Zusammensetzung Raff. I, bei dem Reste an 1,3-Butadien in einem Selektivhydrierungsprozess/SHP weiter reduziert wurden.
- CC₄: typische Zusammensetzung eines C₄-Schnitts, das aus einer katalytischen Spaltanlage erhalten wird.
- CC₄ / SHP: Zusammensetzung eines C₄-Schnitts, bei dem Reste an 1,3-Butadien in einem Selektivhydrierungsprozess/SHP weiter reduziert wurden.

In einer Variante des Verfahrens ist die ungesättigte Verbindung oder deren Gemisch ausgewählt aus:
- Kohlenwasserstoffgemischen aus Dampfspaltanlagen;
- Kohlenwasserstoffgemischen aus katalytisch betriebenen Spaltanlagen, wie z.B. FCC-Spaltanlagen;
- Kohlenwasserstoffgemischen aus Oligomerisierungsprozessen in homogener Phase sowie heterogenen Phasen, wie z.B. dem OCTOL-, DIMERSOL.-, Fischer-Tropsch-, Polygas-, CatPoly-, InAlk-, Polynaphtha-, Selectopol-, MOGD-, COD-, EMOGAS-, NExOCTANE- oder SHOP-Prozess;
- Kohlenwasserstoffgemischen umfassend mehrfach ungesättigte Verbindungen;
- ungesättigten Carbonsäurederivaten.

In einer Variante des Verfahrens weist das Gemisch ungesättigte Verbindungen mit 2 bis 30 Kohlenstoffatomen auf.

In einer besonderen Variante des Verfahrens weist das Gemisch ungesättigte Verbindungen mit 2 bis 8 Kohlenstoffatomen auf.

In einer weiteren Variante des Verfahrens weist das Gemisch mehrfach ungesättigte Kohlenwasserstoffe auf. In einer besonderen Ausführungsform umfasst das Gemisch Butadiene.

Die ungesättigten Verbindungen, welche in dem erfindungsgemäßen Verfahren hydroformyliert werden, umfassen weiterhin ungesättigte Carbonsäurederivate. In einer besonderen Ausführungsform sind diese ungesättigten Carbonsäurederivate ausgewählt unter Fettsäureestern.

Die Durchführung des erfindungsgemäßen Verfahrens erfolgt in unterschiedlichen Ausführungsformen, welche in den Beispielen im Detail offenbart werden.

Das erfindungsgemäße mehrphasige Reaktionsgemisch umfasst neben einem aus Kohlenmonoxid und Wasserstoff bestehenden Gasgemisch mindestens eine ungesättigte Verbindung, wie sie zuvor offenbart wurde und umfasst neben Kohlenwasserstoffgemischen, welche aus Dampfspalt-, katalytisch betriebenen Spaltanlagen oder Oligomerisierungsprozessen stammen oder andere Quellen von einfach ungesättigten und/oder mehrfach ungesättigten Kohlenstoffverbindungen oder ungesättigte Carbonsäurederivate beinhalten, mindestens ein Hydroformylierungsprodukt dieser ungesättigten Verbindungen, wie sie in den nachfolgenden Beispielen aufgeführt sind und die jeweils verwendete Zusammensetzung, wie sie zuvor offenbart wurde.

### Figurenbeschreibung: Berechnung der Komplexverbindung (1ca)

Die erfindungsgemäßen Komplexverbindungen der Formeln (1c) und (2c) werden in situ während der Hydroformylierungsreaktion gebildet.

In einer besonderen Ausführungsform der Erfindung liegen die Komplexverbindungen (1c) und (2c) neben den ungebundenen konstitutionsisomeren Bisphosphiten (1a) und (2a) vor.

Die Charakterisierung des Hydridocarbonylkomplexes des Liganden (1a) mit Rhodium als Metall, der erfindungsgemäßen Verbindung (1ca), erfolgte mittels theoretischer Berechnungen. Das Ergebnis ist in der Figur 1 im Anhang dargestellt.

Die Strukturberechnung wurde mit dem BP86-Funktional und dem def-SV(P)-Basissatz durchgeführt.
Die Strukturberechnungen für die Modellstrukturen erfolgten mit dem Turbomole-Programmpaket (R. Ahlrichs, M. Bär, M. Häser, H. Horn, C. Kölmel, Chem. Phys. Lett., 1989, 162, 16; TURBOMOLE V6.3 2011, a development of University of Karlsruhe and Forschungszentrum Karlsruhe GmbH, 1989-2007, TURBOMOLE GmbH, since 2007. http://www.turbomole.com) auf Basis der Dichtefunktionaltheorie (DFT). Verwendet wurde das BP86-Funktional (S. H. Vosko, L. Wilk, M. Nusair, Can. J. Phys. , 1980, 58, 1200; A. D. Becke, Phys. Rev. A, 1988, 38, 3098; J. Perdew, Phys. Rev. B, 1986, 33, 8822) und der def-SV(P)-Basissatz (A. Schäfer, H. Horn and R. Ahlrichs, J. Chem. Phys., 1992, 97, 2571).

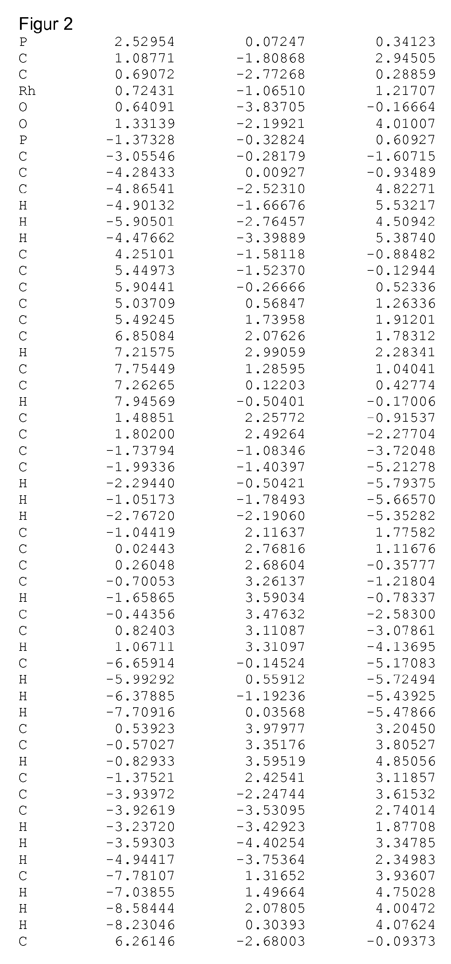

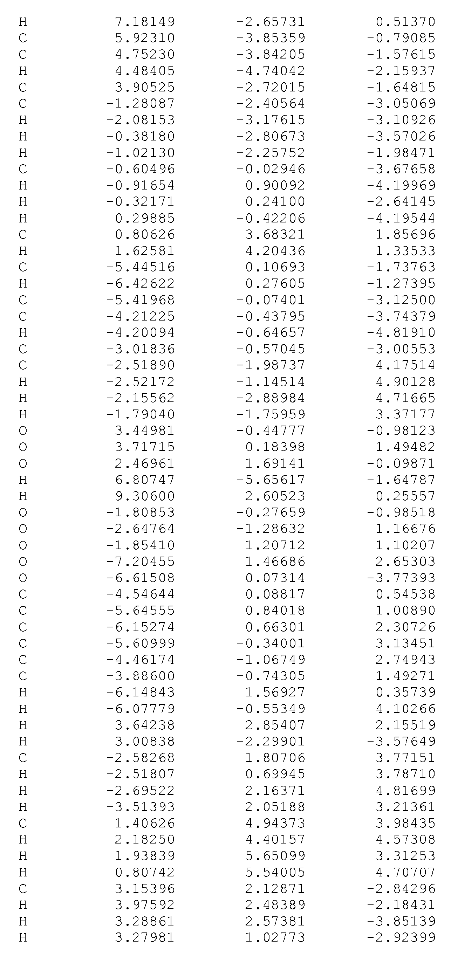

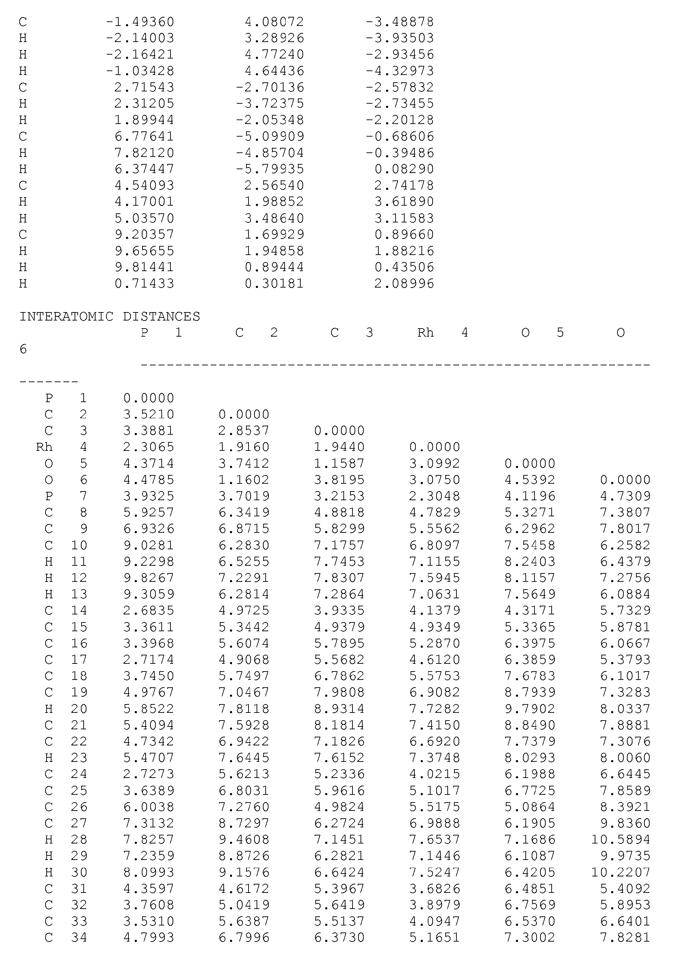

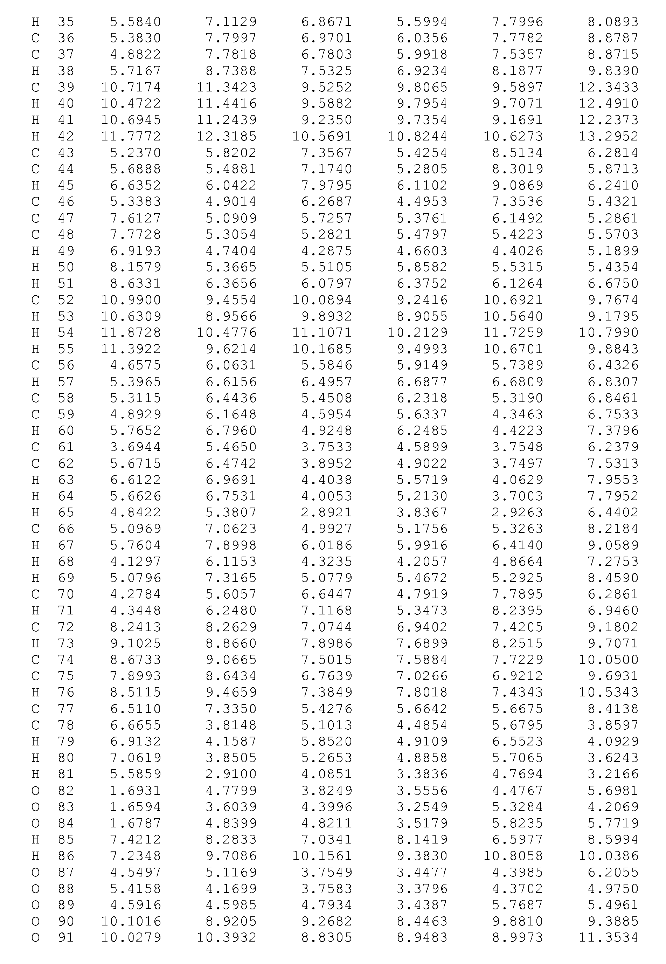

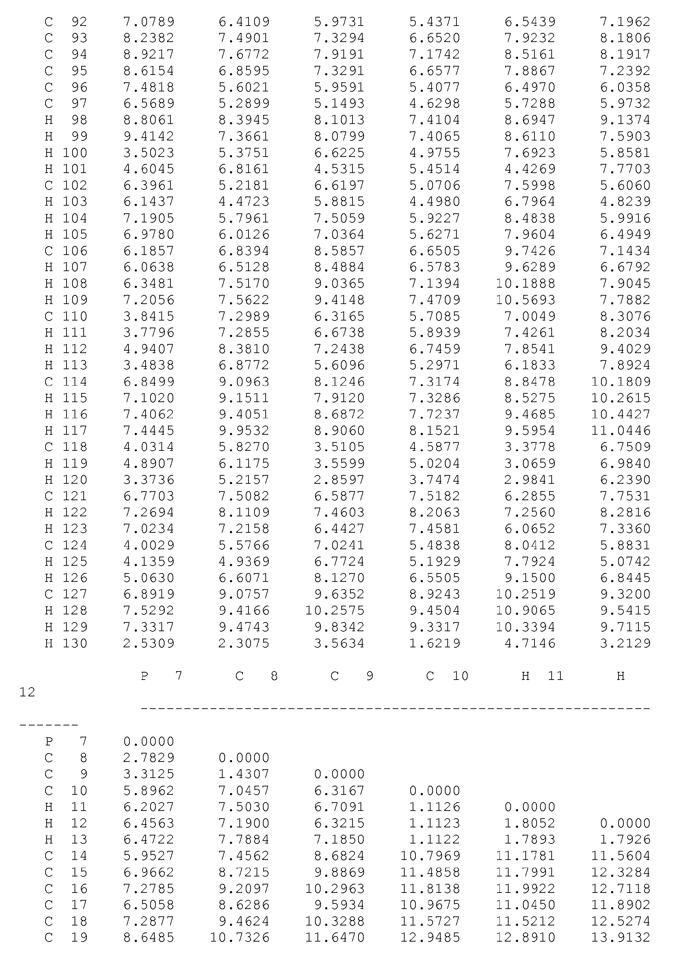

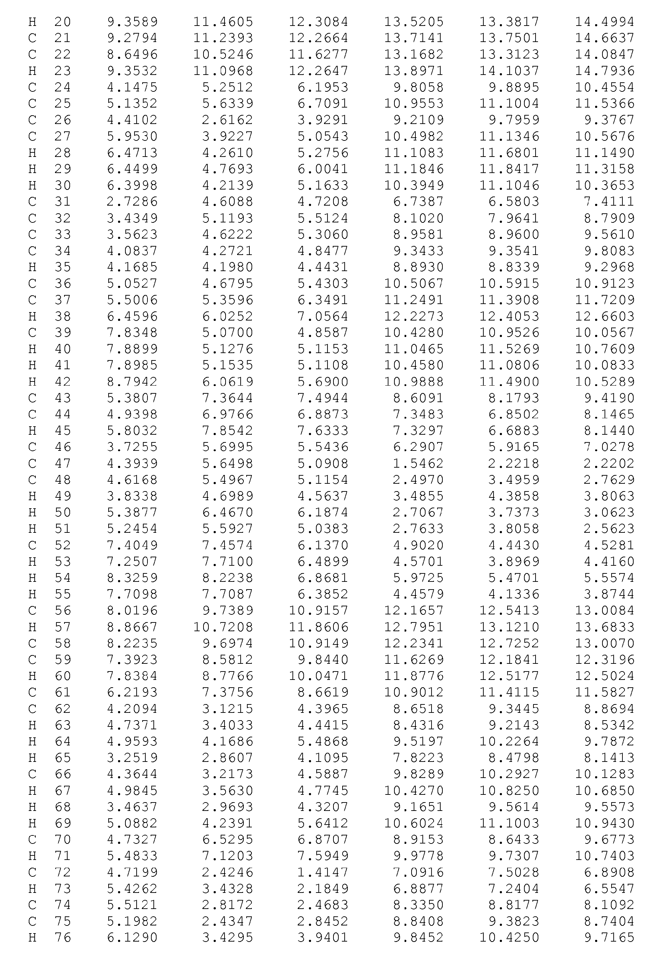

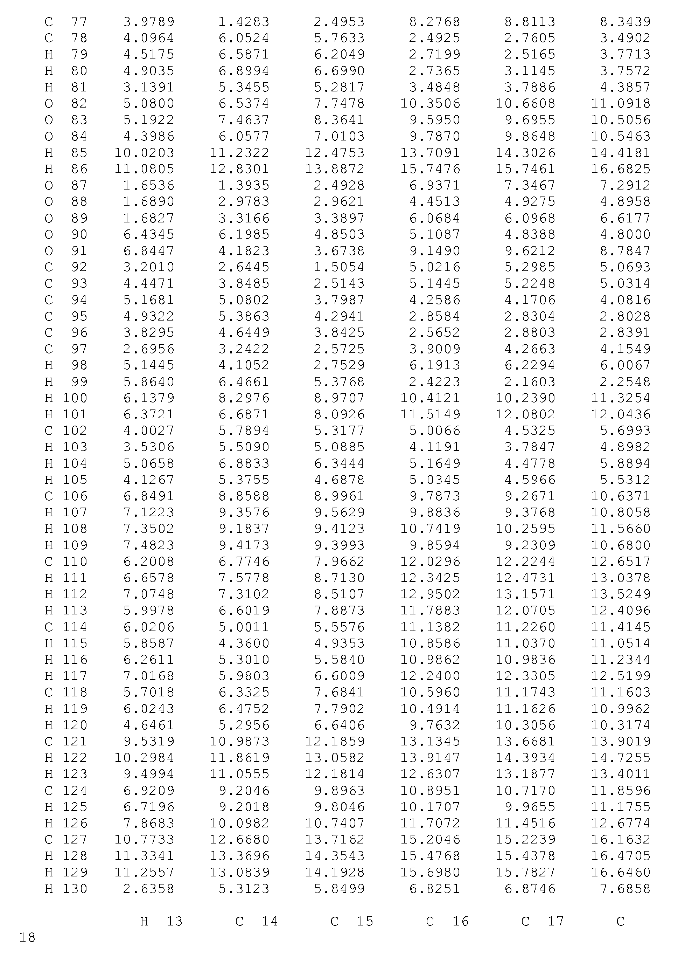

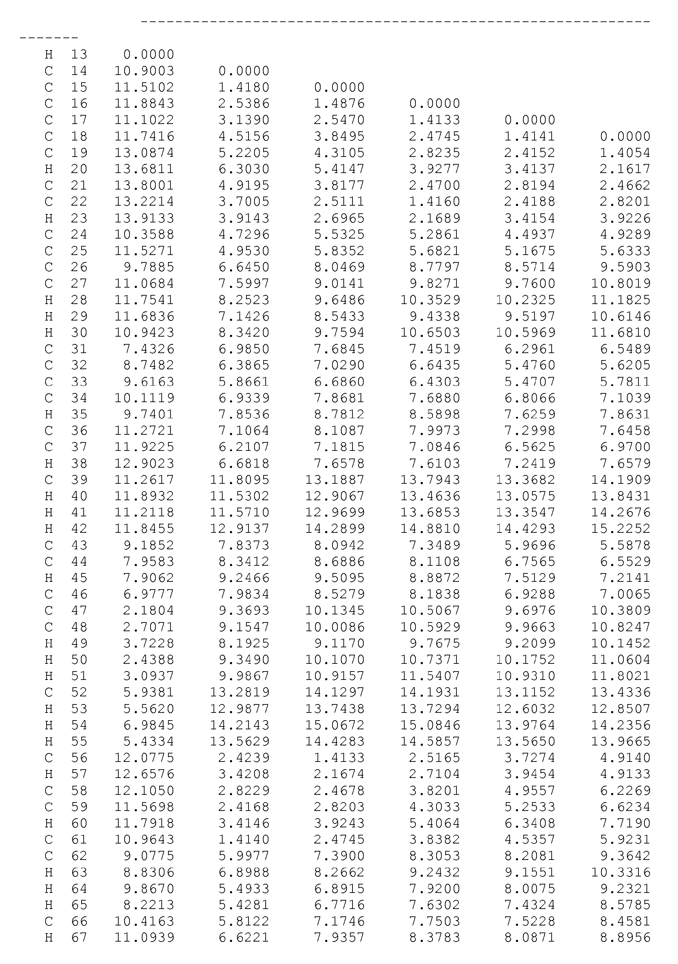

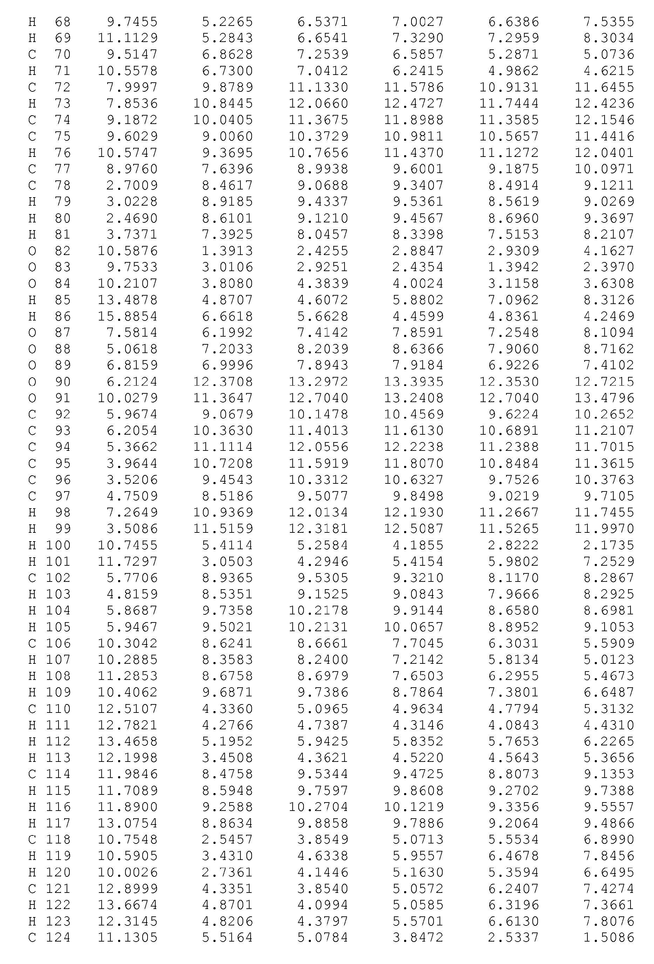

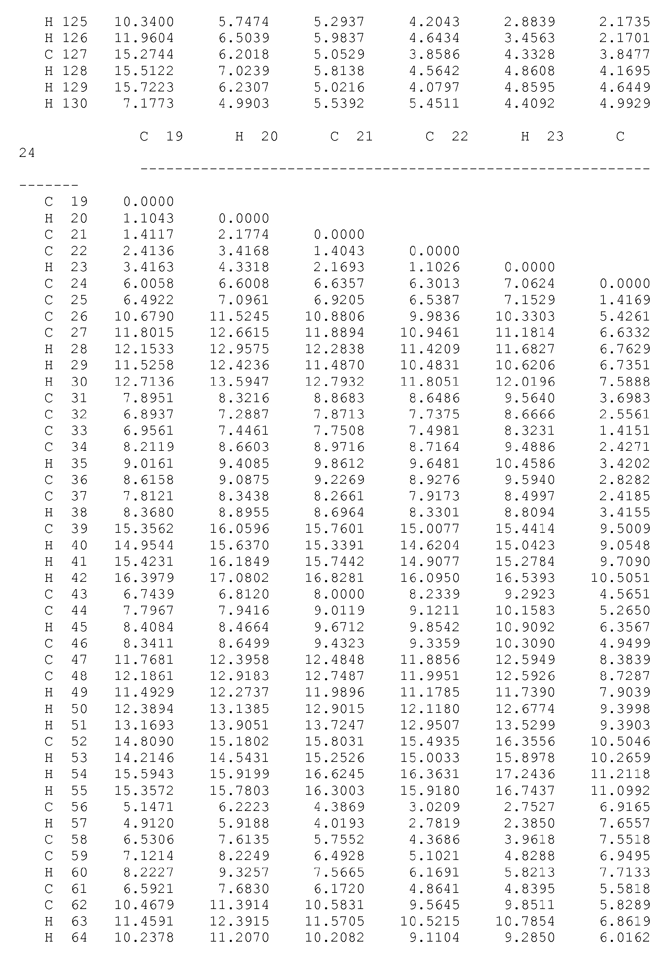

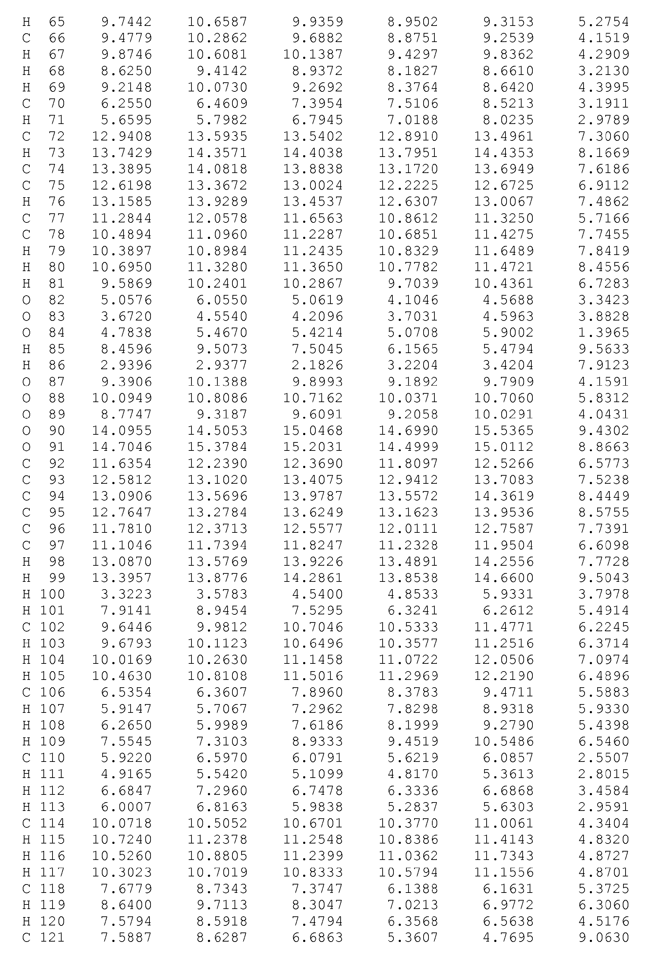

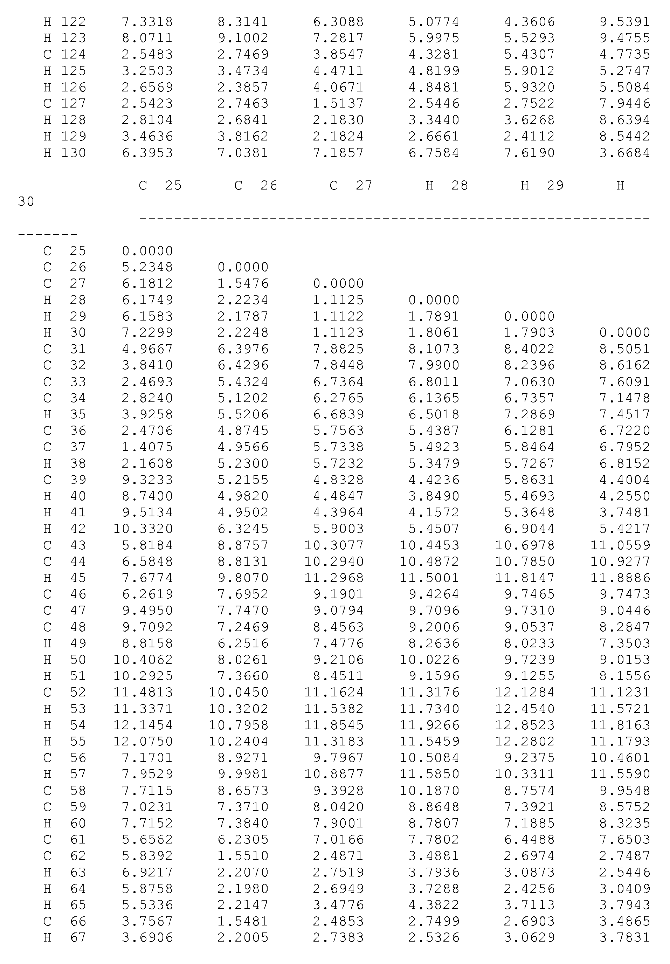

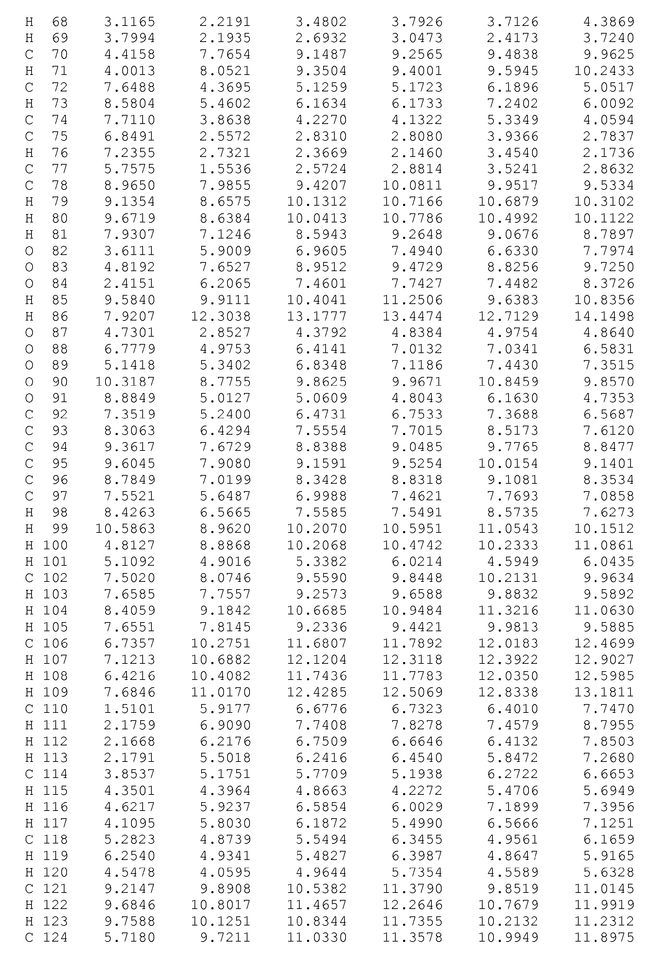

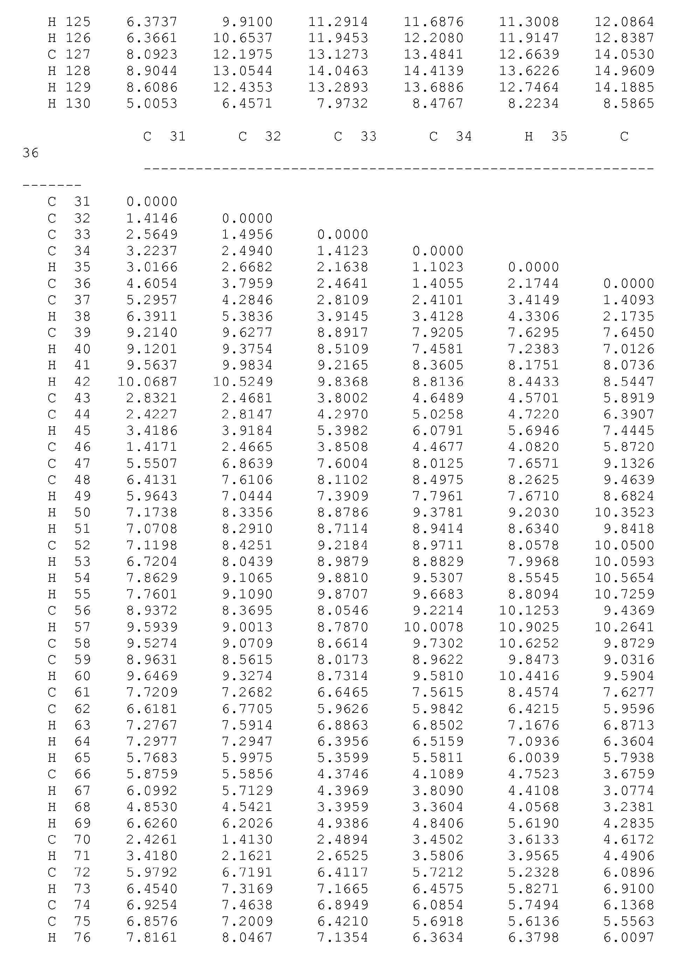

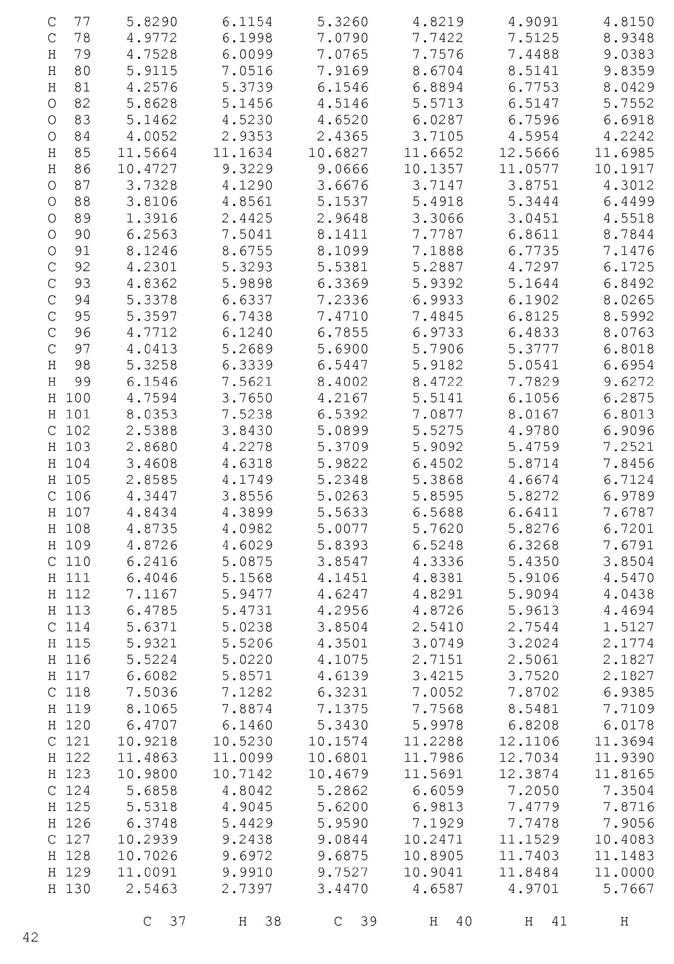

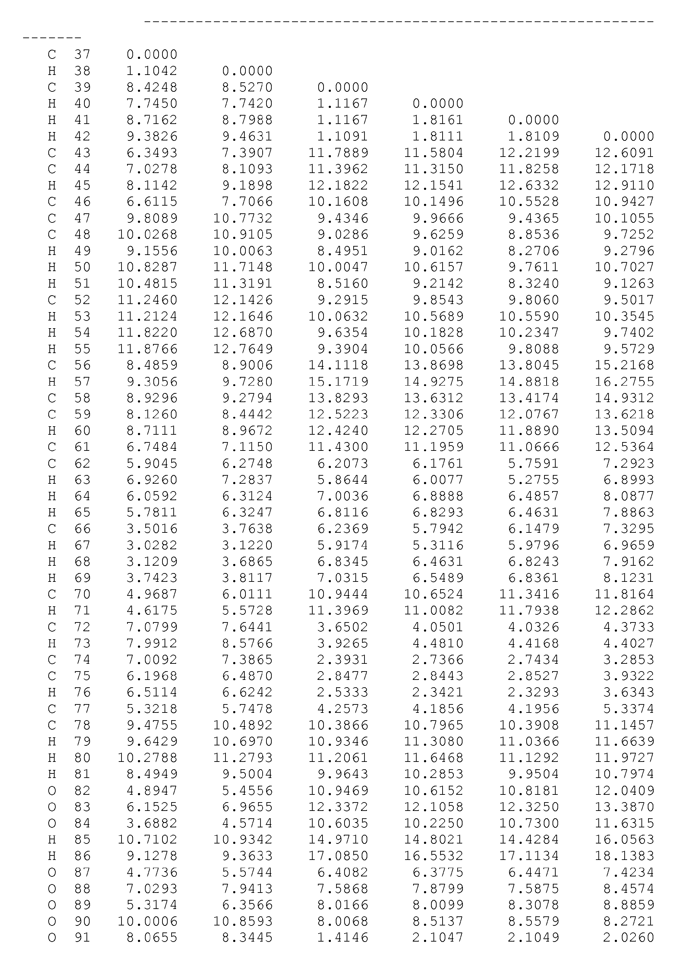

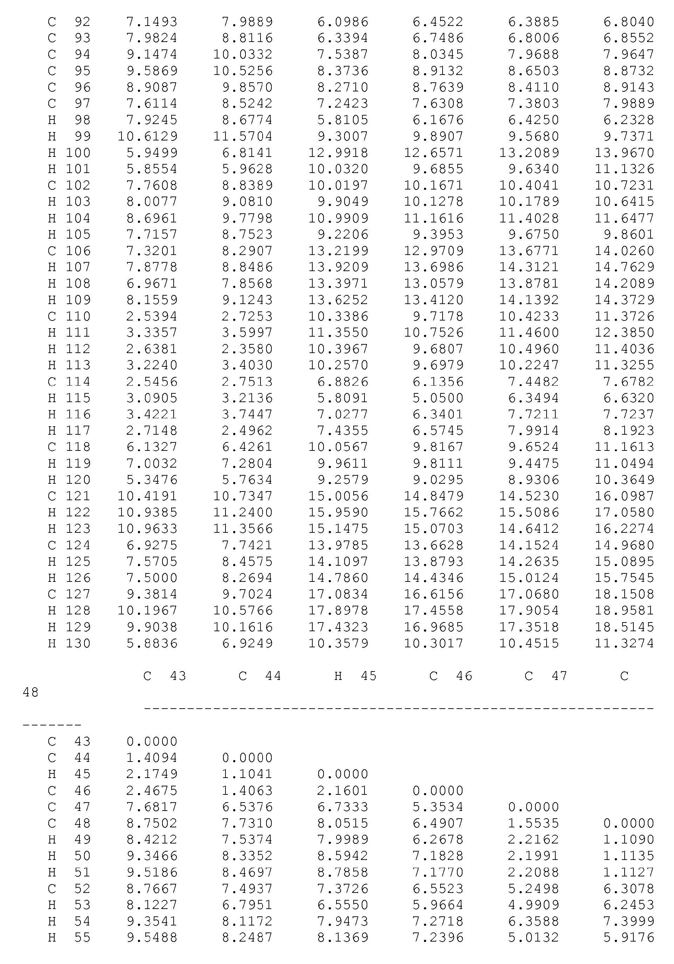

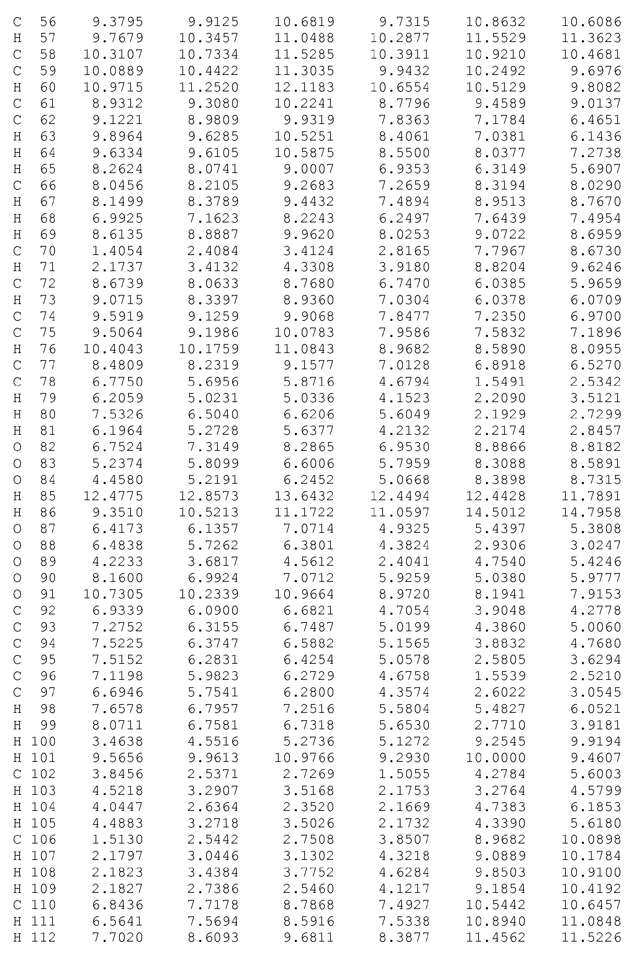

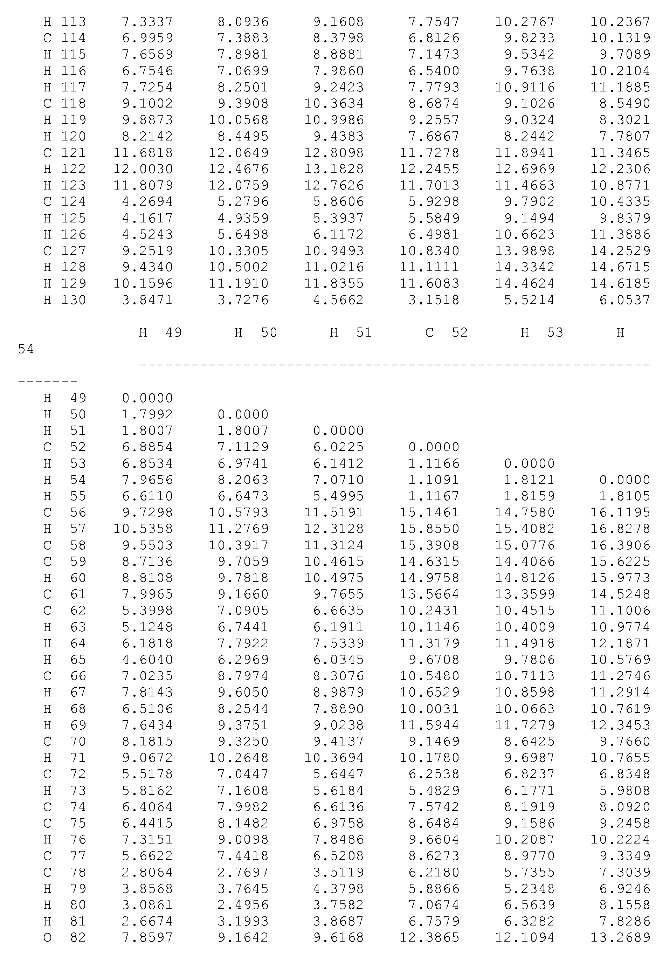

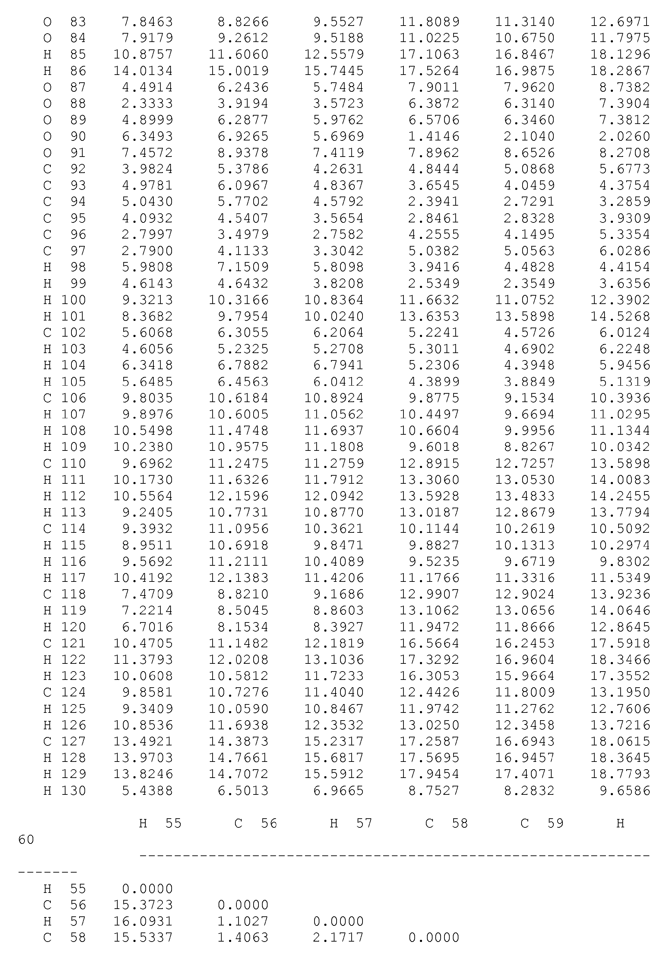

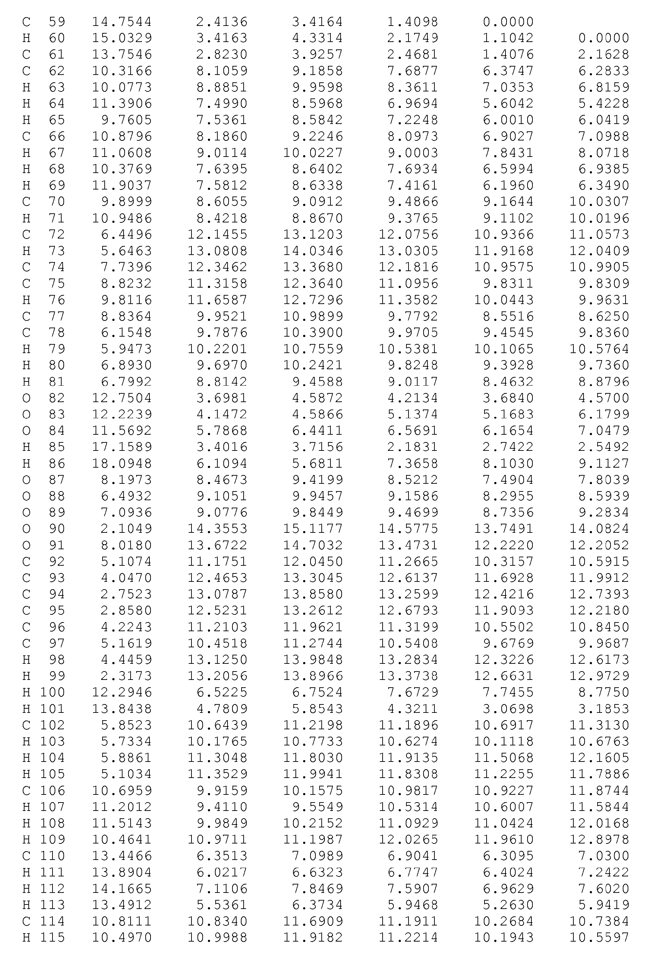

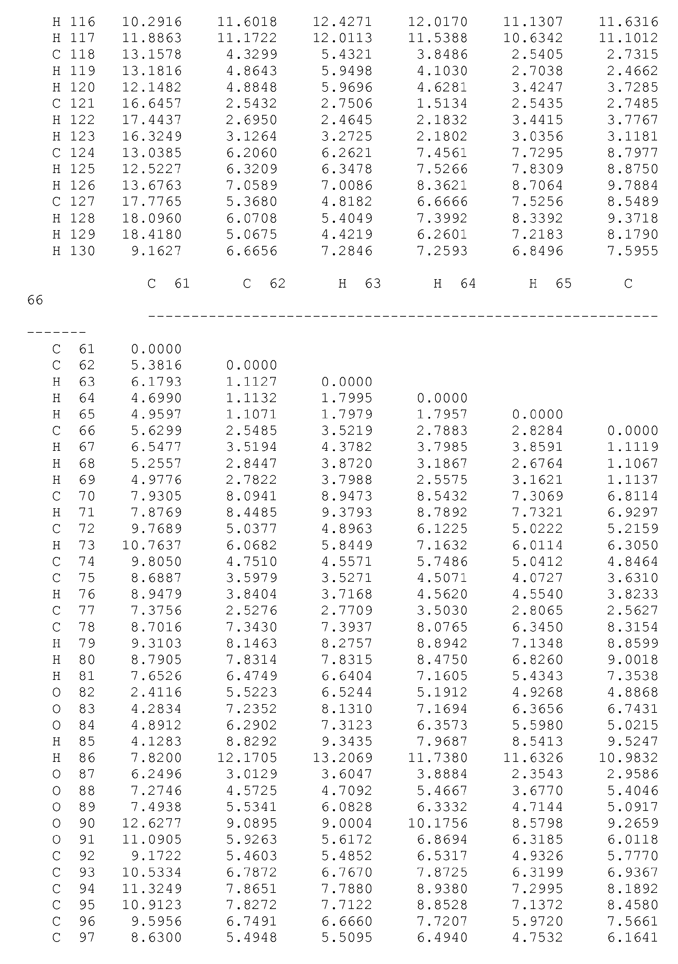

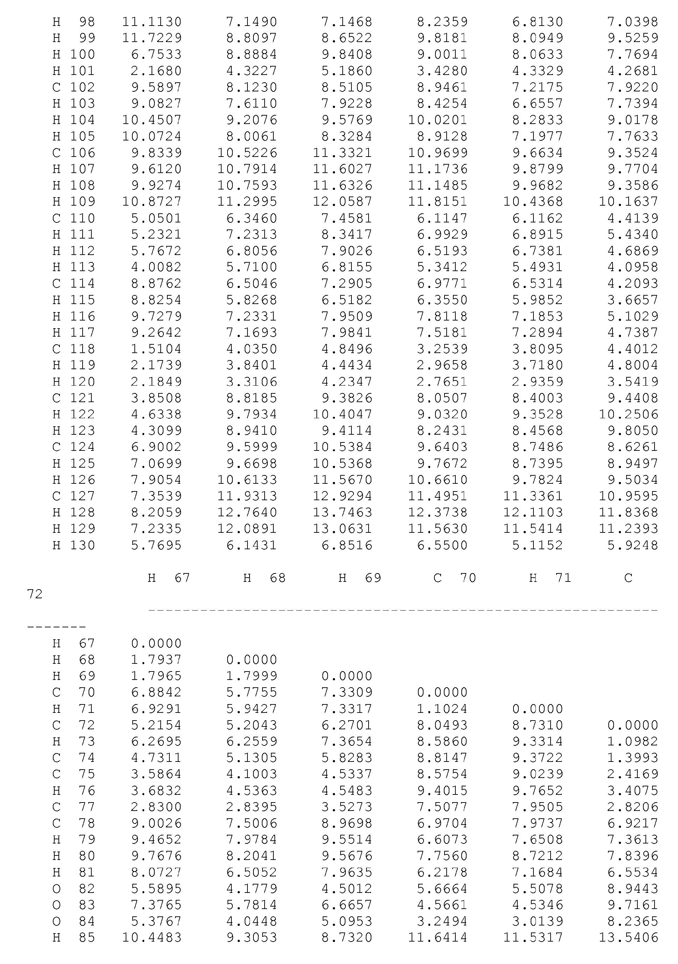

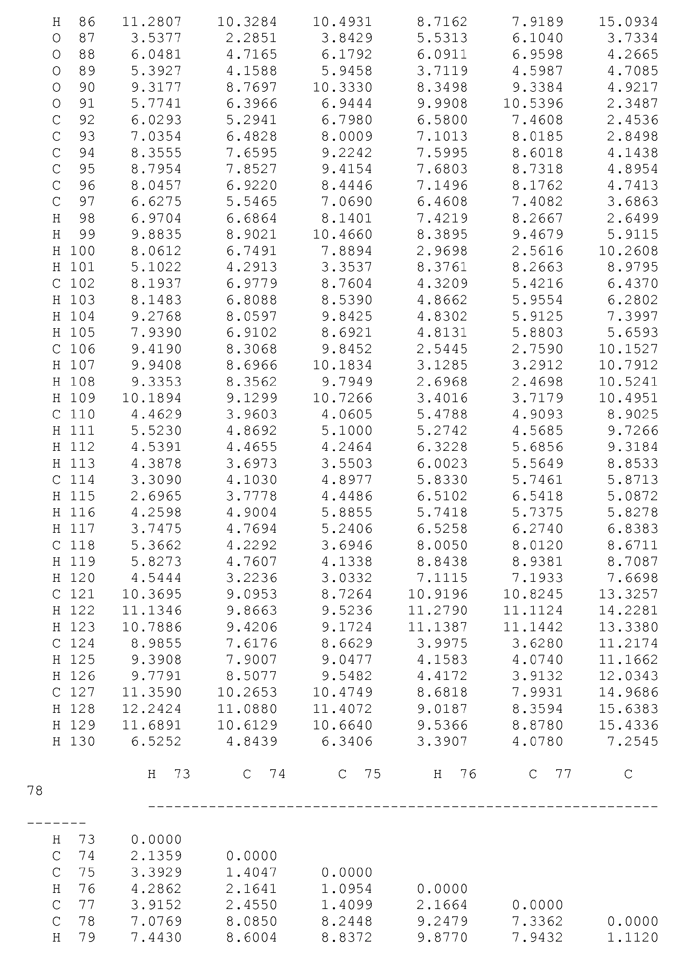

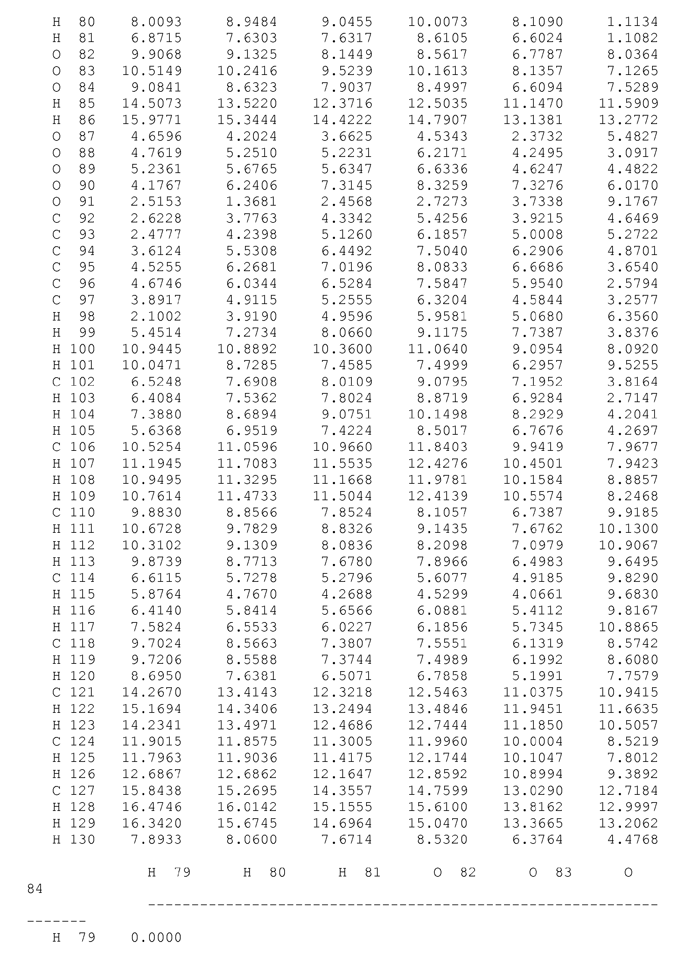

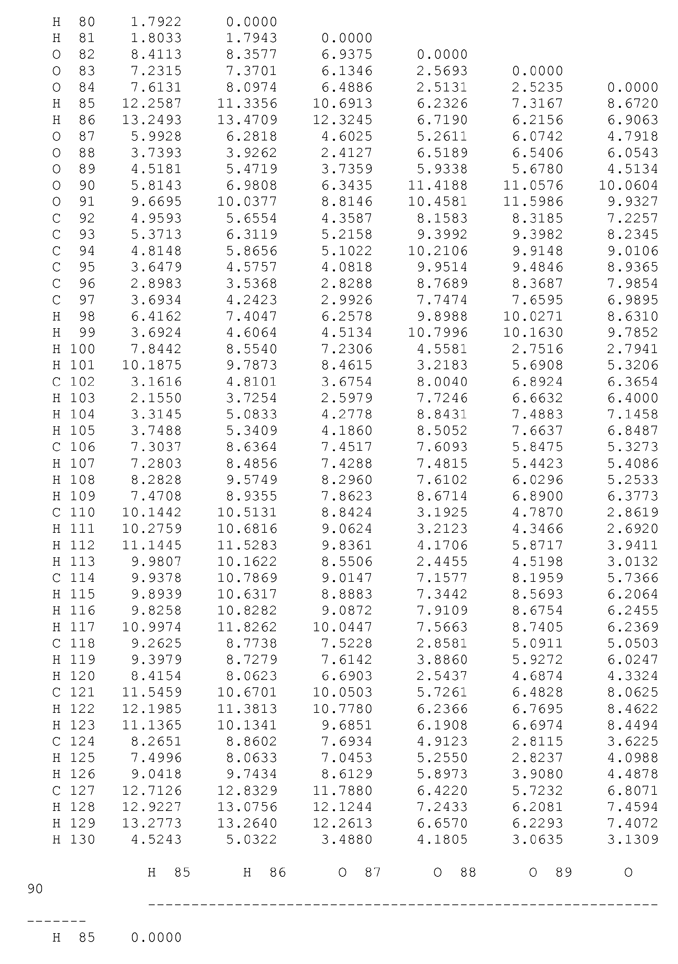

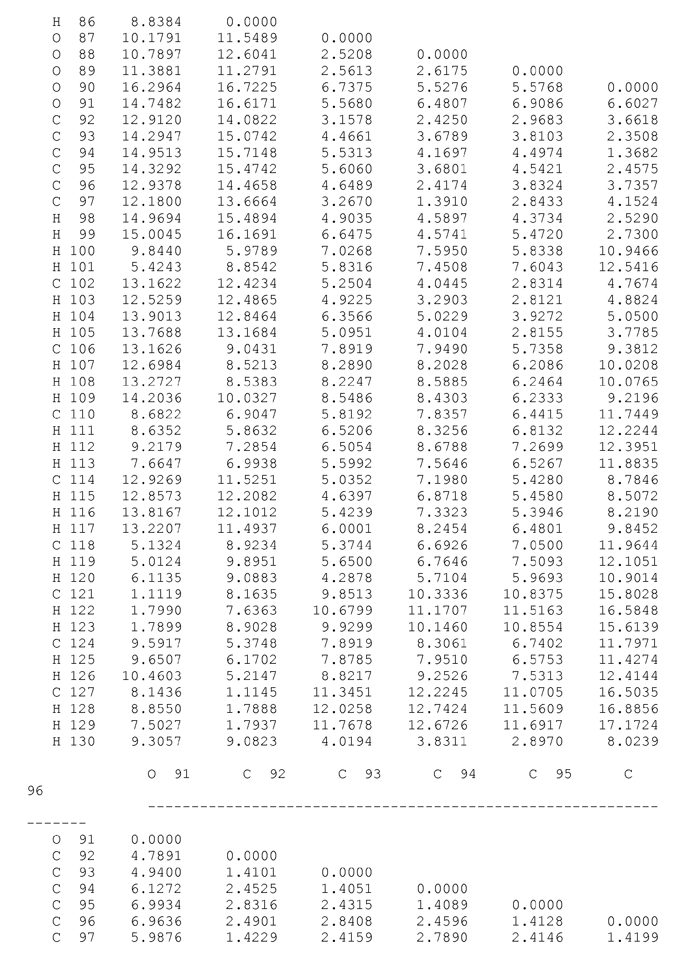

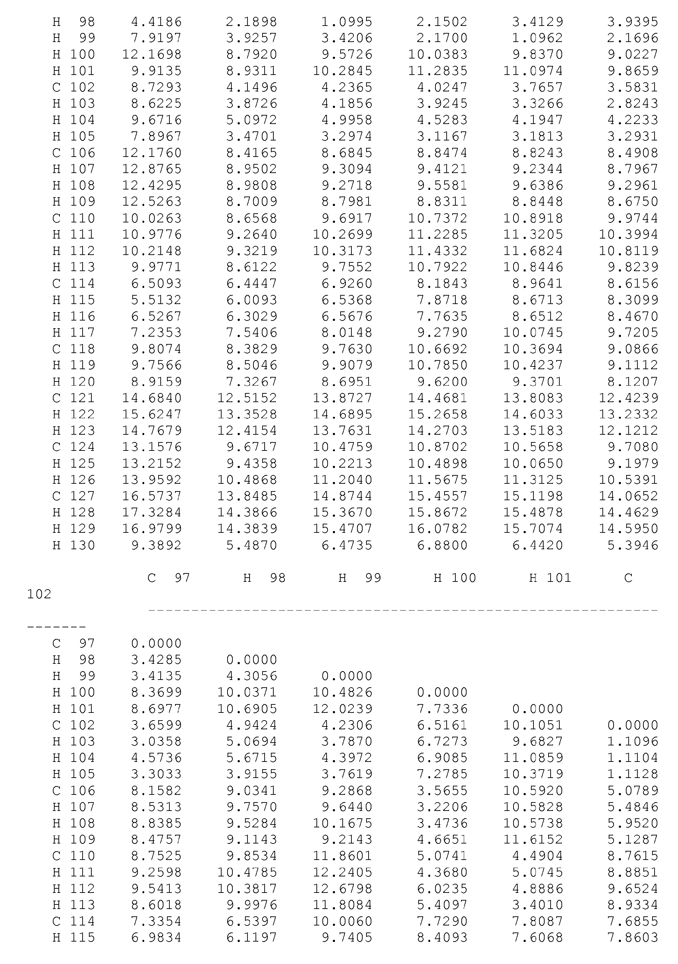

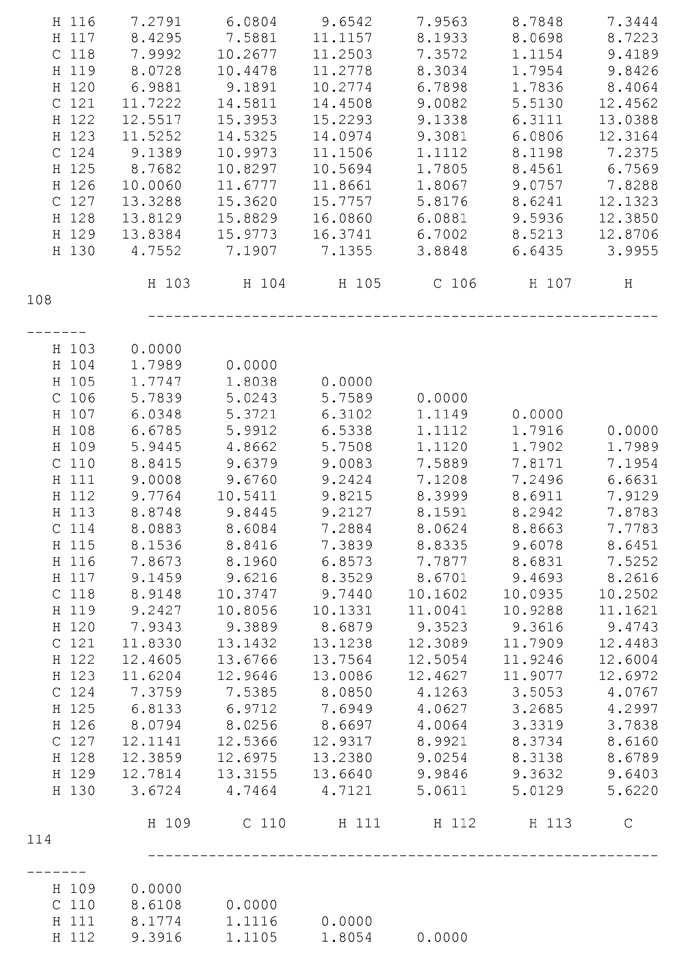

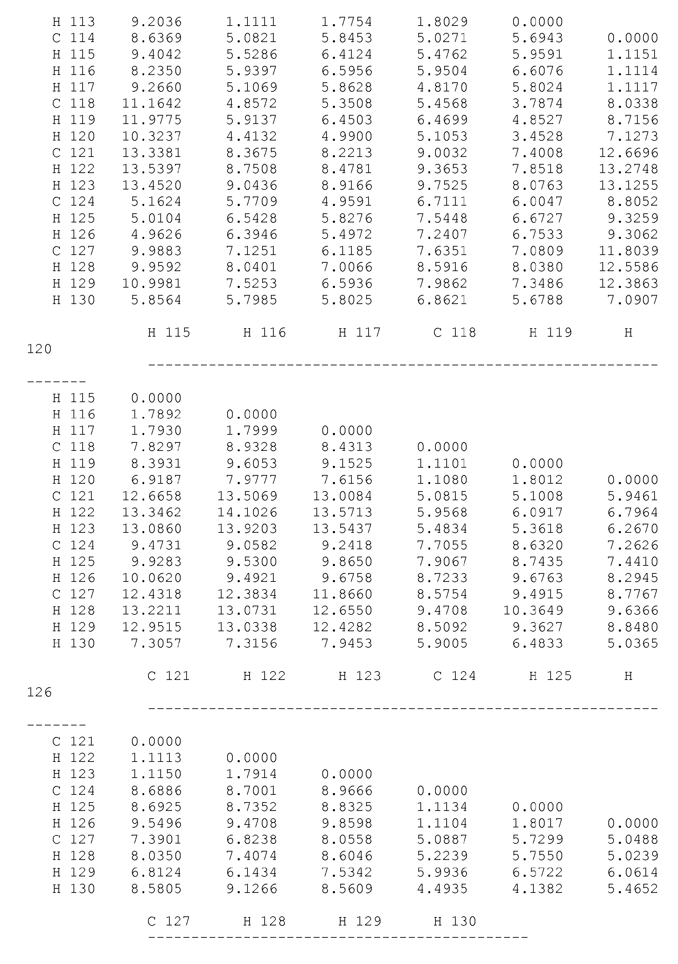

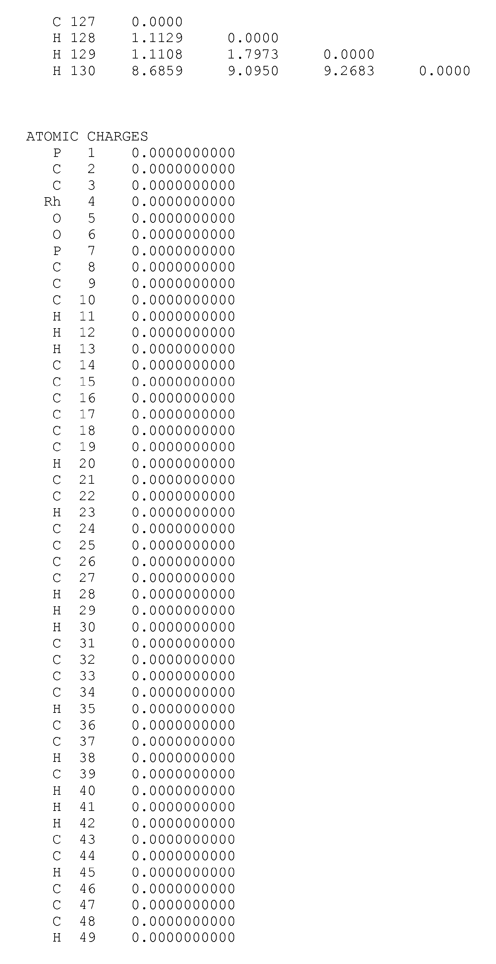

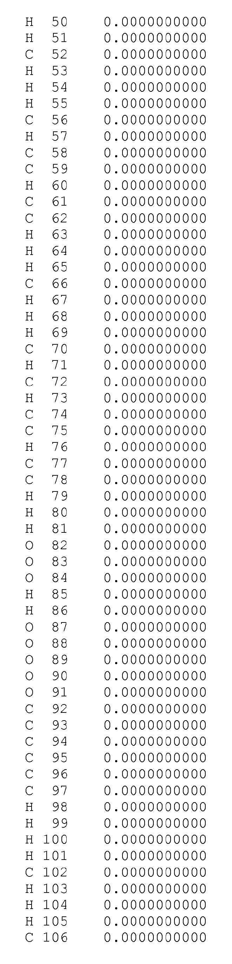

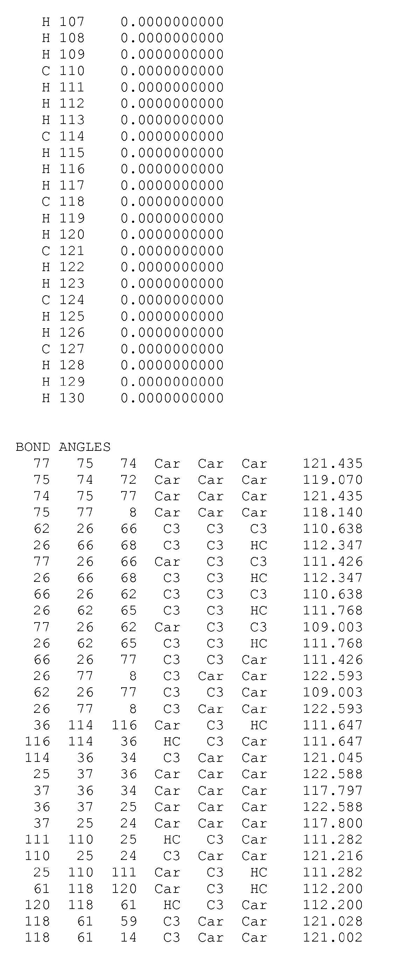

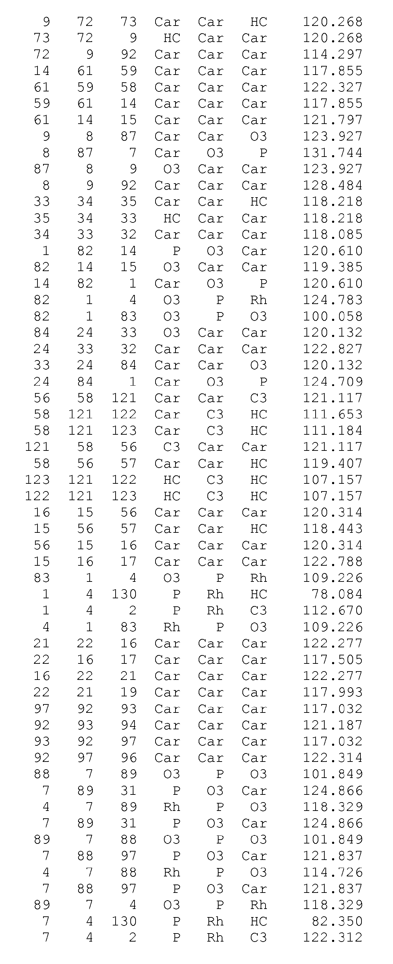

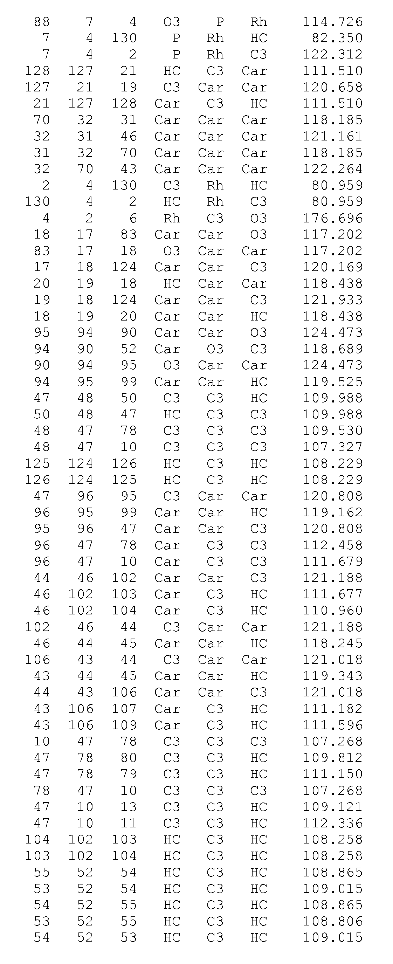

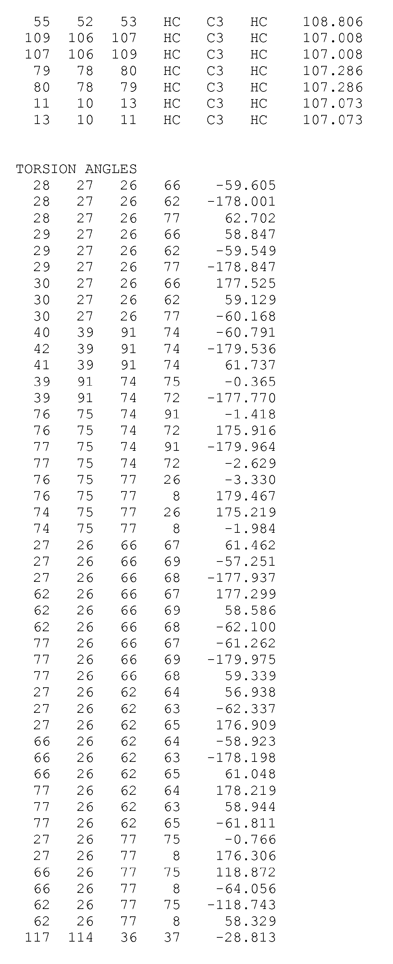

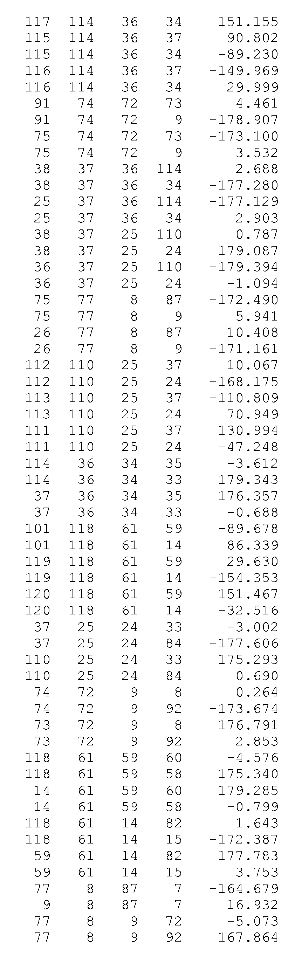

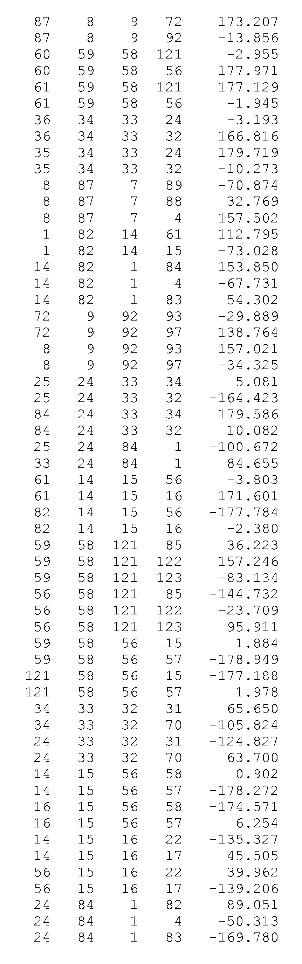

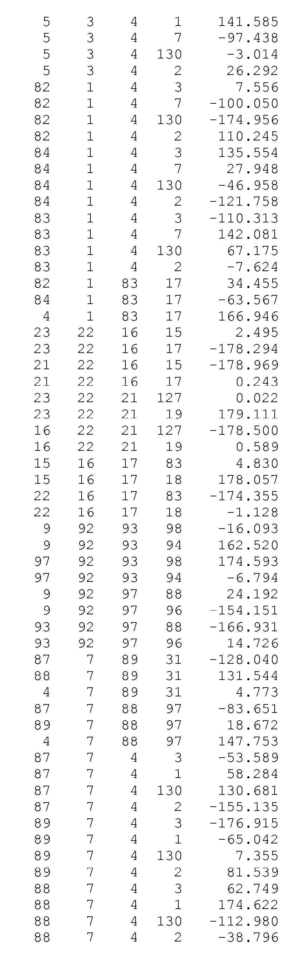

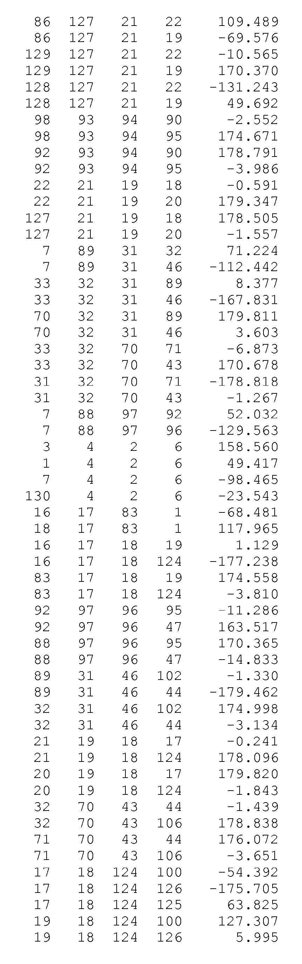

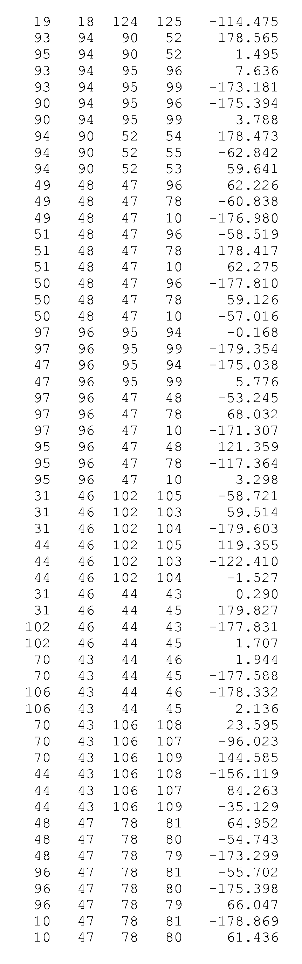

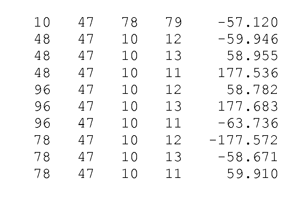

Figur 2 im Anhang liefert alle berechneten Koordinaten, Abstände und Winkel der Verbindung (1ca).

### Beispiele

### Allgemeine Reaktionsgleichung

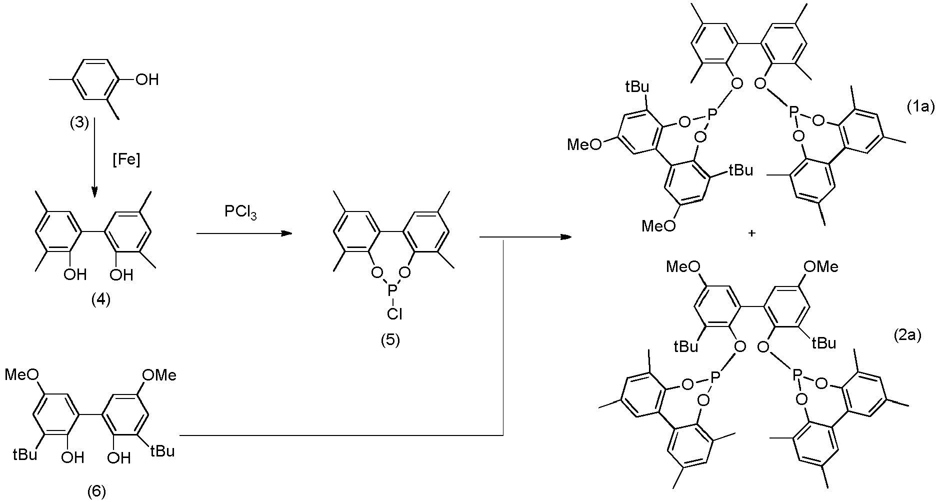

### Abkürzungen:

VE-Wasser = demineralisiertes Wasser
KPG = Kerngezogenes Präzisions-Glasgerät
ACN = Acetonitril
EtOAc = Ethylacetat
DMAB = Dimethylaminobutan
NMP = N-Methylpyrrolidon
ÖV = Ölvakuum
acac = acetylacetonat
NEt₃ = Triethylamin
TIPB = 1,2,4,5-Tetraisopropylbenzol

### Synthese des 2,2'-Bis(3,5-dimethylphenol) (4)

Das als Vorstufe eingesetzte Biphenol (4) wurde nach folgender Synthesevorschrift hergestellt.

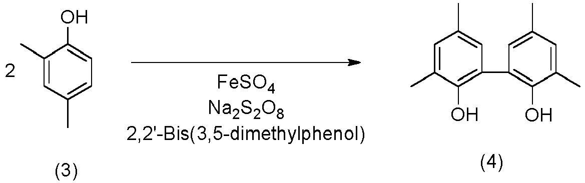

In einem 500 ml Schlenk mit KPG-Rührer, Zwischenaufsatz und Glasrührer wurden 1,42 g (0,005 mol) Eisen(II)-sulfatheptahydrat und 12,35 g (0,1 mol) 2,4-Dimethylphenol in 150 ml VE-Wasser und 5 ml Cyclohexan vorgelegt und auf 40 °C erwärmt.
In einem 100 ml Becherglas löste man 25.36 g (0,146 mol) Natriumperoxodisulfat in 80 ml VE-Wasser. Zum Start der Reaktion wurde eine kleine Portion Na₂S₂O₈-Lösung zum Phenol gegeben. Anschließend wurde alle 10 min eine kleinere Portion der Lösung hinzugegeben. Nach 30 min war die Na₂S₂O₈-Lösung hinzugegeben.
Nach einer Reaktionszeit von 5h wurde zur Reaktionslösung 300 ml Cyclohexan und 200 ml Wasser hinzugegeben, 20 min rühren gelassen, dann warm in den Scheidetrichter überführt.
Die organische Phase wurde abgetrennt und bis zur Trockene eingeengt. Das Produkt konnte in 69%iger Ausbeute (10,6g) erhalten werden.

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).
Die Charakterisierung des Produktes erfolgte mittels NMR-Spektroskopie (Bruker Avance 500 MHz FT-NMR-Spektrometer). Chemische Verschiebungen (δ) werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, and Pierre Granger, Pure Appl. Chem., 2001, 73, 1795 - 1818; Robin K. Harris, Edwin D. Becker, Sonia M. Cabral de Menezes, Pierre Granger, Roy E. Hoffman and Kurt W. Zilm, Pure Appl. Chem., 2008, 80, 59-84). Mittels der ³¹P-NMR wurde das Mengenverhältnis der beiden Liganden (Ligand (1a) und Ligand (2a)) zueinander bestimmt. Der unsymmetrische Ligand (1a) wird durch zwei Phosphorsignale im Bereich von (δ) = 140,6 ppm bis (δ) = 142,8 ppm charakterisiert, wohingegen für der symmetrischen Ligand (2a) nur ein Phosphorsignal im Bereich (δ) = 139, 1 ppm bis (δ) = 139,8 ppm aufweist.

### Synthese des 2,2'-Bis-(3,5-dimethylphenol)chlorophosphits (5)

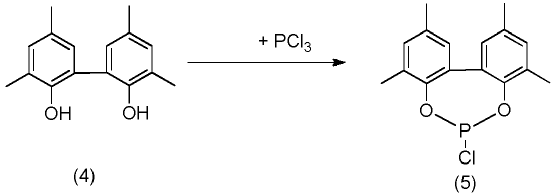

In einem sekurierten 2 L Schlenk mit Magnetrührer wurden 440 ml (692.56 g) Phosphortrichlorid vorgelegt. In einem zweiten sekurierten 1 L Schlenk wurden 120 g 2,2'-Bis-(3,5-dimethylphenol) eingewogen und unter Rühren 500 ml getrocknetes Toluol hinzugefügt. Die Biphenol-Toluol-Suspension wurde innerhalb von 4 h bei 63°C zum Phosphortrichlorid dosiert. Nach vollständiger Zugabe wurde die Reaktionsmischung über Nacht bei Temperatur gerührt. Am nächsten Morgen wurde die Lösung in der Wärme (45°C) eingeengt und das Produkt konnten in 96,5%iger Ausbeute (153 g) erhalten werden. ³¹P-NMR: 175,59 (94,8% 2,2'-Bis-(3,5-dimethylphenol)chlorophosphit), 4,4% diverse PCI-Verbindungen, 0,8% P-H-Verbindung.

### Erfindungsgemäße Synthesevarianten zur Herstellung des Isomerengemisches, bestehend aus den Liganden (1a) und (2a):

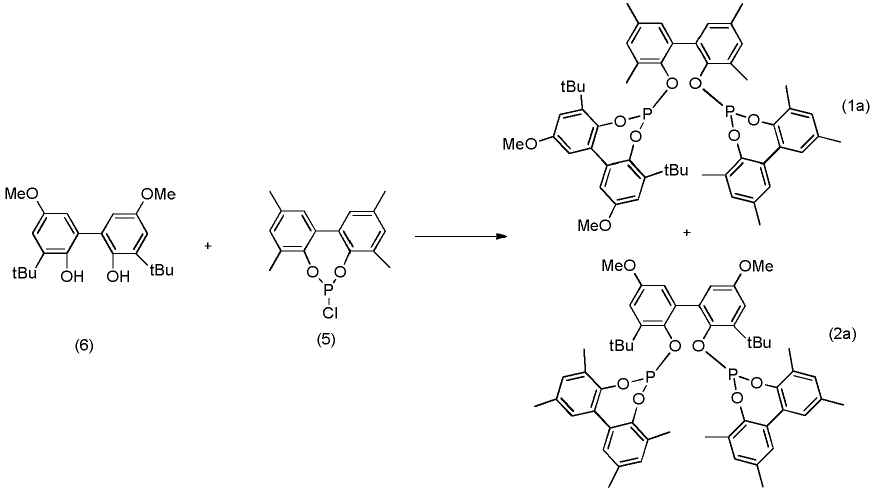

### Variante 1: ACN/NEt₃

In einem 1000 ml Schlenk wurde unter Schutzgas 38,75 g (0,121 mol) 2,2'-Bis-(3,5-dimethylphenyl)chlorophosphit in 150 ml entgastem ACN gelöst und auf 35°C erwärmt. In einem zweiten Schlenk (500 ml) wurden 20,1 g (0,056 mol) 3,3'-Di-tert.-butyl-5,5'-dimethoxy-[1,1'-biphenyl]-2,2'diol in 150 ml entgastem ACN gelöst und unter Rühren mit 40,9 ml entgastem Triethylamin (0,29 mol) versetzt. Dann wurde langsam die Biphenol-Triethylamin-Lösung zu der Chlorophosphitlösung getropft. Nach einer Nachreaktionszeit von 1h wurde die Reaktionslösung über Nacht bei 45°C gerührt.
Anschließend wurde die Lösung filtriert und der Feststoff dreimal mit 100 ml warmen (45°C) ACN gewaschen. Das Zielprodukt konnte als weißer Feststoff (43,3g, 86%) erhalten werden. ³¹P-NMR (202,4 MHz, toluene-d₈): 142,5 und 140,9 (95,4%) 139,2 (4,6%).

### Variante 2: EtOAc/NEt₃

In einem 100 ml Schlenk wurde unter Schutzgas 7,3 g (21,0 mmol) 2,2'-Bis-(3,5-dimethylphenyl)chlorophosphit in 15 ml entgastem Ethylacetat gelöst und auf 35°C erwärmt. In einem zweiten Schlenk (100 ml) wurden 3,9 g (9,5 mmol) 3,3'-Di-tert.-butyl-5,5'-dimethoxy-[1,1'-biphenyl]-2,2'diol in 7,0 ml NEt₃ gelöst. Anschließend wurde die Biphenol/Triethylamin-Lösung langsam innerhalb von 20 Minuten zu der Chlorophosphitlösung getropft. Die Lösung wurde eine weitere Stunde bei 35°C und anschließend über Nacht bei 45°C gerührt.
Am nächsten Tag wurde die Lösung filtriert und der Feststoff dreimal mit ACN gewaschen. Das Zielprodukt konnte als weißer Feststoff (6,7g, 78%) erhalten werden. ³¹P-NMR (202,4 MHz, toluene-d₈): 142,5 und 140,9 (91,3%), 139,5 (8,7%).

### Variante 4: ACN/DMAB (Dimethylaminobutan)

In einem 100 ml Schlenk wurde unter Schutzgas 6 g (19,0 mmol) 2,2'-Bis-(3,5-dimethylphenyl)chlorophosphit in 20 ml entgastem ACN gelöst und auf 35°C erwärmt. In einem zweiten Schlenk (50 ml) wurden 3,4 g (9,0 mmol) 3,3'-Di-tert.-butyl-5,5'-dimethoxy-[1,1'-biphenyl]-2,2'diol in 15 ml Dimethylaminobutan (DMAB) gelöst und anschließend langsam zu der Chlorophosphitlösung getropft. Die Reaktion wurde bei 35°C über Nacht rühren gelassen.
Am nächsten Tag wurde die Lösung filtriert und der Feststoff zweimal mit ACN gewaschen. Das Zielprodukt konnte als weißer Feststoff (5,3g, 66%) erhalten werden. ³¹P-NMR (202,4 MHz, toluene-d₈): 142,8 und 141,2 (97,5%), 139,4 (2,5%).

### Variante 5: ACN/NMP (N-Methylpyrrolidon)

In einem 100 ml Schlenk wurde unter Schutzgas 6 g (19,0 mmol) 2,2'-Bis-(3,5-dimethylphenyl)chlorophosphit in 20 ml entgastem ACN gelöst und auf 35°C erwärmt. In einem zweiten Schlenk (50 ml) wurden 3,4 g (9,0 mmol) 3,3'-Di-tert.-butyl-5,5'-dimethoxy-[1,1'-biphenyl]-2,2'diol in 9,4 ml N-Methylpyrrolidion (NMP) gelöst und langsam zu der Chlorophosphitlösung getropft. Die Reaktion wurde bei 35°C über Nacht rühren gelassen. Anschließend wurde die Lösung filtriert und der Feststoff zweimal mit ACN gewaschen. Das Zielprodukt konnte als weißer Feststoff (3,4g, 42%) erhalten werden. ³¹P-NMR (202,4 MHz, toluene-d₈): 142,2 und 141,0 (96,1%), 139,8 (3,9%).

### Variante 6: ACN/Diisopropylethylamin

In einem 500 ml Schlenk wurde unter Schutzgas 19,4 g (61,0 mmol) 2,2'-Bis-(3,5-dimethylphenyl)chlorophosphit in 75 ml entgastem ACN suspendiert. In einem zweiten Schlenk (250 ml) wurden 10,5 g (28,5 mmol) 3,3'-Di-tert.-butyl-5,5'-dimethoxy-[1,1'-biphenyl]-2,2'diol in 75 ml Acetonitril und 39 ml Diisopropylethylamin suspendiert und langsam zu der Chlorophosphitlösung gegeben. Die Reaktion wurde über Nacht rühren gelassen.
Anschließend wurde die Lösung filtriert und der Feststoff dreimal mit ACN gewaschen. Das Zielprodukt konnte als weißer Feststoff (14,6g, 57%) erhalten werden. ³¹P-NMR (202,4 MHz, toluene-d₈): 142,2 und 141,1 (76,8%), 139,1 (23,2%).

### Variante 7: Toluol/ Dimethylaminobutan (DMAB)

In einem 100 ml Schlenk wurde unter Schutzgas 7,7 g (24,0 mmol) 2,2'-Bis-(3,5-dimethylphenyl)chlorophosphit in 15 ml entgastem Toluol gelöst und auf 35°C erwärmt. In einem zweiten Schlenk (50 ml) wurden 3,4 g (9,0 mol) 3,3'-Di-tert.-butyl-5,5'-dimethoxy-[1,1'-biphenyl]-2,2'diol in 15 ml Dimethylaminobutan (DMAB) gelöst und langsam zu der Chlorophosphitlösung getropft. Die Reaktion wurde bei 45°C 4 Tage rühren gelassen. Im Anschluss daran wurde die Lösung nach weiterer Zugabe von 120 ml Toluol für 30 Minuten auf 75°C erwärmt
Anschließend wurde die Lösung filtriert, das Filtrat bis zur Trockene eingeengt und getrocknet. Das Zielprodukt konnte als weißer Feststoff (7,2g, 88%) erhalten werden. ³¹P-NMR (202,4 MHz, toluene-d₈): 142,5 und 140,9 (91,4%), 139,2 (8,6%).

### Variante 8: Variation der Aminmenge (ACN/NEt₃)

A: In einem 500 ml Schlenk wurde unter Schutzgas 17,81 g (0,073 mol) 2,2'-Bis-(3,5-dimethylphenyl)chlorophosphit in 60 ml entgastem ACN versetzt und auf 35°C erwärmt. In einem zweiten Schlenk (250 ml) wurden 9,91 g (0,0276 mol) 3,3'-Di-tert.-butyl-5,5'-dimethoxy-[1,1'-biphenyl]-2,2'diol in 60 ml entgastem ACN gelöst und unter Rühren mit 38,4 ml entgastem Triethylamin versetzt. Diese Biphenol/Triethylamin-Lösung wurde dann langsam zu der Chlorophosphitlösung getropft. Nach einer Nachreaktionszeit von 1h wurde die Reaktionslösung über Nacht bei 35°C gerührt.
Anschließend wurde die Lösung filtriert und der Feststoff mit ACN gewaschen. Das Zielprodukt konnte als weißer Feststoff (27,8g, 86%) erhalten werden. ³¹P-NMR (202,4 MHz, toluene-d₈): 142,8 und 141,2 (91,6%), 139,4 (8,4%).

**B**: In einem 250 ml Schlenk wurde unter Schutzgas 1,57 g (5,1 mmol) 2,2'-Bis-(3,5-dimethylphenyl)chlorophosphit in 7 ml entgastem ACN versetzt und auf 35°C erwärmt. In einem zweiten Schlenk (100 ml) wurden 0,932 g (2,6 mmol) 3,3'-Di-tert.-butyl-5,5'-dimethoxy-[1,1'-biphenyl]-2,2'diol in 9 ml entgastem ACN gelöst und unter Rühren mit 2,09 ml entgastem Triethylamin versetzt. Dann wurde langsam die Biphenol/Triethylamin-Lösung zu der Chlorophosphitlösung getropft. Nach einer Nachreaktionszeit von 1h wurde die Reaktionslösung über Nacht bei 35°C gerührt.
Anschließend wurde die Lösung filtriert und der Feststoff mit ACN gewaschen. Das Zielprodukt konnte als weißer Feststoff in 40%iger Ausbeute erhalten werden. ³¹P-NMR (202,4 MHz, toluene-d₈): 142,8 und 141,8 (92,4 %), 139,3 (7,6%).

### Variante 9: Verkürzte Reaktionszeiten

### A (8 Stunden): EtOAc/NEt₃

In einem 100 ml Schlenk wurde unter Schutzgas 8 g (25,0 mmol) 2,2'-Bis-(3,5-dimethylphenyl)chlorophosphit in 20 ml entgastem Ethylacetat gelöst und auf 45°C erwärmt. In einem zweiten Schlenk (50 ml) wurden 4,48 g (12,5 mmol) 3,3'-Di-tert.-butyl-5,5'-dimethoxy-[1,1'-biphenyl]-2,2'diol in 20 ml Ethylacetat und 8,0 ml NEt₃ suspendiert. Anschließend wurde die Biphenol/Triethylamin-Suspension langsam innerhalb von 30 Minuten zu der Chlorophosphitlösung getropft. Die Lösung wurde acht Stunden bei 45°C gerührt.

Anschließend wurde die Lösung filtriert. Das Zielprodukt konnte als weißer Feststoff (12,26 g, 84,7%) erhalten werden. ³¹P-NMR (202,4 MHz, toluene-d₈): 142,2 und 141,1 (88,1), 139,1 (11,9).

### B (4 Stunden): EtOAc/NEt₃

In einem 100 ml Schlenk wurde unter Schutzgas 10,07 g (31,0 mmol) 2,2'-Bis-(3,5-dimethylphenyl)chlorophosphit in 20 ml entgastem Ethylacetat gelöst und auf 45°C erwärmt. In einem zweiten Schlenk (50 ml) wurden 5,54 g (15 mmol) 3,3'-Di-tert.-butyl-5,5'-dimethoxy-[1,1'-biphenyl]-2,2'diol in 26ml Ethylacetat und 9,0 ml NEt₃ suspendiert. An-schließend wurde die Biphenol/Triethylamin-Suspension langsam innerhalb von 30 Minuten zu der Chlorophosphitlösung getropft. Die Lösung wurde vier Stunden bei 45°C gerührt.
Anschließend wurde die Lösung filtriert und der Feststoff zweimal mit ACN gewaschen. Das Zielprodukt konnte als weißer Feststoff (6,4g, 47%) erhalten werden. ³¹P-NMR (202,4 MHz, toluene-d₈): 142,2 und 141,1 (99,3%), 139,1 (0,7%).

### C (4 Stunden): ACN/Pyridin

In einem 250 ml Schlenk wurde unter Schutzgas 10 g (31,0 mmol) 2,2'-Bis-(3,5-dimethylphenyl)chlorophosphit in 40 ml entgastem ACN gelöst und auf 45°C erwärmt. In einem zweiten Schlenk (50 ml) wurden 5,5 g (15,0 mmol) 3,3'-Di-tert.-butyl-5,5'-dimethoxy-[1,1'-biphenyl]-2,2'diol in40 ml ACN und 8,8 ml Pyridin gelöst. Dann wurde die entstandene klare Biphenol/Pyridin-Lösung langsam innerhalb von 30 Minuten zu der Chlorophosphitlösung getropft. Nach einer Reaktionszeit von 4 Stunden wurde die Lösung filtriert und der Feststoff zweimal mit ACN gewaschen. Das Zielprodukt konnte als weißer Feststoff (8,5g, 63%) erhalten werden. ³¹P-NMR (202,4 MHz, toluene-d₈): 142,2 und 141,1 (98,4%), 139,4 (1,6%).

### Variante 10: Tieftemperaturversuche (ACN/NEt₃)

**A:** In einem 250 ml Schlenk wurde unter Schutzgas 8,0 g (0,025 mol) 2,2'-Bis-(3,5-dimethylphenyl)chlorophosphit in 30 ml entgastem ACN gelöst und auf -40°C gekühlt. In einem zweiten Schlenk (100 ml) wurden 4,32 g (0,012 mol) 3,3'-Di-tert.-butyl-5,5'-dimethoxy-[1,1'-biphenyl]-2,2'diol in 30 ml entgastem ACN gelöst und unter Rühren mit 8,5 ml entgastem Triethylamin versetzt. Dann wurde langsam die Biphenol/Trietylamin-Lösung zu der Chlorophosphitlösung getropft. Nach einer Nachreaktionszeit von 1h wurde die Reaktionslösung langsam über Nacht auf Raumtemperatur gebracht.
Anschließend wurde die Lösung filtriert und der Feststoff mit kaltem ACN gewaschen. Das Zielprodukt konnte als weißer Feststoff (8,9g, 82%) erhalten werden. ³¹P-NMR (202,4 MHz, toluene-d₈): 142,5 und 140,9 (98,4%), 139,4 (1,6%).

### Variante 11: Durchführung bei verschiedenen Reaktionstemperaturen (ACN/Pyridin)

**A:** In einem 250 ml Schlenk wurde unter Schutzgas 9,4 g (28,8 mmol) 2,2'-Bis-(3,5-dimethylphenyl)chlorophosphit in 100 ml entgastem ACN gelöst. In einem zweiten Schlenk (100 ml) wurden 5,0 g (14,4 mmol) 3,3'-Di-tert.-butyl-5,5'-dimethoxy-[1,1'-biphenyl]-2,2'diol in 8,8 ml Pyridin gelöst. Dann wurde die Biphenol/Pyridin-Lösung langsam innerhalb von 1.5 Stunden zu der Chlorophosphitlösung getropft. Die Lösung wurde weitere 2 Stunden bei Raumtemperatur und anschließend über Nacht bei 60°C gerührt. Anschließend wurde die Lösung filtriert und der Feststoff zweimal mit ACN gewaschen. Das Zielprodukt konnte als weißer Feststoff (9,5g, 73%) erhalten werden. ³¹P-NMR (202,4 MHz, toluene-d₈): 142,8 und 141,2 (90%), 139,5 (10%).

**B**: In einem 250 ml Schlenk wurde unter Schutzgas 10 g (31,0 mmol) 2,2'-Bis-(3,5-dimethylphenyl)chlorophosphit in 40 ml entgastem ACN gelöst und auf 45°C erwärmt. In einem zweiten Schlenk (50 ml) wurden 5,5 g (15,0 mmol) 3,3'-Di-tert.-butyl-5,5'-dimethoxy-[1,1'-biphenyl]-2,2'diol in 40 ml ACN und 8,8 ml Pyridin gelöst. Dann wurde die entstandene klare Biphenol/Pyridin-Lösung langsam innerhalb von 30 Minuten zu der Chlorophosphitlösung getropft. Die Lösung wurde über Nacht bei 45°C gerührt. Am nächsten Morgen wurde die Lösung filtriert und der Feststoff zweimal mit ACN gewaschen. Das Zielprodukt konnte als weißer Feststoff (9,5g, 72%) erhalten werden. ³¹P-NMR (202,4 MHz, toluene-d₈): 142,2 und 141,1. (89,9%), 139,1 (10,1%).

### Vergleichsbeispiel Variante 12: "Eintopfsynthese", nicht erfindungsgemäß

In einem sekurierten 250 ml Schlenk wurden 8,45 g (0,0335 mol) 2,2'-Bis-(3,5-dimethylphenol) und 5,95 g (0,0166 mol) 3,3'-Di-tert.-butyl-5,5'-dimethoxy-[1,1'-biphenyl]-2,2'diol vorgelegt und unter Rühren in 50 ml getrocknetem Toluol suspendiert. Dann wurden nacheinander 7,1 g (0,051 mol) Phosphortrichlorid und 0,1 ml (0,001 mol) Pyridin bei 0°C zu der Suspension hinzugegeben und diese Suspension wurde innerhalb von 60 Minuten auf RT gebracht. Die Reaktionsmischung wurde anschließend auf 35 °C erwärmt und bei dieser Temperatur über Nacht gerührt.
Am Morgen wurden mittels ÖV bei RT das überschüssige Phosphortrichlorid und das Lösungsmittel entfernt. Im Anschluss wurde unter Rühren 25 ml entgastes ACN hinzu zugegeben und die Lösung auf 0 °C abgekühlt. In einem zweiten Schlenk (50 ml) wurden 25 ml entgastes ACN vorgelegt und unter Rühren 10,2 g = 14 ml (0,1 mol) Triethylamin hinzugegeben. Die erhaltene Lösung wurde innerhalb von 45 min. in die abgekühlte Reaktionsmixtur getropft. Dann wurde die Mischung unter Rühren über Nacht auf RT erwärmt. Am Morgen wurde der Feststoff abfiltriert, mit 2 x 25 ml entgastem ACN nachgewaschen. Das gewünschte Zielprodukt konnte in 77% iger Ausbeute (13g) erhalten werden. ³¹P-NMR (202,4 MHz, toluene-d₈): 142.2 und 141.1 (96,4%), 139,2 (3,6%).

### Einfluss der Base /Basenmischung

### Allgemeine Synthesevorschrift

In einem 1000 ml Schlenk wurde unter Schutzgas 38,75 g (0,121 mol) 2,2'-Bis-(3,5-dimethylphenyl)chlorophosphit in 150 ml entgastem ACN gelöst und auf 45 °C erwärmt. In einem zweiten Schlenk (500 ml) wurden 20,1 g (0,056 mol) 3,3'-Di-tert.-butyl-5,5'-dimethoxy-[1,1'-biphenyl]-2,2'diol in 150 ml entgastem ACN gelöst und unter Rühren mit der entsprechenden Base (die verwendete Menge wird auf das Chlorophosphit bezogen) versetzt. Dann wurde langsam die Biphenol/Basen-Lösung zu der Chlorophosphitlösung getropft. Nach einer Nachreaktionszeit von 1h wurde die Reaktionslösung über Nacht bei 45 °C gerührt. (Anderweitige Temperaturen oder Reaktionszeiten sind den Tabellen zu entnehmen.)
Anschließend wurde die Lösung filtriert und der Feststoff mit 100 ml warmen (45 °C) ACN gewaschen. Verbindung **1a** konnte als weißer Feststoff (Ausbeute in %) erhalten werden. ³¹P-NMR (202,4 MHz, toluene-d₈): 142,5 und 140,9 (Ligand **1a** in %), 139,2 (Ligand **2a** in %).

### Syntheseroute:

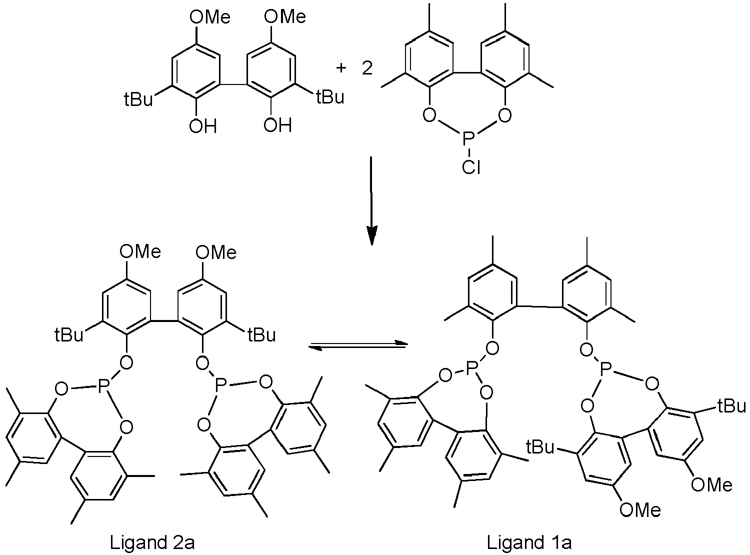

### A) Pyridin und Derivate

**Tabelle 2:**

| Äquivalent Base | Base | Anteil **1a** in [Massen-%] | Anteil **2a** in [Massen-%] | Ausbeute in [%] | |
|---|---|---|---|---|---|
| 4 | Pyridin | 72,0 | 28,0 | 81 | * |
| 4 | Pyridin | 74,0 | 26,0 | 81 | |
| 3 | Pyridin | 80,6 | 19,4 | 80 | |
| 2,5 | Pyridin | 81,9 | 18,1 | 78 | |
| 2 | Pyridin | 84,2 | 15,8 | 78 | |
| 1.7 | Pyridin | 84,2 | 15,8 | 88 | |
| 1,5 | Pyridin | 86,3 | 13,7 | 79 | |
| 1,5 | Pyridin | 84,5 | 15,5 | 82 | ** |
| 2,5 | Pyridin | 81,8 | 18,2 | 78 | *** |
| 2,5 | Pyridin | 86,8 | 13,2 | 81 | **** |
| 2,5 | DMAP | 46,6 | 53,4 | 51 | |
| 2 | DMAP | 42,7 | 57,3 | 50 | # |
| 2 | DMAP | 47,8 | 52,2 | 89 | ## |
| 2 | DMAP | 65,1 | 34,9 | 90 | ### |
| 2 | 2-Picolin | 76,0 | 24,0 | 67 | |

| | | | | | |
|---|---|---|---|---|---|
| DMAP = Dimethylaminopyridin *: Versuch bei 0°C **: Versuch bei 50°C ***: verlängerte Reaktionszeit (5 Tage) ****: sofortige Zugabe anstelle des langsamen Zutropfens #: Reaktion bei 0°C ##: Reaktion bei 3-7°C ###: Reaktion bei 45°C | | | | | |

Wie in Tabelle 2 deutlich erkennbar, ist es möglich, die Isomerenverteilung der beiden Konstitutionsisomere (1a) und (2a) durch die Wahl der Base bzw. der entsprechenden Basenmenge zu steuern. So ist es beispielsweise möglich eine 1:1 Mischung der beiden Isomeren (1a) und (2a) durch Verwendung von DMAP als Base bei niedrigeren Temperaturen zu erhalten.

### B) verschiedene Alkylamine

**Tabelle 3:**

| Äquivalent Base | Base | Anteil **1a** in [Massen-%] | Anteil **2a** in [Massen-%] | Ausbeute in [%] |
|---|---|---|---|---|
| 2,2 | NEt₃ | 95,4 | 4,6 | 86 |
| 2,3 | DMAB | 97,4 | 2,6 | 86 |
| 2,2 | Tributylamin | 94,4 | 5,6 | 90 |
| 2 | Tripentylamin | 96,0 | 4,0 | n.b. |
| 2 | Trihexylamin | 97,8 | 2,2 | 94 |

| | | | | |
|---|---|---|---|---|
| NEt₃: Triethylamin DMAB: Dimethylaminobutan n.b.: nicht bestimmt | | | | |

Wie in Tabelle 3 deutlich erkennbar, ist es möglich, durch die Wahl von Trialkylaminen als Base eine Isomerenmischung zu erhalten, in der das unsymmetrische Isomer (1a) mit einer Reinheit von > 90% als Hauptkomponente vorliegt und das symmetrische Isomer (2a) die entsprechende Nebenkomponente darstellt.

### C) verschiedene Basenmischungen

**Tabelle 4:**

| Basen | Verhältnis | Anteil **1a** in [Massen-%] | Anteil **2a** in [Massen-%] | Ausbeute in [%] |
|---|---|---|---|---|
| Pyr/NEt₃ | 4:1 | 78,2 | 21,8 | 56 |
| Pyr/NEt₃ | 4:0,5 | 59,6 | 40,4 | 87 |
| Pyr/NEt₃ | 4:0,25 | 59,7 | 40,3 | 80 |
| Pyr/NEt₃ | 3:0,5 | 62,4 | 37,6 | 81 |
| Pyr/NEt₃ | 2:0,5 | 69,1 | 30,9 | 84 |
| Pyr/NBu₃ | 2:0,5 | 72,4 | 27,6 | 78 |
| Pyr/NBu₃ | 2:0,25 | 47,9 | 52,1 | 81 |
| Pyr/NBu₃ | 2:2 | 91,8 | 8,2 | 83 |
| Pyr/NBu₃ | 2,5:0,2 | 81,0 | 19,0 | 80 |
| Pyr/NBu₃ | 2,5:2 | 92,6 | 7,4 | 69 |

| | | | | |
|---|---|---|---|---|
| NEt₃: Triethylamin NBu₃: Tributylamin Pyr: Pyridin | | | | |

Wie in Tabelle 4 deutlich erkennbar, ist es möglich, die Isomerenverteilung der beiden Konstitutionsisomere (1a) und (2a) durch den Einsatz von Basenmischungen und deren entsprechenden Basenmenge zu steuern.

Es ist somit möglich, die Isomerenverteilung der beiden Konstitutionsisomeren (1a) und (2a) durch die Wahl der verwendeten Base bzw. Basenmischung so zu beeinflussen, sodass ein Isomer als Hauptkomponente vorliegt. Durch die Wahl von Trialkylaminen als Base ist es möglich eine Isomerenmischung zu erhalten, in der das unsymmetrische Isomer (1a) mit einer Reinheit von > 90% als Hauptkomponente vorliegt und das symmetrische Isomer (2a) die entsprechende Nebenkomponente darstellt. Da diese Mischung auch in der Hydroformylierung eine sehr gute Gesamtperformance zeigt, kann auf weitere Aufreinigungsschritte verzichtet werden.

### Arbeitsvorschrift für die Katalyseversuche der Isomerenmischungen

### Versuchsbeschreibung - allgemein

Die Versuche wurden in 100 ml-Autoklaven der Fa. Parr Instrument durchgeführt. Die Autoklaven sind mit einer elektrischen Beheizung ausgerüstet. Die Druckkonstanthaltung erfolgt über Massedurchflußmesser und Druckregler. Während der Versuchszeit kann über eine Spritzenpumpe eine genau definierte Eduktmenge unter Reaktionsbedingungen eingespritzt werden. Über Kapillarleitungen und HPLC-Ventile können während der Versuchszeit Proben gezogen und sowohl über GC- als auch über LC-MS-Analytik untersucht werden.

### Erfindungsgemäße Ergebnisse der Prüfung verschiedener Gemische von konstitutionsisomeren Bisphosphiten, bestehend aus den Liganden (1a) und (2a), in der Hydroformylierung^{[a]}:

**Tabelle 5:**

| **Nr** | **Ligand** | **Gehalt an Ligand in [%]** | **Pentanalselektivität in [%]^{[b]}** | **Ausbeute In [%]^{[b]}** |
|---|---|---|---|---|
| 1 | Ligand (1 a) | 100 | 94,0 | 92,9 |
| 2* | Ligand (1 a) + Ligand (2a) | 99.3 + 0.7 | 93,9 | 91,0 |
| 3* | Ligand (1a) + Ligand (2a) | 91.9 + 8.1 | 93,7 | 93,1 |
| 4* | Ligand (1a) + Ligand (2a) | 90.3 + 9.7 | 93,8 | 92,6 |
| 5* | Ligand (1a) + Ligand (2a) | 74 + 26 | 93,7 | 92,7 |
| 6* | Ligand (1a) + Ligand (2a) | 80 + 20 | 92,5 | 92,5 |
| 7* | Ligand (1a) + Ligand (2a) | 98.7 + 1.3✦ | 87,9 | 78,7 |

| | | | | |
|---|---|---|---|---|
| * erfindungsgemäß [a] Bedingungen: cis-2-Buten, Rh(acac)(CO)₂ ([Rh]= 95 ppm), L/Rh = 6:1, 40 ml Toluol, Verbindung (Ib), 120°C, 20 bar CO/H₂ (1:1), 1,2,4,5-Tetra-isopropyl-benzol als internen GC-Standard. [b] GC-Analyse mit 1,2,4,5-Tetra-isopropyl-benzol als internen GC-Standard. ✦ weitere Nebenkomponenten u.a. nicht umgesetztes Chlorophosphit in größeren Mengen enthalten. Die gewünschte Zusammensetzung aus den beiden Liganden (1a) und (2a) ist nur in einer Reinheit von 30% in einem Gemisch mit anderen Komponenten/Verunreinigungen enthalten. | | | | |

Bei einem Vergleich der verschiedenen Ligandenmischungen aus den Liganden (1a) und (2a) (Tabelle 5, Einträge 2-6) mit dem Hydroformylierungsergebnis des reinen Liganden (1a) (Tabelle 5, Eintrag 1) zeigt sich, dass die Mischungen sehr gute Pentanalselektivitäten und Ausbeuten aufweisen. Auch bei der Verwendung einer Ligandenmischung, in der der Ligand (1a) nur in einer Reinheit von ca. 30% enthalten ist (Tabelle 5, Eintrag 7) konnte immer noch eine sehr gute Ausbeute und Selektivität generiert werden. Durch die Verwendung dieses Gemisches von konstitutionsisomeren Bisphosphiten, bestehend aus Ligand (1a) und (2a), konnte die technische Aufgabe somit vollends erfüllt werden und die entsprechenden Aldehyde in guten bis sehr guten Selektivitäten und Ausbeuten erhalten werden.

### Versuchsbeschreibung - Langzeitversuch

Der Rh-Precursor (Rh(acac)(CO)₂) (acac= acetylacetonat) und der Ligand werden in 40 ml Isononylbenzoat im Autoklaven vorgelegt. Die Rh-Konzentration beträgt 100 ppm bezogen auf die gesamte eingesetzte Reaktionsmasse. Der Ligandüberschuß beträgt molar 4:1 bezogen auf Rhodium.
Als weitere Komponente wird im Verhältnis 2:1 zum Liganden die Verbindung (Ib) als Amin zugegeben. Als GC-Standard werden 0,5 g 1,2,4,5-Tetraisopropylbenzol hinzugegeben.
Reaktionstemperatur ist 120 °C. Der Reaktionsdruck beträgt 20 bar Synthesegas (H₂:CO=50:50 Vol%).

Als Olefin wurden mit der Spritzenpumpe in Abständen von ca. 1 Tag jeweils 4 ml cis-2-Buten zudosiert. GC-Proben wurden nach 1, 2, 4 Stunden und vor der nächsten Dosierung gezogen.
Es wurden folgende Liganden hinsichtlich ihrer Stabilität untersucht:

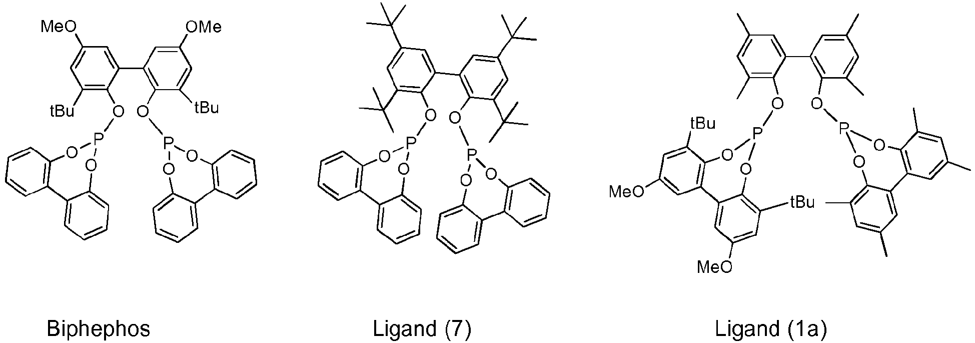

Ferner wurde ein Gemisch von konstitutionsisomeren Bisphosphiten, bestehend aus den Liganden (1a) und (2a) (³¹P-NMR bestimmtes Mengenverhältnis : L1a=91% + L2a=9%), untersucht. 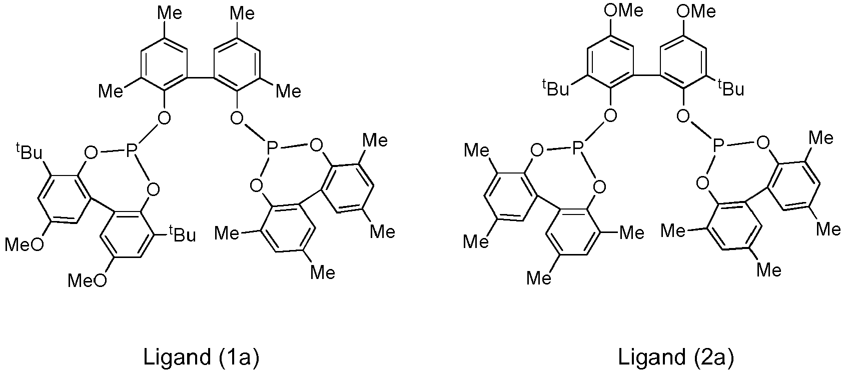 sowie eine Mischung aus Ligand (7) und Ligand (8) (³¹P-NMR bestimmtes Mengenverhältnis: L7=75% + L8=25%)

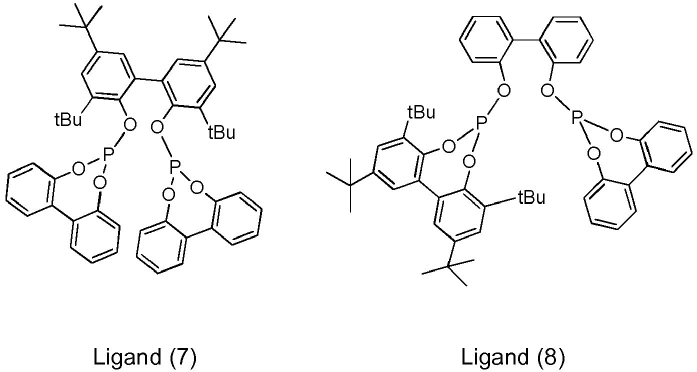

### Ergebnisse - Langzeitversuche

Die relativen Aktivitäten werden durch das Verhältnis von k 1.Ordnung zu k0, d.h. dem k-Wert zum Zeitpunkt 0 der Reaktion (Reaktionsstart), bestimmt und beschreiben die relative Aktivitätsabnahme während der Versuchslaufzeit.
Die k-Werte 1.Ordnung erhält man aus einer Auftragung von (-ln(1-Umsatz)) gegen die Zeit.

**Tabelle 6:**

| **Lfd. Nr.** | **Ligand** | **Dosierung bei Laufzeit (h)** | **k 1. Ordnung (min^-1)** | **k/k0 rel. Aktivität** | **n/i-Selektivitäten** |
|---|---|---|---|---|---|
| 1 | Biphephos | 0 | 1,39E-02 | 1 | 21 |
| 2 | Biphephos | 20,5 | 4,45E-03 | 0,32 | 21 |
| 3 | Biphephos | 44,3 | 2,91 E-03 | 0,209 | 20 |
| 4 | Biphephos | 66,6 | 1,72E-03 | 0,124 | 20 |
| 5 | Ligand (7) + Ligand (8) | 0 | 1,36E-02 | 1 | 3,1 |
| 6 | Ligand (7) + Ligand (8) | 20,5 | 5,32E-03 | 0,391 | 2,4 |
| 7 | Ligand (7) + Ligand (8) | 44,3 | 4,80E-03 | 0,353 | 1,8 |
| 8 | Ligand (1 a) | 0 | 7,74E-03 | 1 | 17 |
| 9 | Ligand (1 a) | 20,8 | 5,10E-03 | 0,659 | 16 |
| 10 | Ligand (1a) | 44,8 | 3,19E-03 | 0,412 | 15 |
| 11 | Ligand (1a) | 117,8 | 2,99E-03 | 0,386 | 14 |
| 12* | Ligand (1a) + Ligand (2a) | 0 | 1,09E-02 | 1 | 14 |
| 13* | Ligand (1a) + Ligand (2a) | 20,8 | 5,65E-03 | 0,518 | 14 |
| 14* | Ligand (1a) + Ligand (2a) | 44,8 | 4,13E-03 | 0,379 | 13 |
| 15* | Ligand (1a) + Ligand (2a) | 117,8 | 3,35E-03 | 0,307 | 13 |
| 16 | Ligand (7) | 0 | 1,72E-02 | 1 | 14 |
| 17 | Ligand (7) | 22,4 | 9,00E-03 | 0,523 | 13 |
| 18 | Ligand (7) | 44,7 | 5,39E-03 | 0,313 | 13 |
| 19 | Ligand (7) | 68,3 | 3,31E-03 | 0,192 | 13 |

| | | | | | |
|---|---|---|---|---|---|
| * erfindungsgemäß | | | | | |

**Fazit:** Der Aktivitätsabfall des Katalysators mit den Liganden Biphephos und Ligand (7) ist (Tabelle 6; Einträge 1-4, 16-19) deutlich stärker als mit dem Liganden (1a). Bemerkenswert ist, dass die relative Aktivität des Liganden (1a) nach annähernd der doppelten Reaktionszeit (Tabelle 6; Eintrag 11) immer noch mehr als doppelt so hoch ist wie bei den anderen beiden Liganden nach der halben Reaktionszeit (Tabelle 6; Einträge 4 und 19) bei weiterhin sehr guten n/i-Selektivitäten.

Vergleicht man das Gemisch von konstitutionsisomeren Bisphosphiten, bestehend aus den Liganden (1a) und (2a), mit dem reinen Liganden (1a) (Tabelle 6; Einträge 8-11, 12-15) so zeigt das Gemisch nach 117 Stunden Laufzeit eine vergleichbare Aktivität und Selektivität wie der reine Ligand (1a). Sowohl der reine Ligand (1a) als auch das Gemisch von konstitutionsisomeren Bisphosphiten, bestehend aus den Liganden (1a) und (2a), zeichnen sich somit durch eine exzellente Standzeit aus. Das Gemisch von konstitutionsisomeren Bisphosphiten, bestehend aus den Liganden (7) und (8), zeigt von Anfang an eine deutlich schlechtere Selektivität als der reine Ligand (7) wie auch das Gemisch von konstitutionsisomeren Bisphosphiten, bestehend aus den Liganden (1a) und (2a), (Tabelle 6; Einträge 5-7, 12-15 und 16-19).

Die Zugabe des unsymmetrischen Liganden (8) zum symmetrischen Liganden (7) führt zu einem drastischen Selektivitätseinbruch (Tabelle 6; Einträge 5-7).

Dies entspricht den Ergebnissen aus dem Stand der Technik (siehe in Rhodium-catalyzed Hydroformylation, ed. by P.W.N.M. van Leeuwen et C. Claver, Kluwer Academic Publishers 2006, AA Dordrecht, NL, S.eite 45- 46). ). Im Gegensatz dazu zeichnet sich der unsymmetrische Ligand (1a), sowohl als Reinstoff als auch in der Mischung mit dem Liganden (2a) (Tabelle 6; Einträge 8-11, 12-15) völlig überraschend durch exzellente Standzeiten als auch sehr gute Selektivitäten aus. Ferner konnte gezeigt werden, dass somit auch Mischungen von konstitutionsisomeren Liganden (1a) und (2a) ohne zusätzliche aufwendige Reinigungsprozesse direkt aus der Synthese eingesetzt werden können und die technische Aufgabe lösen.

### Beispiele für Landzeitversuche

### Beispiel L1: Hydroformylierung mit dem nicht erfindungsgemäßen Liganden (100) über 1200 h (Vergleichsbeispiel 1)

Der aus EP2280920B1 bekannte, nicht erfindungsgemäße Ligand der Formel (100) wurde in der Hydroformylierung einer Buten/Butan-Mischung eingesetzt.

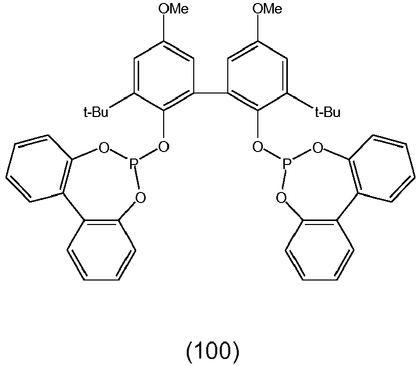

Dabei wurde Ligand (100) mit dem Amin der Formel (Ib) stabilisiert.

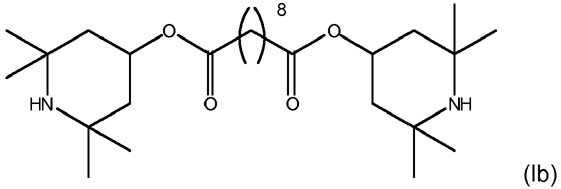

Die kontinuierlich betriebene Versuchsanlage bestand im Wesentlichen aus einem 20 Liter fassenden Druckreaktor mit einem nachgeschalteten Kondensator und Phasentrennbehälter (Gas/Flüssigkeit) für die aus dem Reaktor stammende Gasphase sowie einem Kreisgasverdichter, der die Gasphase aus dem Phasentrennbehälter wieder unten in die Reaktionszone zurück führt. Ein Teil dieses Kreisgases wird nach der Phasentrennung als Abgas aus dem Reaktionssystem gefahren. Um eine optimale Gasverteilung im Reaktorsystem zu realisieren, wurde hier ein Gasverteilerring mit Bohrungen verbaut. Über installierte Heiz- und Kühlvorrichtungen konnte der Reaktor temperiert werden.

Vor der Hydroformylierung wurde das System mit Stickstoff frei von Sauerstoff gespült. Anschließend wurde der Reaktor mit 12 Liter Katalysatorlösung gefüllt.

Diese Katalysatorlösung setzte sich aus 12 kg eines eutektischen Gemisches aus Biphenyl und Diphenylether (Diphyl^{®} ,Wärmeträgeröl der Fa. Lanxess), 3 g Rh(acac)(CO)₂, 36 g Bisphosphit-Ligand der Formel (100), 67.5 g Amin der Formel (Ib) zusammen und wurde vorher in einem Behälter gemischt. Das eutektische Gemisch aus Biphenyl und Diphenylether (Diphyl^{®}) wurde zuvor mit Stickstoff gestrippt, um Sauerstoff und Wasser aus dem Wärmeträgeröl zu entfernen.

Anschließend wurde das Reaktorsystem mit Synthesegas frei von Stickstoff gespült. Nachdem der Stickstoffgehalt unter 10 Vol.-% gefallen war, wurde das Reaktorsystem mit Synthesegas auf 1.0 MPa aufgedrückt und anschließend auf 120 °C aufgeheizt. Nach Erreichen der Betriebstemperatur wurde das Reaktorsystem mit Synthesegas auf 1.7 MPa Reaktionsdruck gebracht.

Sodann wurde die Zugabe der Ausgangsstoffe gestartet. Hierzu wurde ein Einsatzgemisch über einen Verdampfer gefahren, um es gasförmig in das Kreisgas zu fahren. Bei dem Einsatzgemisch handelte es sich um eine Mischung aus 35 Gew.-% 2-Butenen und 1-Buten in einer Konzentration von ca. 1 %. Der Rest war n-Butan.

Folgende Durchsätze wurden eingestellt: 0,3 kg/h Einsatzgemisch, 75 NI/h Synthesegas (50 Vol% H₂ und 50 Vol% CO)

Zur täglichen Dosierung des Bisphosphit-Liganden (100) und Amins (Ib) wurde eine 1.4%-ige Lösung des Bisphosphit-Liganden (100) in n-Pentanal angesetzt, welches zuvor durch Strippen mit Stickstoff von restlichen C₄-Kohlenwasserstoffen (< 3 %) befreit wurde. Das Amin (Ib) wurde in einem dreifachen molaren Überschuss zum Bisphosphit-Liganden (100) eingesetzt. Zur besseren Stabilisierung dieser Lösung wurde das Amin (Ib) vor dem Bisphosphit-Liganden (100) zur Lösung gegeben.

Nach ca. 1000 h wurde ein stationärer Zustand erreicht. Die Reaktionsprodukte wurden kontinuierlich über den Kreisgasstrom aus dem Reaktor entfernt und im Kondensator bei 50 °C partiell auskondensiert. Die auskondensierte Phase wurde kontinuierlich aus dem Phasentrennbehälter gefahren. Zur Umsatzbestimmung wurden aus dem Kreisgas vor und nach Reaktor Proben gezogen.

Durch eine tägliche Dosierung der oben beschriebenen Ligandenlösung, konnte der Umsatz und die Regioselektivität konstant gehalten werden.

Zur Bestimmung des Reaktorinhaltes wurden Proben aus dem Reaktor entnommen und mittels Flüssigchromatographie (HLPC) untersucht. Unter den gewählten Reaktionsbedingungen wurden Butenumsätze von rund 65 bis 70 % erzielt. Die prozentuale Verteilung zwischen n-Pentanal und 2-Methylbutanal, bzw. die n/iso-Selektivität, betrug 95 % zu 5 %.In der stationären Phase des Versuches konnte kein Rhodiumabbau verzeichnet werden.

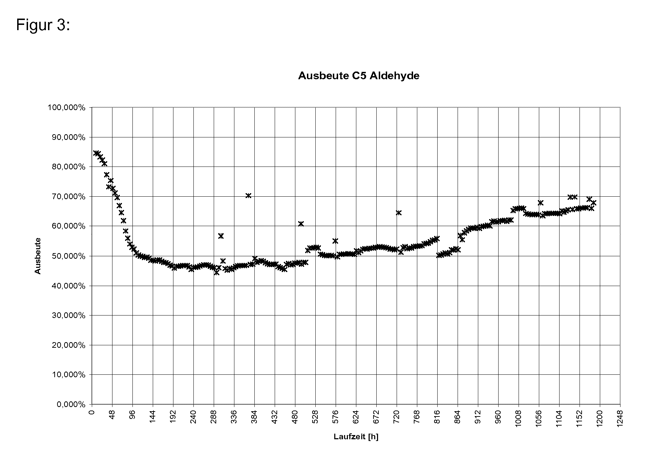

Die Ausbeute der C₅-Aldehyde über die Versuchszeit ist in Figur 3 aufgetragen.

### Figur 3: Pentanal-Ausbeute zu Beispiel L1

Nach 1200 h wurde der Reaktor entspannt und die Katalysatorlösung untersucht. Im Reaktor zeigte sich ein Niederschlag. Eine Analyse dieses Niederschlages ergab, dass dieser aus phosphorhaltigen Folgeprodukten des Bisphosphit-Liganden (100) und dem eingesetzten Amin (Ib) bestanden. Es wurden keinerlei Anbackungen dieser Ausfällungen in dem Reaktor festgestellt.

Ein Teil des Reaktorinhaltes wurde, nach Abtrennung des Niederschlages, bei 1.2 KPa abs. und 220 °C Sumpftemperatur auf 13 % bezogen auf die Einsatzmasse eingeengt. Der erhaltene Rückstand aus dem Sumpf war noch fließfähig und es wurde kein Niederschlag festgestellt. Eine Rhodiumanalyse zeigte, dass das sich das gesamte Rhodium aus der Einsatzmasse in diesem Sumpfrückstand befand.

### Beispiel L2: Hydroformylierung mit dem nicht erfindungsgemäßen Liganden (100) über 8000 h (Vergleichsbeispiel 2)

Die Versuchsdurchführung fand in der, in Beispiel L1 beschriebenen Versuchsanlage statt. Die Vorbereitung des Versuches und die Durchführung fand analog zum Beispiel L1 statt.

In diesem Beispiel setzte sich die Katalysatorlösung aus 12 kg Isononylbenzoat, 4.5 g Rh(acac)(CO)₂, 55 g Bisphosphit-Ligand der Formel (100), 67.5 g Amin der Formel (Ib) zusammen. Das Isononylbenzoat wurde ebenfalls zuvor mit Stickstoff gestrippt, um Sauerstoff und Wasser aus dem Lösemittel zu entfernen.

Anschließend wurde das Reaktorsystem mit Synthesegas frei von Stickstoff gespült. Nachdem der Stickstoffgehalt unter 10 Vol.-% gefallen war, wurde das Reaktorsystem mit Synthesegas auf 1.0 MPa aufgedrückt und anschließend auf 120 °C aufgeheizt. Nach Erreichen der Betriebstemperatur wurde das Reaktorsystem mit Synthesegas auf 1.7 MPa Reaktionsdruck gebracht.

Anschließend wurde die Zugabe der Ausgangsstoffe gestartet. Hierzu wurde ein Einsatzgemisch über einen Verdampfer gefahren, um es gasförmig in das Kreisgas zu fahren. Bei dem Einsatzgemisch handelte es sich um eine Mischung aus 35 Gew.-% 2-Butenen und 1-Buten in einer Konzentration von ca. 1 %. Der Rest war n-Butan. Folgende Durchsätze wurden eingestellt: 0,3 kg/h Einsatzgemisch, 75 NI/h Synthesegas (50 Vol% H₂ und 50 Vol% CO).

Zur täglichen Dosierung des Bisphosphit-Liganden (100) und Amins (Ib) wurde eine 1.4%-ige Lösung des Bisphosphit-Liganden (100) in n-Pentanal angesetzt, welches zuvor durch Strippen mit Stickstoff von restlichen C₄-Kohlenwasserstoffen (< 3 %) befreit wurde. Das Amin (Ib) wurde in einem dreifachen molaren Überschuss zum Bisphosphit-Liganden (100) eingesetzt. Zur besseren Stabilisierung dieser Lösung wurde das Amin (Ib) vor dem Bisphosphit-Liganden (100) zur Lösung gegeben.

Wie in Beispiel L1 wurde nach etwa 1000 h ein stationärer Zustand erreicht. Die Reaktionsprodukte wurden kontinuierlich über den Kreisgasstrom aus dem Reaktor entfernt und im Kondensator bei 50 °C partiell auskondensiert. Die auskondensierte Phase wurde kontinuierlich aus dem Phasentrennbehälter gefahren. Zur Umsatzbestimmung wurden aus dem Kreisgas vor und nach Reaktor Proben gezogen.

Durch eine tägliche Dosierung der oben beschriebenen Ligandenlösung, konnte der Umsatz und die Regioselektivität konstant gehalten werden.

Zur Bestimmung des Reaktorinhaltes wurden Proben aus dem Reaktor entnommen und mittels Flüssigchromatographie (HLPC) untersucht. Unter den gewählten Reaktionsbedingungen wurden Butenumsätze von rund 65 bis 70 % erzielt. Die prozentuale Verteilung zwischen n-Pentanal und 2-Methylbutanal, bzw. die n/iso-Selektivität, betrug 95 % zu 5 %.In der stationären Phase des Versuches konnte kein Rhodiumabbau verzeichnet werden.

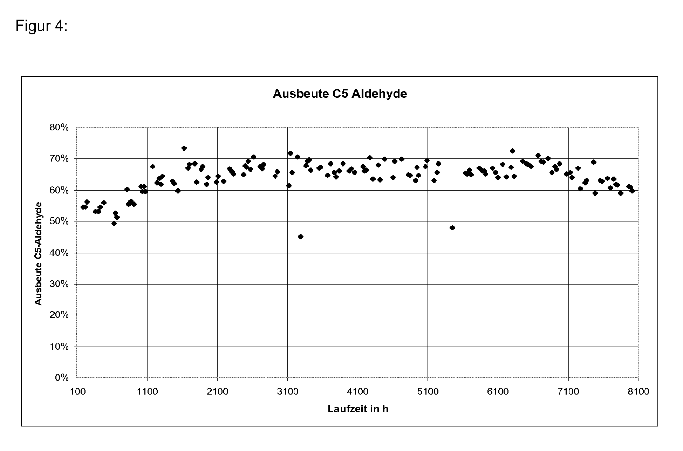

Die Ausbeute der C₅-Aldehyde über die Versuchszeit ist in Figur 4 aufgetragen.

### Figur 4: Pentanal-Ausbeute zu Beispiel L2

Nach 1500 h zeigten sich in den Proben aus dem Reaktor erste Niederschläge. Die Analyse dieser Niederschläge ergab, dass dieser, ebenso wie in Beispiel L1, aus phosphorhaltigen Folgeprodukten des Bisphosphit-Liganden (100) und dem eingesetzten Amin (Ib) bestanden.

Die Reaktion wurde insgesamt 8100 h betrieben, die Rhodiumverluste durch Probenahmen wurden durch Zugabe entsprechender Mengen Rh(acac)(CO)₂ in die tägliche Ligandendosierungslösung ausgeglichen.

Im Verlauf wurde nach ca. 7000 h ein Aktivitätsrückgang in der Reaktion beobachtet und die Reaktionslösung neigte zum Schäumen. Der Prozess konnte nicht mehr betrieben werden und der Versuch musste beendet werden.

Nach Ende der Reaktion wurde der Reaktor entspannt und die Reaktionsmischung untersucht. Es zeigten sich große Mengen an Feststoff. 250 ml der Reaktionslösung wurden 4 h unter N₂ Atomsphase bei 40°C gerührt und anschließend die Viskosität des Rückstands vermessen. Die Viskosität betrug 300 mPas.

### Beispiel L3: Hydroformylierung mit erfindungsgemäßem Katalysatorsystem

Es wurde dieselbe Versuchsanlage eingesetzt wie in Beispiel L3. Es wurde dasselbe Einsatzgemisch und dasselbe Synthesegas verwendet. Als Ligand wurde indes eine Mischung aus den beiden Bisphosphit-Liganden (1a) und (2a) eingesetzt. Der aus EP2280920B1 bekannte Ligand der Formel (100) war nicht im Reaktionsgemisch enthalten. Es wurde dasselbe Amin (Ib) wie im Vergleichsbeispiel 1 (L1) als Stabilisator verwendet. Als Lösungsmittel wurde Isononylbenzoat eingesetzt.

Vor der Hydroformylierung wurde das System mit Stickstoff frei von Sauerstoff gespült. Anschließend wurde der Reaktor mit 12 Liter Katalysatorlösung gefüllt.

Diese Katalysatorlösung setzte sich aus 12 kg Isononylbenzoat, 4.5 g Rh(acac)(CO)2, 63 g Liganden-Isomerengemisch der Formeln (1a) und (2a), 200g Amin der Formel (Ib) zusammen und wurde vorher in einem Behälter gemischt. Das Isononylbenzoat wurde zuvor mit Stickstoff gestrippt, um Sauerstoff und Wasser aus dem Lösemittel zu entfernen.

Anschließend wurde das Reaktorsystem mit Synthesegas frei von Stickstoff gespült. Nachdem der Stickstoffgehalt unter 10 Vol-% gefallen war, wurde das Reaktorsystem mit Synthesegas auf 1.0 MPa aufgedrückt und anschließend auf 120 °C aufgeheizt. Nach Erreichen der Betriebstemperatur wurde das Reaktorsystem mit Synthesegas auf 1.7 MPa Reaktionsdruck gebracht.

Sodann wurde die Zugabe der Ausgangsstoffe gestartet. Das Einsatzgemisch wurde über einen Verdampfer gefahren, um es gasförmig in das Kreisgas zu fahren. Folgende Durchsätze wurden eingestellt: 0.3 kg/h Einsatzgemisch, 75 NI/h Synthesegas.

Zur täglichen Dosierung des Isomerengemisches bestehend aus (1a) und (2a) und Amins (Ib) wurde eine 1.4%-ige Lösung der Ligandenmischungen aus den Bisphosphit-Liganden (1a) und (2a) in n-Pentanal angesetzt, welches zuvor durch Strippen mit Stickstoff von restlichen C₄-Kohlenwasserstoffen (< 3 %) befreit wurde. Das Amin (Ib) wurde in einem dreifachen molaren Überschuss zum Ligandenisomerengemisch bestehend aus (1a) und (2a) eingesetzt. Zur besseren Stabilisierung dieser Lösung wurde das Amin (Ib) vor dem Bisphosphit-Ligandenisomerengemisch zur Lösung gegeben.

Die Reaktionsprodukte wurden kontinuierlich über den Kreisgasstrom aus dem Reaktor entfernt und im Kondensator bei 50 °C partiell auskondensiert. Die auskondensierte Phase wurde kontinuierlich aus dem Phasentrennbehälter gefahren. Zur Ausbeutebestimmung wurden aus dem Kreisgas vor und nach Reaktor Proben gezogen und mittels Gaschromatograph analysiert.

Durch eine tägliche Dosierung der oben beschriebenen Ligandenlösung, konnte der Umsatz und die Regioselektivität konstant gehalten werden. Zur Bestimmung des Reaktorinhaltes wurden Proben aus dem Reaktor entnommen und mittels Flüssigchromatographie (HLPC) untersucht.

Unter den gewählten Reaktionsbedingungen stellte sich zum Start der Reaktion eine Aldehydausbeute zwischen 80% und 90% ein. Nach 8000 h Betriebszeit fiel die Ausbeute auf ca. 65% ab, bedingt durch die Rhodiumverluste durch die Probennahmen. Ein Schäumen der Reaktionslösung konnte in diesem Fall nicht festgestellt werden. Die prozentuale Verteilung zwischen n-Pentanal und 2-Methylbutanal, bzw. die Regio-Selektivität, betrug 92 % zu 8 %.

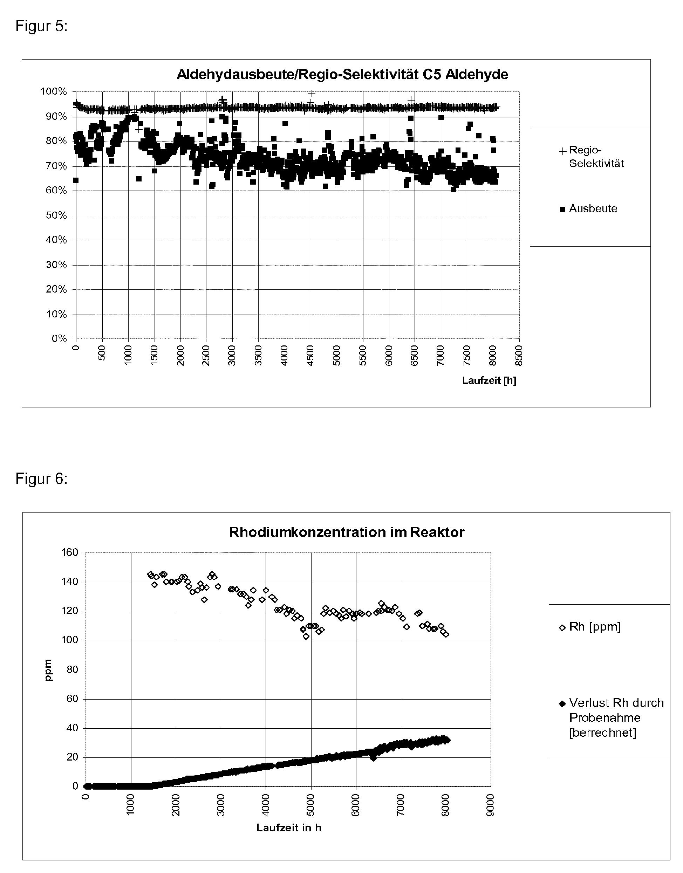

Aldehydausbeute und Regio-Selektivität sind über die Versuchsdauer in Figur 5 aufgetragen.

### Figur 5: Aldehydausbeute und Regio-Selektivität zu Beispiel L3

In der stationären Phase des Versuches konnte, abgesehen von den Rhodiumverlusten durch die Probenahme, kein weiterer Rhodiumabbau verzeichnet werden.

Die Rhodium-Konzentration im Reaktor über die Versuchsdauer ist in Figur 6 aufgetragen.

### Figur 6: Rh-Konzentration zu Beispiel L3

Nach Ende der Reaktion wurde der Reaktor entspannt und die Reaktionsmischung untersucht. Es zeigte sich kein Feststoff. 250ml der Reaktionslösung wurden 4 h unter N₂ Atmosphäre bei 40 °C gerührt und anschließend die Viskosität des Rückstands vermessen. Die Viskosität betrug 20 mPas.

### Vergleich der Beispiele L1, L2 und L3

Vergleicht man die entsprechenden Beispiele, so hebt sich das erfindungsgemäß durchgeführte Beispiel L3 durch folgende Merkmale deutlich von den Beispielen L1 und L2 ab, die den Stand der Technik wiedergegeben:
Das erfindungsgemäße Beispiel L3 zeigt keine Einfahrphase, das heißt das System zeigt keinen Aktivitätsrückgang in den ersten 1000 h Betriebszeit und somit produziert die Anlage im erfindungsgemäßen Beispiel L3 im gleichen Zeitraum deutlich mehr Produkt. Im Vergleichsbeispiel 2 (L2) fällt im Laufe der Reaktion Feststoff an, der nur über eine aufwendige Filtration entfernt werden kann. Das erfindungsgemäße Beispiel L3 zeigt auch nach über 8000 h keinen Feststoffanfall, somit kann in diesem Verfahren auf die Filtration verzichtet werden.

Das Vergleichsbeispiel 2 (L2) zeigt ein deutliches Schäumen der Reaktionslösung zum Ende des Versuches, sodass der Prozess nicht mehr betrieben werden kann. Ein solches Verhalten ließe sich nur durch aufwendige Schaumbrecher verhindern. Das erfindungsgemäße Verfahren kommt ohne diese Hilfsmittel aus.

### Erfindungsgemäße Ergebnisse - Substratvariation

Für die nachfolgenden Versuche wurde ein Gemisch von konstitutionsisomeren Bisphosphiten, bestehend aus den Liganden (1a) und (2a) (³¹P-NMR bestimmtes Mengenverhältnis: L1a=91% + L2a=9%), untersucht.

### Beispiel 1

In einem 100 ml-Autoklaven der Fa. Parr Instruments wurde bei 120 °C und 30 bar 4.8 g Propen hydroformyliert. Als Precursor wurden 0.005 g Rh(acac)(CO)₂ in 43.08 g Toluol vorgelegt. Als Ligand wurden 0.0708 g der oben beschriebenen Ligandenmischung (L1a=91% + L2a=9%) in der Katalysatoransatzlösung eingesetzt. Als organisches Amin wurden 0.0401 g der Verbindung (Ib) und 0.5033 g TIPB als GC-Standard zugefügt. Das Edukt wurde nach Erreichen der vorgesehenen Reaktionstemperatur zudosiert.
Während der Reaktion wurde der Druck über eine Synthesegasregelung mit Massedurchflussmesser konstant gehalten. Proben wurden aus der Reaktionsmischung nach 20 Stunden gezogen. Es wurden 88.4 mol-% Butanal, 6,.48 mol-% 2-Methylpropanal und 2,79 mol-% Propan gebildet. Die Regioselektivität zu n-Butanal beträgt 93.2 %.

### Beispiel 2

In einem 100 ml-Autoklaven der Fa. Parr Instruments wurde bei 120 °C und 20 bar 6.7 g cis-2-Buten hydroformyliert. Als Precursor wurden 0.0053 g Rh(acac)(CO)₂ in 43.48 g Toluol vorgelegt. Als Ligand wurden 0.0671 g der oben beschriebenen Ligandenmischung (L1a=91% + L2a=9%) in der Katalysatoransatzlösung eingesetzt. Als organisches Amin wurden 0.0381 g der Verbindung (Ib) und 0.5099 g TIPB als GC-Standard zugefügt. Das Edukt wurde nach Erreichen der vorgesehenen Reaktionstemperatur zudosiert.
Während der Reaktion wurde der Druck über eine Synthesegasregelung mit Massedurchflussmesser konstant gehalten. Proben wurden aus der Reaktionsmischung nach 20 Stunden gezogen. Es wurden 84.6 mol-% Pentanal, 5.70 mol-% 2-Methylbutanal und 3.43 mol-% n-Butan gebildet. Die Regioselektivität zu n-Pentanal beträgt 93.7 %.

### Beispiel 3

In einem 100 ml-Autoklaven der Fa. Parr Instruments wurde bei 120 °C und 20 bar 6.7 g 1-Buten hydroformyliert. Als Precursor wurden 0.0052 g Rh(acac)(CO)₂ in 43.08 g Toluol vorgelegt. Als Ligand wurden 0.0694 g der oben beschriebenen Ligandenmischung (L1a=91% + L2a=9%) in der Katalysatoransatzlösung eingesetzt. Als organisches Amin wurden 0.0378 g der Verbindung (Ib) und 0.5052 g TIPB als GC-Standard zugefügt. Das Edukt wurde nach Erreichen der vorgesehenen Reaktionstemperatur zudosiert.
Während der Reaktion wurde der Druck über eine Synthesegasregelung mit Massedurchflussmesser konstant gehalten. Proben wurden aus der Reaktionsmischung nach 20 Stunden gezogen. Es wurden 86.5 mol-% Pentanal, 5.08 mol-% 2-Methylbutanal und 3.23 mol-% n-Butan gebildet. Die Regioselektivität zu n-Pentanal beträgt 98.9 %.

### Beispiel 4

In einem 100 ml-Autoklaven der Fa. Parr Instruments wurde bei 120 °C und 20 bar 6.7 g Isobuten hydroformyliert. Als Precursor wurden 0.0051 g Rh(acac)(CO)₂ in 42.1 g Toluol vorgelegt. Als Ligand wurden 0.0678 g der oben beschriebenen Ligandenmischung (L1a=91% + L2a=9%) in der Katalysatoransatzlösung eingesetzt. Als organisches Amin wurden 0.0369 g der Verbindung (Ib) und 0.4937 g TIPB als GC-Standard zugefügt. Das Edukt wurde nach Erreichen der vorgesehenen Reaktionstemperatur zudosiert.
Während der Reaktion wurde der Druck über eine Synthesegasregelung mit Massedurchflussmesser konstant gehalten. Proben wurden aus der Reaktionsmischung nach 20 Stunden gezogen. Es wurden 64.0 mol-% 3-Methylbutanal, 0.07 mol-% Pivalinaldehyd und 2.92 mol-% iso-Butan gebildet.

### Beispiel 5

In einem 100 ml-Autoklaven der Fa. Parr Instruments wurde bei 120 °C und 20 bar 7.4 g eines C-4-Gemisches mit folgender Zusammensetzung: 2.9 mol-% Isobutan, 9,9 mol-% n-Butan, 28.7 mol-% 1-Buten, 43.5 mol-% Isobuten, 14,6 mol % 2-Butene und 0.2 mol % 1,3-Butadien. hydroformyliert. Als Precursor wurden 0.0048 g Rh(acac)(CO)₂ in 41.49 g Toluol vorgelegt. Als Ligand wurden 0.0681 g der oben beschriebenen Ligandenmischung (L1a=91% + L2a=9%) in der Katalysatoransatzlösung eingesetzt. Als organisches Amin wurden 0.0367 g der Verbindung (Ib) und 0.5027 g TIPB als GC-Standard zugefügt. Das Edukt wurde nach Erreichen der vorgesehenen Reaktionstemperatur zudosiert.
Während der Reaktion wurde der Druck über eine Synthesegasregelung mit Massedurchflussmesser konstant gehalten. Proben wurden aus der Reaktionsmischung nach 20 Stunden gezogen. Der Austrag enthält 32.7 % 3-Methylbutanal (Umsatz Isobuten 75.2 mol -%), 39.44 mol-% n-Pentanal und 2.18 mol% 2-Methylbutanal (Umsatz Butene 78.1 mol-%, Regioselektivität zu n-Pentanal 94.8 %). Als Hydrierprodukte werden im Austrag 4.13 mol-% Isobutan und 9.95 mol % n-Butan gefunden.

### Beispiel 6

In einem 100 ml-Autoklaven der Fa. Parr Instruments wurde bei 120 °C und 20 bar 7.0 g eines C-4-Gemisches mit folgender Zusammensetzung: 5.9 mol-% Isobutan, 15.6 mol-% n-Butan, 52.9 mol-% 1-Buten, 0.1 mol-% Isobuten, 24.8 mol % 2-Butene und 0.5 mol % 1,3-Butadien hydroformyliert. Als Precursor wurden 0.0054 g Rh(acac)(CO)₂ in 46.93 g Toluol vorgelegt. Als Ligand wurden 0.0755 g der oben beschriebenen Ligandenmischung (L1a=91% + L2a=9%) in der Katalysatoransatzlösung eingesetzt. Als organisches Amin wurden 0.0412 g der Verbindung (Ib) und 0.5467 g TIPB als GC-Standard zugefügt. Das Edukt wurde nach Erreichen der vorgesehenen Reaktionstemperatur zudosiert.
Während der Reaktion wurde der Druck über eine Synthesegasregelung mit Massedurchflussmesser konstant gehalten. Proben wurden aus der Reaktionsmischung nach 20 Stunden gezogen. Der Austrag enthält 0.17 mol-% 3-Methylbutanal, 70.31 mol-% n-Pentanal und 4.20 mol-% 2-Methylbutanal (Umsatz Butene 93.4 mol-%, Regioselektivität zu n-Pentanal 94.4 %). Als Hydrierprodukte werden im Austrag 5.52 mol-% Isobutan und 18.1 mol % n-Butan gefunden.

### Beispiel 7

In einem 100 ml-Autoklaven der Fa. Parr Instruments wurde bei 120 °C und 20 bar 5.0 g eines C-4-Gemisches mit folgender Zusammensetzung: 5.9 mol-% Isobutan, 22.0 mol-% n-Butan, 45.5 mol-% 1-Buten, 2.1 mol-% Isobuten, 17.1 mol % 2-Butene und 0.2 mol % 1,3-Butadien hydroformyliert. Als Precursor wurden 0.0044 g Rh(acac)(CO)₂ in 37.96 g Toluol vorgelegt. Als Ligand wurden 0.0611 g der oben beschriebenen Ligandenmischung (L1a=91% + L2a=9%) in der Katalysatoransatzlösung eingesetzt. Es Als organisches Amin wurden 0.0333 g der Verbindung (Ib) und 0.4422 g TIPB als GC-Standard zugefügt. Das Edukt wurde nach Erreichen der vorgesehenen Reaktionstemperatur zudosiert.
Während der Reaktion wurde der Druck über eine Synthesegasregelung mit Massedurchflussmesser konstant gehalten. Proben wurden aus der Reaktionsmischung nach 20 Stunden gezogen. Der Austrag enthält 1.52 mol-% 3-Methylbutanal (Umsatz Isobuten 72.1 mol-%), 63.2 mol-% n-Pentanal und 3.13 mol-% 2-Methylbutanal (Umsatz Butene 95.6 mol-%, Regioselektivität zu n-Pentanal 95.3 %). Als Hydrierprodukte werden im Austrag 5.41 mol-% Isobutan und 23.89 mol % n-Butan gefunden.

### Beispiel 8

In einem 100 ml-Autoklaven der Fa. Parr Instruments wurde bei 120 °C und 20 bar 6.4 g eines C-4-Gemisches mit folgender Zusammensetzung: 3.4 mol-% Isobutan, 13.0 mol-% n-Butan, 47.3 mol-% 1-Buten, 13.9 mol-% Isobuten, 21.6 mol % 2-Butene und 0.4 mol % 1,3-Butadien hydroformyliert. Als Precursor wurden 0.0052 g Rh(acac)(CO)₂ in 44.95 g Toluol vorgelegt. Als Ligand wurden 0.0704 g der oben beschriebenen Ligandenmischung (L1a=91% + L2a=9%) in der Katalysatoransatzlösung eingesetzt. Als organisches Amin wurden 0.0387 g der Verbindung (Ib) und 0.5318 g TIPB als GC-Standard zugefügt. Das Edukt wurde nach Erreichen der vorgesehenen Reaktionstemperatur zudosiert.
Während der Reaktion wurde der Druck über eine Synthesegasregelung mit Massedurchflussmesser konstant gehalten. Proben wurden aus der Reaktionsmischung nach 20 Stunden gezogen. Der Austrag enthält 9.93 mol-% 3-Methylbutanal (Umsatz Isobuten 71.7 mol-%), 62.6 mol-% n-Pentanal und 2.98 mol-% 2-Methylbutanal (Umsatz Butene 95.6 mol-%, Regioselektivität zu n-Pentanal 95.5 %). Als Hydrierprodukte werden im Austrag 3.59 mol-% Isobutan und 15.41 mol % n-Butan gefunden.

### Beispiel 9

In einem 100 ml-Autoklaven der Fa. Parr Instruments wurde bei 120 °C und 20 bar 6.8 g eines C-4-Gemisches mit folgender Zusammensetzung: 0.1 mol-% Isobutan, 27.6 mol-% n-Butan, 27.9 mol-% 1-Buten, 0.1 mol-% Isobuten und 44.0 mol % 2-Butene hydroformyliert. Als Precursor wurden 0.0051 g Rh(acac)(CO)₂ in 42.29 g Toluol vorgelegt. Als Ligand wurden 0.0681 g der oben beschriebenen Ligandenmischung (L1a=91% + L2a=9%) in der Katalysatoransatzlösung eingesetzt. Als organisches Amin wurden 0.0371 g der Verbindung (Ib) und 0.4960 g TIPB als GC-Standard zugefügt. Das Edukt wurde nach Erreichen der vorgesehenen Reaktionstemperatur zudosiert.
Während der Reaktion wurde der Druck über eine Synthesegasregelung mit Massedurchflussmesser konstant gehalten. Proben wurden aus der Reaktionsmischung nach 20 Stunden gezogen. Der Austrag enthält 60.45 mol-% n-Pentanal und 3.51 mol-% 2-Methylbutanal (Umsatz Butene 92.8 mol-%, Regioselektivität zu n-Pentanal 94.5 %). Als Hydrierprodukte werden im Austrag 0.1 mol-% Isobutan und 28.8 mol % n-Butan gefunden.

### Beispiel 10

In einem 100 ml-Autoklaven der Fa. Parr Instruments wurde bei 120 °C und 20 bar 6.8 g eines C-4-Gemisches mit folgender Zusammensetzung: 63.6 mol-% n-Butan, 1.0 mol-% 1-Buten und 35.8 mol % 2-Butene hydroformyliert. Als Precursor wurden 0.0049 g Rh(acac)(CO)₂ in 40.42 g Toluol vorgelegt. Als Ligand wurden 0.0651 g der oben beschriebenen Ligandenmischung (L1a=91% + L2a=9%) in der Katalysatoransatzlösung eingesetzt. Als organisches Amin wurden 0.0354 g der Verbindung (Ib) und 0.4740 g TIPB als GC-Standard zugefügt. Das Edukt wurde nach Erreichen der vorgesehenen Reaktionstemperatur zudosiert.

Während der Reaktion wurde der Druck über eine Synthesegasregelung mit Massedurchflussmesser konstant gehalten. Proben wurden aus der Reaktionsmischung nach 20 Stunden gezogen. Der Austrag enthält 27.76 mol-% n-Pentanal und 2.14 mol-% 2-Methylbutanal (Umsatz Butene 81.0 mol-%, Regioselektivität zu n-Pentanal 92.8 %). Als Hydrierprodukte werden im Austrag 65.0 mol % n-Butan gefunden.

### Beispiel 11

In einem 100 ml-Autoklaven der Fa. Parr Instruments wurde bei 120 °C und 20 bar 6.8 trans-2-Buten hydroformyliert. Als Precursor wurden 0.0054 g Rh(acac)(CO)₂ in 43.78 g Toluol vorgelegt. Als Ligand wurden 0.0696 g der oben beschriebenen Ligandenmischung (L1a=91% + L2a=9%) in der Katalysatoransatzlösung eingesetzt. Als organisches Amin wurden 0.0370 g der Verbindung (Ib) und 0.5121 g TIPB als GC-Standard zugefügt. Das Edukt wurde nach Erreichen der vorgesehenen Reaktionstemperatur zudosiert.
Während der Reaktion wurde der Druck über eine Synthesegasregelung mit Massedurchflussmesser konstant gehalten. Proben wurden aus der Reaktionsmischung nach 20 Stunden gezogen.
Der Austrag enthält 85.4 mol-% n-Pentanal und 5.95 mol-% 2-Methylbutanal (Regioselektivität zu n-Pentanal 93.4 %). Als Hydrierprodukte werden im Austrag 3.99 mol % n-Butan gefunden.

### Beispiel 12

In einem 100 ml-Autoklaven der Fa. Parr Instruments wurde bei 120 °C und 20 bar 6.0 g eines Kohlenwasserstoffgemisches aus katalytisch betriebenen Spaltanlagen mit folgender Zusammensetzung: 1.5 mol-% Propan, 0.8 mol-% Propen, 28.1 mol-% Isobutan, 8.1 mol-% n-Butan, 16.4 mol-% 1-Buten, 16.9 mol-% Isobuten, 28.2 mol-% 2-Butene, 0.5 mol-% 1,3-Butadien und Anteile an C5-Olefinen und -Kohlenwasserstoffen hydroformyliert. Als Precursor wurden 0.0046 g Rh(acac)(CO)₂ in 39.43 g Toluol vorgelegt. Als Ligand wurden 0.0672 g der oben beschriebenen Ligandenmischung (L1a=91% + L2a=9%) in der Katalysatoransatzlösung eingesetzt. Als organisches Amin wurden 0.0331 g der Verbindung (Ib) und 0.4665 g TIPB als GC-Standard zugefügt. Das Edukt wurde nach Erreichen der vorgesehenen Reaktionstemperatur zudosiert. Während der Reaktion wurde der Druck über eine Synthesegasregelung mit Massedurchflussmesser konstant gehalten. Proben wurden aus der Reaktionsmischung nach 20 Stunden gezogen.

Der Austrag enthält 1.2 mol-% Propan, 0.68 mol-% Butanal, 26.9 mol-% Isobutan, 9.66 mol-% n-Butan, 12.66 mol-% 3-Methylbutanal (74.8 % Isobutenumsatz), 39.5 mol-% Pentanal, 2.07 mol % 2-Methylbutanal (Umsatz n-Butene 97.9 %, Regioselektivtät zu n-Pentanal 95.0 %).

### Beispiel 13

In einem 100 ml-Autoklaven der Fa. Parr Instruments wurde bei 120 °C und 20 bar 5.8 g 1,3-Butadien hydroformyliert. Als Precursor wurden 0.0048 g Rh(acac)(CO)₂ in 41.19 g Toluol vorgelegt. Als Ligand wurden 0.0677 g der oben beschriebenen Ligandenmischung (L1a=91% + L2a=9%) in der Katalysatoransatzlösung eingesetzt. Als organisches Amin wurden 0.0364 g der Verbindung (Ib) und 0.4991 g TIPB als GC-Standard zugefügt. Das Edukt wurde nach Erreichen der vorgesehenen Reaktionstemperatur zudosiert. Während der Reaktion wurde der Druck über eine Synthesegasregelung mit Massedurchflussmesser konstant gehalten. Proben wurden aus der Reaktionsmischung nach 20 Stunden gezogen.
Der Austrag enthält 0.26 mol-% n-Butan, 14.25 % n-Butene, 16.65 % Aldehyde und 9.68 mol-% 4-Vinyl-Cyclohexen. Der Gesamtumsatz an 1-3-Butadien beträgt 42.4 %.

### Beispiel 14

In einem 100 ml-Autoklaven der Fa. Parr Instruments wurde bei 120 °C und 50 bar 1.8 g Ethen hydroformyliert. Als Precursor wurden 0.0050 g Rh(acac)(CO)₂ in 42.68 g Toluol vorgelegt. Als Ligand wurden 0.0668 g der oben beschriebenen Ligandenmischung (L1a=91% + L2a=9%) in der Katalysatoransatzlösung eingesetzt. Als organisches Amin wurden 0.0363 g der Verbindung (Ib) und 0.5095 g TIPB als GC-Standard zugefügt. Das Edukt wurde nach Erreichen der vorgesehenen Reaktionstemperatur zudosiert. Während der Reaktion wurde der Druck über eine Synthesegasregelung mit Massedurchflussmesser konstant gehalten. Proben wurden aus der Reaktionsmischung nach 20 Stunden gezogen. Der Umsatz zu Propanal beträgt 98.7 %.

### Beispiel 15

In einem 100 ml-Autoklaven der Fa. Parr Instruments wurde bei 120 °C und 20 bar 5.74 g Ölsäuremethylester hydroformyliert. Als Precursor wurden 0.0049 g Rh(acac)(CO)₂ in 42.00 g Toluol vorgelegt. Als Ligand wurden 0.0665 g der oben beschriebenen Ligandenmischung (L1a=91% + L2a=9%) in der Katalysatoransatzlösung eingesetzt. Als organisches Amin wurden 0.0345 g der Verbindung (Ib) und 0.4956 g TIPB als GC-Standard zugefügt. Das Edukt wurde nach Erreichen der vorgesehenen Reaktionstemperatur zudosiert. Während der Reaktion wurde der Druck über eine Synthesegasregelung mit Massedurchflussmesser konstant gehalten. Proben wurden aus der Reaktionsmischung nach 20 Stunden gezogen. Aus ¹H- und ¹³C-NMR-Spektren wurden eine Aldehydausbeute von 43.3 mol-% berechnet. Die Regioselektivtät zu endständigen Aldeyden beträgt 22.2 mol-%. Der Doppelbindungsanteil beträgt 36.3 mol-%.

## Patentansprüche

1. Gemisch aus Verbindungen der Formeln (1a) und (2a):

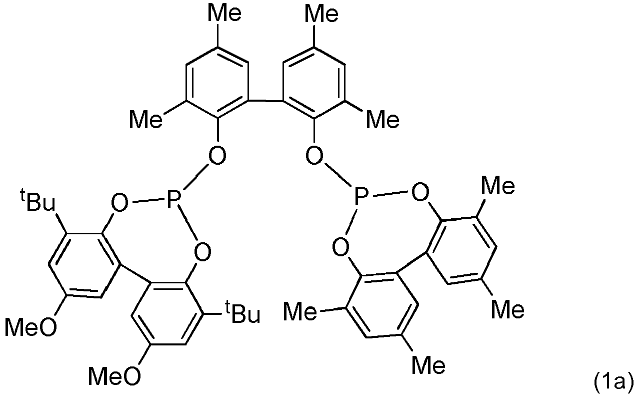

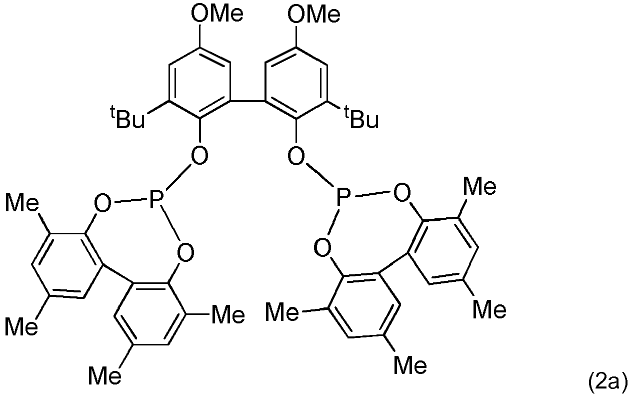

2. Gemisch nach Anspruch 1, wobei der Gehalt an Isomerem der Formel (1a) in einem Bereich von 74 bis 99 Massen-%, der Gehalt an Isomerem der Formel (2a) in einem Bereich von 1 bis 26 Massen-% liegt.

3. Verfahren zur Herstellung des Gemisch nach Anspruch 1 oder 2,
umfassend die Schritte:
i) oxidative Kopplung von 2,4-Dimethylphenol zu 3, 3', 5, 5'-Tetramethyl-2,2'-dihydroxybiphenyl;
ii) oxidative Kopplung von 3-tert.-Butyl-4-Hydroxyanisol zu 5, 5'-Dimethoxy-3, 3'-di-tert.-Butyl-2, 2'-dihydroxybiphenyl;
iii) Umsetzung von 3, 3', 5, 5'-Tetramethyl-2,2'-dihydroxybiphenyl aus i) mit PCl₃ zu einem Phosphorochloriditderivat unter Inertgasatmosphäre;
iv) Umsetzung von zumindest 2 Äquivalenten des Phophorochloriditderivats aus iii) mit 1 Äquivalent des 5, 5'-Dimethoxy-3, 3'-di-tert.-Butyl-2, 2'-dihydroxybiphenyl aus ii) unter Inertgasatmosphäre.

4. Verfahren nach Anspruch 3,
wobei im Verfahrensschritt iv) ein Lösungsmittelgemisch verwendet wird.

5. Verfahren nach Anspruch 4, wobei das Lösungsmittelgemisch, welches im Verfahrensschritt iv) verwendet wird, ausgewählt ist aus organischen Stickstoffverbindungen, organischen Estern, Aromaten.

6. Verfahren nach Anspruch 5, **dadurch gekennzeichnet, dass** die organischen Stickstoffverbindungen ausgewählt sind aus: Nitrilen, Aminen, Amiden.

7. Komplexgemisch aus Verbindungen der Formeln (1b) und (2b): 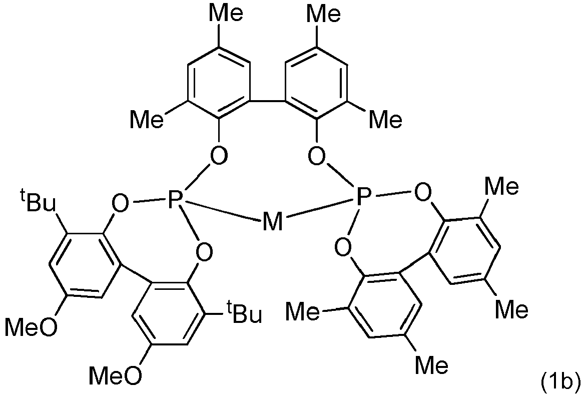 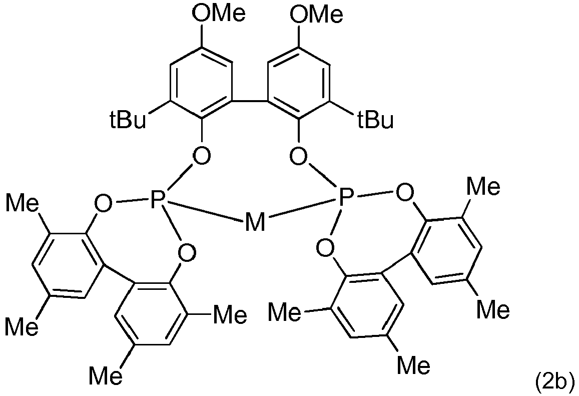 wobei M jeweils ausgewählt ist aus Fe, Ru, Os, Co, Rh, Ir, Ni, Pd, Pt und M zusätzliche Bindungen eingehen kann.

8. Komplexgemisch nach Anspruch 7,
welches zusätzlich mindestens eine der Verbindungen (1a) oder (2a) umfasst, die nicht an M gebunden sind.

9. Komplexgemisch nach einem der Ansprüche 7 oder 8,
wobei M für Rh steht.

10. Komplexgemisch nach Anspruch 9, welches Verbindung (1 ca) aufweist.

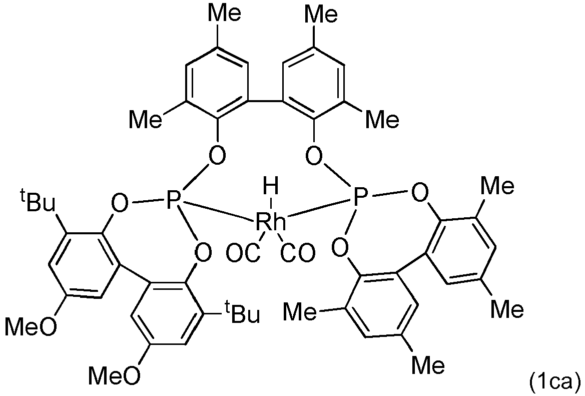

11. Zusammensetzung umfassend:
- ein Komplexgemisch nach einem der Ansprüche 7 bis 10,
- eine weitere Komponente ausgewählt aus Basen, organische Amine, Epoxide, Pufferlösungen, Ionenaustauscher.

12. Zusammensetzung nach Anspruch 11, wobei das organische Amin zumindest eine 2,2,6,6-Tetramethylpiperidineinheit aufweist.

13. Zusammensetzung nach Anspruch 12, wobei das organische Amin ein Sebacinsäuredi-4-(2,2,6,6-tetramethylpiperidinyl)-ester ist.

14. Verfahren zur Hydroformylierung einer ungesättigten Verbindung oder eines Gemisches von ungesättigten Verbindungen umfassend die Schritte:
a) Vorlegen eines Gemisches nach den Ansprüchen 1 und 2 oder einer Zusammensetzung nach einem der Ansprüche 11 bis 13;
b) Einleiten eines Gasgemisches umfassend Kohlenmonoxid und Wasserstoff;
c) Zugabe mindestens einer ungesättigten Verbindung oder eines Gemisches von ungesättigten Verbindungen.

15. Verfahren nach Anspruch 14, wobei die ungesättigten Verbindungen und deren Gemische ausgewählt sind aus:
- Kohlenwasserstoffgemischen aus Dampfspaltanlagen;
- Kohlenwasserstoffgemischen aus katalytisch betriebenen Spaltanlagen;
- Kohlenwasserstoffgemischen aus Oligomerisierungsprozessen;
- Kohlenwasserstoffgemischen umfassend mehrfach ungesättigte Verbindungen;
- ungesättigten Carbonsäurederivaten.

16. Verfahren nach Anspruch 15, **dadurch gekennzeichnet, dass** die Kohlenwasserstoffgemische ungesättigte Verbindungen mit 2 bis 30 Kohlenstoffatomen aufweisen.

17. Verfahren nach einem der Ansprüche 15 bis 16, **dadurch gekennzeichnet, dass** das Gemisch ungesättigte Verbindungen mit 2 bis 8 Kohlenstoffatomen aufweist.

18. Verfahren nach Anspruch 15, **dadurch gekennzeichnet, dass** die ungesättigten Carbonsäurederivate ausgewählt sind unter Fettsäureeestern.

## Claims

1. Mixture of compounds of the formulae (1a) and (2a):

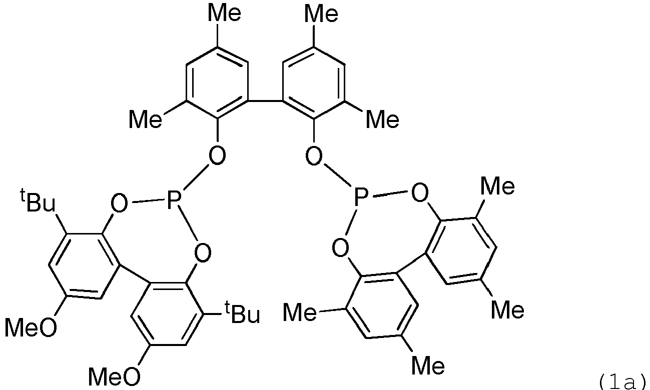

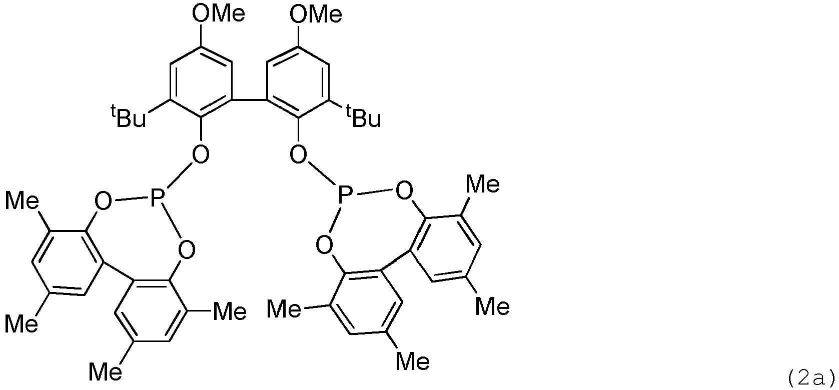

2. Mixture according to Claim 1, wherein the content of isomers of the formula (1a) is within a range from 74 to 99% by mass, and the content of isomers of the formula (2a) within a range from 1 to 26% by mass.

3. Process for preparing the mixture according to Claim 1 or 2,
comprising the steps of:
i) oxidatively coupling 2,4-dimethylphenol to give 3,3',5,5'-tetramethyl-2,2'-dihydroxybiphenyl;
ii) oxidatively coupling 3-tert-butyl-4-hydroxyanisole to give 5,5'-dimethoxy-3,3'-di-tert-butyl-2,2'-dihydroxybiphenyl;
iii) reacting 3,3',5,5'-tetramethyl-2,2'-dihydroxybiphenyl from i) with PCl₃ to give a phosphorochloridite derivative under inert gas atmosphere;
iv) reacting at least 2 equivalents of the phosphorochloridite derivative from iii) with 1 equivalent of the 5,5'-dimethoxy-3,3'-di-tert-butyl-2,2'-dihydroxybiphenyl from ii) under inert gas atmosphere.

4. Process according to Claim 3,
wherein a solvent mixture is used in process step iv).

5. Process according to Claim 4, wherein the solvent mixture which is used in process step iv) is selected from organic nitrogen compounds, organic esters, aromatics.

6. Process according to Claim 5, **characterized in that** the organic nitrogen compounds are selected from: nitriles, amines, amides.

7. Complex mixture of compounds of the formulae (1b) and (2b): 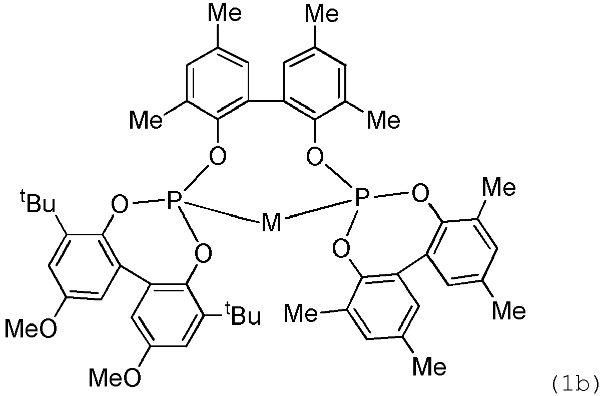 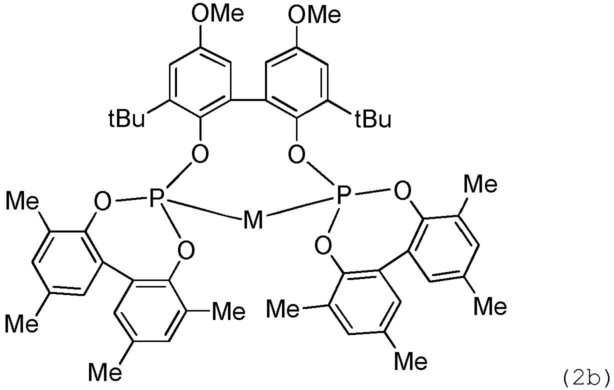 where each M is selected from Fe, Ru, Os, Co, Rh, Ir, Ni, Pd, Pt, and M may enter into additional bonds.

8. Complex mixture according to Claim 7,
which additionally comprises at least one of the compounds (1a) or (2a) not bonded to M.

9. Complex mixture according to either of Claims 7 and 8,
where M is Rh.

10. Complex mixture according to Claim 9, including compound (1ca).

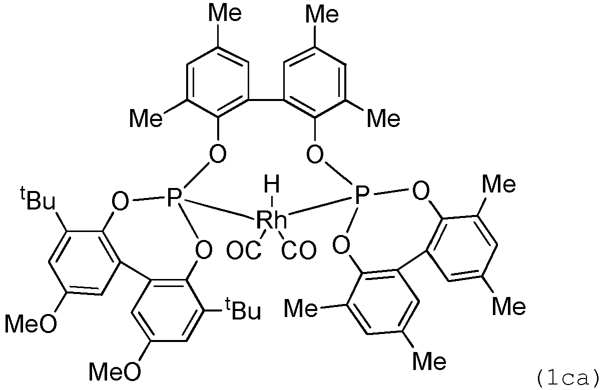

11. Composition comprising:
- a complex mixture according to any of Claims 7 to 10,
- a further component selected from bases, organic amines, epoxides,
buffer solutions, ion exchangers.

12. Composition according to Claim 11, wherein the organic amine has at least one 2,2,6,6-tetramethylpiperidine unit.

13. Composition according to Claim 12, wherein the organic amine is a di-4-(2,2,6,6-tetramethylpiperidinyl) sebacate.

14. Process for hydroformylating an unsaturated compound or a mixture of unsaturated compounds, comprising the steps of:
a) initially charging a mixture according to Claims 1 and 2 or a composition according to any of Claims 11 to 13;
b) introducing a gas mixture comprising carbon monoxide and hydrogen;
c) adding at least one unsaturated compound or a mixture of unsaturated compounds.

15. Process according to Claim 14, wherein the unsaturated compounds and mixtures thereof are selected from:
- hydrocarbon mixtures from steamcracking plants;
- hydrocarbon mixtures from catalytically operated cracking plants;
- hydrocarbon mixtures from oligomerization operations;
- hydrocarbon mixtures comprising polyunsaturated compounds;
- unsaturated carboxylic acid derivatives.

16. Process according to Claim 15, **characterized in that** the hydrocarbon mixtures include unsaturated compounds having 2 to 30 carbon atoms.

17. Process according to either of Claims 15 and 16, **characterized in that** the mixture includes unsaturated compounds having 2 to 8 carbon atoms.

18. Process according to Claim 15, **characterized in that** the unsaturated carboxylic acid derivatives are selected from fatty acid esters.

## Revendications

1. Mélange de composés de formules (1a) et (2a) :

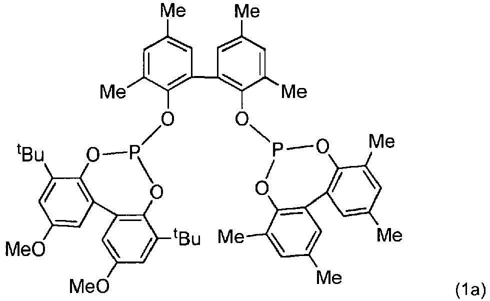

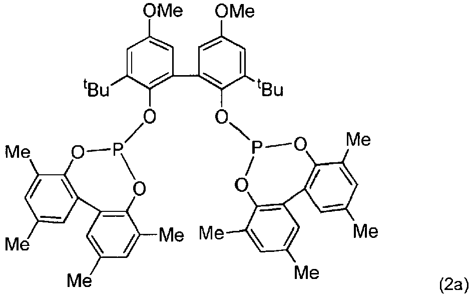

2. Mélange selon la revendication 1, dont la teneur en l'isomère de formule (1a) est comprise dans la plage de 74 à 99 % en masse, la teneur en l'isomère de formule (2a) dans une plage de 1 à 26 % en masse.

3. Procédé de préparation du mélange selon la revendication 1 ou 2, comprenant les étapes :
i) couplage oxydatif de 2,4-diméthylphénol en 3,3',5, 5'-tétraméthyl-2,2'-dihydroxybiphényle ;
ii) couplage oxydatif du 3-tert-butyl-4-hydroxyanisole en 5,5'-diméthoxy-3,3'-di-tert-butyl-2,2'-dihydroxybiphényle ;
iii) réaction du 3,3',5,5'-tétraméthyl-2,2'-dihydroxybiphényle de i) avec du PCl₃, pour donner un dérivé phosphorochloridite sous une atmosphère d'un gaz inerte ;
iv) réaction d'au moins 2 équivalents du dérivé phosphorochloridite de iii) avec 1 équivalent du 5,5'-diméthoxy-3,3'-di-tert-butyl-2,2'-dihydroxybiphényle de ii) sous une atmosphère d'un gaz inerte.

4. Procédé selon la revendication 3, dans lequel, dans l'étape iv), on utilise un mélange de solvants.

5. Procédé selon la revendication 4, dans lequel le mélange de solvants qui est utilisé dans l'étape iv) est choisi parmi les composés azotés organiques, le esters organiques, les composés aromatiques.

6. Procédé selon la revendication 5, **caractérisé en ce que** les composés azotés organiques sont choisis parmi les nitriles, les amines, les amides.

7. Mélange complexe de composés de formules (1b) et (2b) : 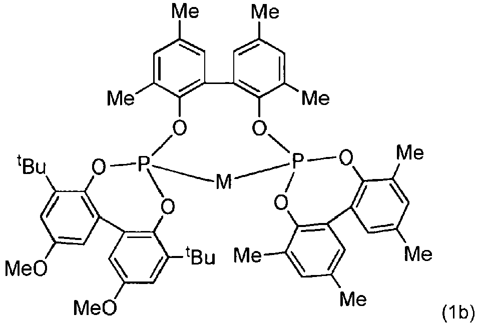 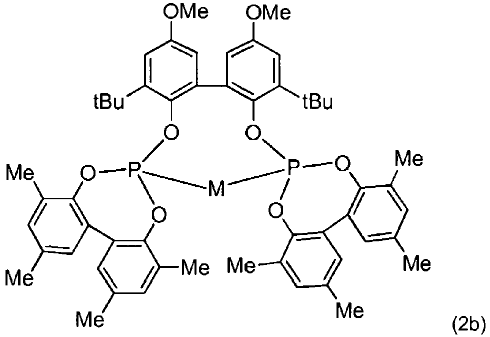 dans lequel chaque M est choisi parmi Fe, Ru, Os, Co, Rh, Ir, Ni, Pd, Pt, et M peut former des liaisons supplémentaires.

8. Mélange complexe selon la revendication 7, qui comprend en outre au moins l'un des composés (1a) ou (2a) qui ne sont pas liés à M.

9. Mélange complexe selon l'une des revendications 7 ou 8, dans lequel M représente Rh.

10. Mélange complexe selon la revendication 9, qui comprend un composé (1ca)

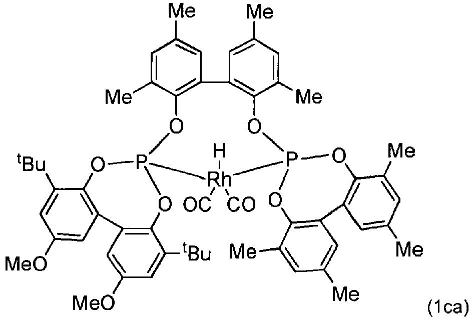

11. Composition comprenant :
- un mélange complexe selon l'une des revendications 7 à 10,
- un composant supplémentaire choisi parmi les bases, les amines organiques, les époxydes, les solutions tampons, les échangeurs d'ions.

12. Composition selon la revendication 11, dans laquelle l'amine organique comprend au moins un motif 2,2,6,6-tétraméthylpipéridine.

13. Composition selon la revendication 12, dans laquelle l'amine organique est un ester di-4-(2,2,6,6-tétraméthylpipéridinylique) de l'acide sébacique.

14. Procédé pour l'hydroformylation d'un composé insaturé ou d'un mélange de composés insaturés, comprenant les étapes :
a) mise en place d'un mélange selon les revendications 1 et 2 ou d'une composition selon l'une des revendications 11 à 13 ;
b) introduction d'un mélange gazeux comprenant du monoxyde de carbone et de l'hydrogène ;
c) addition d'au moins un composé insaturé ou d'un mélange de composés insaturés.

15. Procédé selon la revendication 14, dans lequel les composés insaturés et leurs mélanges sont choisis parmi :
- les mélanges d'hydrocarbures provenant d'installations de vapocraquage ;
- les mélanges d'hydrocarbures provenant d'installations de craquage catalytique ;
- les mélanges d'hydrocarbures provenant de procédés d'oligomérisation ;
- les mélanges d'hydrocarbures comprenant des composés polyinsaturés ;
- les dérivés d'acides carboxyliques insaturés.

16. Procédé selon la revendication 15, **caractérisé en ce que** les mélanges d'hydrocarbures comprennent des composés insaturés ayant 2 à 30 atomes de carbone.

17. Procédé selon l'une des revendications 15 à 16, **caractérisé en ce que** le mélange comprend des composés insaturés ayant 2 à 8 atomes de carbone.

18. Procédé selon la revendication 15, **caractérisé en ce que** les dérivés d'acides carboxyliques insaturés sont choisis parmi les esters d'acides gras.
